# FASCICULE DE BREVET EUROPEEN

(11) **EP 1 514 112 B1**
(45) Date de publication et mention de la délivrance du brevet: **06.09.2006**
(21) Numéro de dépôt: 03748170.2
(22) Date de dépôt: 16.06.2003
(51) Int. Cl.: G01N 33/557, G01N 33/542, G01N 33/566, C07C 229/14

(54) **PROCEDE D'IDENTIFICATION D'EFFECTEURS ALLOSTERIQUES D'UN RECEPTEUR**
VERFAHREN ZUM NACHWEIS VON ALLOSTEREN EFFEKTOREN EINES REZEPTORS
METHOD FOR IDENTIFYING AN ALLOSTERIC EFFECTOR OF A RECEPTOR

(30) Priorité: 17.06.2002 FR 0207436
(43) Date de publication de la demande: 16.03.2005
(73) Titulaire: CENTRE NATIONAL DE LA RECHERCHE SCIENTIFIQUE, 75974 Paris (FR); UNIVERSITE LOUIS PASTEUR DE STRASBOURG, F-67084 Strasbourg Cédex (FR)
(72) Inventeur: GALZI, Jean-Luc, F-67100 Strasbourg (FR); HIBERT, Marcel, F-67114 Eschau (FR); BOURGUIGNON, Jean-Jacques, F-67150 Hipsheim (FR); MAILLET, Emeline, F- 67000 Strasbourg (FR)
(74) Mandataire: Grosset-Fournier, Chantal Catherine
(86) Numéro de dépôt international: PCT/FR2003/001817
(87) Numéro de publication internationale: WO 2003/107004

(56) Documents cités:
- WO-A-98/55873
- REES STEPHEN ET AL: "GPCR drug discovery through the exploitation of allosteric drug binding sites." RECEPTORS & CHANNELS. ENGLAND 2002, vol. 8, no. 5-6, 2002, pages 261-268, XP008017101 ISSN: 1060-6823
- PALANCHE TANIA ET AL: "The neurokinin A receptor activates calcium and cAMP responses through distinct conformational states." JOURNAL OF BIOLOGICAL CHEMISTRY, vol. 276, no. 37, 14 septembre 2001 (2001-09-14), pages 34853-34861, XP002241194 ISSN: 0021-9258 cité dans la demande
- VOLLMER JEAN-YVES ET AL: "Subcellular compartmentalization of activation and desensitization of responses mediated by NK2 neurokinin receptors." JOURNAL OF BIOLOGICAL CHEMISTRY., vol. 274, no. 53, 31 décembre 1999 (1999-12-31), pages 37915-37922, XP002241195 ISSN: 0021-9258 cité dans la demande
- PRINCE RICHARD J ET AL: "Acetylcholine and epibatidine binding to muscle acetylcholine receptors distinguish between concerted and uncoupled models." JOURNAL OF BIOLOGICAL CHEMISTRY, vol. 274, no. 28, 9 juillet 1999 (1999-07-09), pages 19623-19629, XP002241197 ISSN: 0021-9258
- LECAT S ET AL: "Dual responses of the NK2 tachykinin receptor are mediated by multiple conformational states." SOCIETY FOR NEUROSCIENCE ABSTRACTS, vol. 27, no. 1, 2001, page 389, XP001151939 31st Annual Meeting of the Society for Neuroscience;San Diego, California, USA; November 10-15, 2001 ISSN: 0190-5295
- ANGELIDES K J ET AL: "Fluorescence resonance energy transfer on the voltage-dependent sodium channel. Spatial relationship and site coupling between the batrachotoxin and Leiurus quinquestriatus quinquestriatus alpha-scorpion toxin receptors." THE JOURNAL OF BIOLOGICAL CHEMISTRY. UNITED STATES 25 MAY 1984, vol. 259, no. 10, 25 mai 1984 (1984-05-25), pages 6117-6126, XP002241196 ISSN: 0021-9258
- CROCI T ET AL: "IN VITRO CHARACTERIZATION OF TACHYKININ NK2-RECEPTORS MODULATING MOTOR RESPONSES OF HUMAN COLONIC MUSCLE STRIPS" BRITISH JOURNAL OF PHARMACOLOGY, BASINGSTOKE, HANTS, GB, vol. 124, no. 6, juillet 1998 (1998-07), pages 1321-1327, XP000937432 ISSN: 0007-1188
- ILIEN BRIGITTE ET AL: "Fluorescence resonance energy transfer to probe human M1 muscarinic receptor structure and drug binding properties." JOURNAL OF NEUROCHEMISTRY, vol. 85, no. 3, mai 2003 (2003-05), pages 768-778, XP002265260 ISSN: 0022-3042 cité dans la demande

## Description

La présente invention a pour objet un procédé de mise en évidence d'un effecteur allostérique d'un récepteur.

De nombreux médicaments et substances naturelles exercent leur action en interagissant avec des protéines régulatrices, appelées récepteurs, impliquées dans de nombreuses fonctions physiologiques des organismes, et les altérations de leurs fonctions sont la cause de nombreuses pathologies.

Parmi les molécules qui agissent en se liant à des récepteurs, une catégorie particulière, appelée effecteurs allostériques ou modulateurs, exerce son effet en se liant à des sites, présents à la surface des récepteurs, mais topologiquement distincts du site de liaison du ligand endogène. Leur interaction avec le récepteur est de nature non compétitive par rapport à celle du ligand endogène. En se liant à leurs sites régulateurs, les effecteurs n'ont pas ou peu d'effet sur les réponses du récepteur en absence de ligand endogène. En revanche, ils modifient les propriétés de liaison des ligands endogènes compétitifs et altèrent les réponses biologiques évoquées par ledit ligand.

Les effecteurs allostériques présentent un intérêt particulier dans le domaine thérapeutique par le fait que leur action n'est pas "constitutive" mais se révèle uniquement lorsque le ligand endogène vient stimuler le récepteur. Dans les cas où de telles molécules ont pu être identifiées et mises sur le marché, le confort du patient et la sélectivité de l'effet se sont toujours trouvés grandement améliorés par exemple en comparaison avec l'effet observé de l'administration d'un agoniste (Pan et al., 2001).

Afin d'identifier de nouveaux effecteurs allostériques de récepteurs, plusieurs approches expérimentales ont été développées à ce jour, notamment les mesures de liaison d'un ligand à son récepteur et les mesures fonctionnelles de réponses physiologiques.

Parmi les méthodes de mesure de liaison, il y a l'étude de la cinétique de dissociation d'un radioligand. Comme décrit dans Kostenis et Mohr (1996), la modulation allostérique est le plus généralement mise en évidence par un effet de l'effecteur allostérique sur la cinétique de dissociation d'un radioligand. Cette méthode a été mise en oeuvre pour démontrer le caractère non compétitif (et donc allostérique) de molécules régulant les récepteurs M1 à M5 muscariniques de l'acétylcholine (Tucek et Poska, 1995), A1 de l'adénosine (Bruns & Ferguns, 1990), alpha2 de l'adrénaline (Nunnari et al., 1987), ou D2 de la dopamine (Hoare & Strange, 1995). Dans ces différents exemples, les auteurs ont indifféremment utilisé soit des agonistes, soit des antagonistes comme radioligands, et ont défini le caractère positif ou négatif du modulateur par sa capacité à accélérer (positif) ou ralentir (négatif) la vitesse de dissociation du radioligand utilisé.

La deuxième approche la plus répandue d'identification d'un effecteur allostérique consiste à mesurer la quantité de complexe radioligand / récepteur à une concentration subsaturante de radioligand. Dans ces conditions, le fait qu'une tierce molécule provoque une augmentation de la quantité de complexe radioligand / récepteur s'interprète en termes de potentialisation de la liaison par un effet allostérique. Le travail de Bruns et Ferguns, cité plus haut, illustre ce point lorsqu'un radioligand agoniste est utilisé, tandis que l'article de Jakubik et al. (1997) utilise un antagoniste radioactif.

La troisième approche expérimentale consiste à établir la courbe de saturation d'un radoligand en présence et en absence d'effecteur allostérique. Dans ce cas, qui est illustré dans l'article de Massot et al. (1996) pour le récepteur 5HT1B de la sérotonine, l'effecteur 5HT-moduline réduit la quantité de sites récepteurs sans affecter l'affinité apparente déterminée pour le radioligand utilisé.

Les mesures de la réponse biologique évoquée par un ligand de récepteur comprennent soit son activation (par les agonistes) soit son inhibition (par les antagonistes). Ces mesures estiment généralement une variation d'amplitude des réponses qui sont soit potentialisées par les effecteurs positifs ou inhibées par les effecteurs négatifs de la réponse, et incluent :
- l'enregistrement de courants ioniques par des méthodes électrophysiologiques (Krause et al., 1997 ; Galzi et al., 1996) ou par des méthodes optiques utilisant des sondes spécifiques d'ions (Birdsall et al., 1999),
- la mesure de la production de messagers secondaires tels que l'AMPc (Bruns & Ferguns, 1990) ou les inositols phosphates (Waugh et al., 1999),
- la liaison de GTP à la protéine G associée au récepteur (Birdsall et al., 1999; Lazareno & Birdsall, 1995 ; Hoare et al., 2000) ou l'hydrolyse de GTP par la protéine G associée au récepteur (Birdsall et al., 1999). Rees et al., 2002, vol. 8, no. 5-6 pages 261-268 présente un résumé des différentes techniques d'identification d'un effecteur allostérique.

La technique du transfert d'énergie de fluorescence (FRET) en combinaison avec l'utilisation d'un récepteur rendu fluorescent et de l'un de ses ligands fluorescents permet de mesurer, comme décrit dans la demande internationale WO 98/55873, des interactions non covalentes entre ledit récepteur et ledit ligand.

Le récepteur peut être rendu fluorescent grâce à une protéine fluorescente, par exemple une protéine issue de la méduse Aequorea victoria dont le gène correspondant a été séquencé, cloné (Prasher et al., 1992) et optimisé pour une bonne expression chez les eucaryotes supérieurs (Cormack et al., 1996). Il peut être fusionné avec le gène codant pour une autre protéine, notamment un récepteur couplé à une protéine G. (Galzi & Alix, 1997 ; Weill et al., 1999 ; Vollmer et al., 1999 ; Valenzuela et al., 2001) pour permettre l'expression d'une protéine réceptrice fluorescente. La combinaison de la protéine réceptrice fluorescente avec l'un de ses ligands fluorescents permet la formation d'un complexe récepteur-ligand non covalent qui est détecté par transfert d'énergie de fluorescence (Galzi & Alix, 1997 ; Vollmer et al., 1999 ; Valenzuela et al., 2001) et dont on peut mesurer la cinétique de formation et de dissociation (Palanché et al., 2001 ; Valenzuela et al., 2001).

L'un des buts de l'invention est de fournir un procédé simple à mettre en oeuvre, rapide, sensible, permettant d'effectuer des mesures quantitatives des interactions entre un récepteur et un ligand marqueur du site du ligand endogène, et permettant de détecter la présence d'un éventuel effecteur allostérique.

Un autre but de l'invention est de permettre des mesures en temps réel des interactions d'un ligand marqueur du site du ligand endogène avec son récepteur, permettant de détecter la présence d'un éventuel effecteur allostérique.

Un autre but de l'invention est de permettre une mesure en temps résolu de la cinétique d'association et de dissociation d'un ligand fluorescent, marqueur du site du ligand endogène, avec son récepteur, et de détecter la présence d'un éventuel effecteur allostérique.

Un autre but de l'invention est de permettre la détection de l'interaction entre un récepteur et son ligand endogène par transfert d'énergie de fluorescence dans des gammes de concentrations de ligand endogène fluorescent considérablement plus étendues (du nanomolaire au micromolaire) que lorsqu'on utilise un radioligand (domaine du nanomolaire). Ainsi, la présente invention permet de détecter par transfert d'énergie un plus grand nombre d'états conformationnels du récepteur, ce qui facilite grandement l'identification et l'interprétation des effets d'agents modulateurs supposés.

L'un des buts de l'invention est de fournir une mesure expérimentale des constantes de vitesse d'association et de dissociation d'un ligand fluorescent, marqueur du site du ligand endogène, avec son récepteur, en permettant la détection de la présence d'un éventuel effecteur allostérique.

L'invention a également pour but de fournir un procédé de détection d'un effecteur allostérique d'un récepteur, généralisable à de nombreuses protéines réceptrices et à leurs ligands.

L'invention a également pour but de fournir un procédé de détection d'un effecteur allostérique d'un récepteur, automatisable, ne nécessitant ni la purification du récepteur, ni celle du ligand.

L'invention a également pour but de fournir un procédé de détection d'un effecteur allostérique d'un récepteur, non polluant puisqu'il n'utilise pas de radioactivité, économique puisqu'il utilise de la lumière visible et peut être mis en oeuvre sur des équipements existants.

La présente invention concerne un procédé de mise en évidence d'un effecteur allostérique d'un récepteur, par détermination de la variation :
- de la cinétique de dissociation et/ou d'association du complexe formé entre le susdit récepteur et l'un de ses ligands en présence dudit effecteur allostérique, par rapport à la cinétique de dissociation et/ou d'association du complexe formé entre ledit récepteur et ledit ligand, en l'absence dudit effecteur,
- et/ou de l'amplitude de la liaison formée entre le susdit récepteur et l'un de ses ligands en présence dudit effecteur allostérique, par rapport à l'amplitude de la liaison formée entre ledit récepteur et ledit ligand, en l'absence dudit effecteur,
   ledit récepteur et ledit ligand étant impliqués dans au moins une réponse biologique dans des conditions physiologiques appropriées, et l'effecteur allostérique étant capable de moduler au moins l'une des réponses,
   ledit récepteur étant marqué par une protéine fluorescente choisie parmi les protéines fluorescentes issues ou dérivées de protéines autofluorescentes de cnidaires, dont le coefficient d'extinction moléculaire est supérieur à environ 14000M⁻¹.cm⁻¹ et le rendement quantique de fluorescence est supérieur à environ 0,38,
   ledit ligand étant marqué par un marqueur constitué :
   - soit par une molécule susceptible d'absorber la lumière émise par la protéine fluorescente,
   - soit par une substance fluorescente,
   lesdites déterminations de variation de cinétique de dissociation et/ou d'association et de variation d'amplitude étant effectuées par transfert d'énergie de fluorescence :
   - entre la protéine fluorescente susmentionnée et la susdite substance fluorescente, la substance fluorescente étant telle que soit elle est excitable à la longueur d'onde d'émission de la protéine fluorescente susmentionnée, soit elle émet à la longueur d'excitation de la protéine fluorescente susmentionnée, ou
   - entre la protéine fluorescente susmentionnée et la susdite molécule susceptible d'absorber la lumière émise par la protéine fluorescente.

La présente invention concerne un procédé de mise en évidence d'un effecteur allostérique d'un récepteur, par détermination de la variation :
- de la cinétique de dissociation et/ou d'association du complexe formé entre le susdit récepteur et l'un de ses ligands en présence dudit effecteur allostérique, par rapport à la cinétique de dissociation et/ou d'association du complexe formé entre ledit récepteur et ledit ligand, en l'absence dudit effecteur,
- et/ou de l'amplitude de la liaison formée entre le susdit récepteur et l'un de ses ligands en présence dudit effecteur allostérique, par rapport à l'amplitude de la liaison formée entre ledit récepteur et ledit ligand, en l'absence dudit effecteur, sous réserve que lorsque la variation de la susdite amplitude est négative, on détecte également l'existence de la variation de la susdite cinétique,
   ledit récepteur et ledit ligand étant impliqués dans au moins une réponse biologique dans des conditions physiologiques appropriées, et l'effecteur allostérique étant capable de moduler au moins l'une des réponses,
   ledit récepteur étant marqué par une protéine fluorescente choisie parmi les protéines fluorescentes issues ou dérivées de protéines autofluorescentes de cnidaires, dont le coefficient d'extinction moléculaire est supérieur à environ 14000M⁻¹.cm⁻¹ et le rendement quantique de fluorescence est supérieur à environ 0,38,
   ledit ligand étant marqué par un marqueur constitué :
   - soit par une molécule susceptible d'absorber la lumière émise par la protéine fluorescente,
   - soit par une substance fluorescente,
   lesdites déterminations de variation de cinétique de dissociation et/ou d'association et de variation d'amplitude étant effectuées par transfert d'énergie de fluorescence :
   - entre la protéine fluorescente susmentionnée et la susdite substance fluorescente, la substance fluorescente étant telle que soit elle est excitable à la longueur d'onde d'émission de la protéine fluorescente susmentionnée, soit elle émet à la longueur d'excitation de la protéine fluorescente susmentionnée, ou
   - entre la protéine fluorescente susmentionnée et la susdite molécule susceptible d'absorber la lumière émise par la protéine fluorescente.

L'invention répond au problème technique posé par la détermination du caractère compétitif ou non compétitif de l'interaction entre un récepteur et un agent pharmacologique supposé agir comme effecteur d'un récepteur ou comme compétiteur du ligand endogène d'un récepteur, par des mesures de cinétiques d'association et de dissociation. De manière simple, l'effecteur allostérique d'un récepteur altérera la cinétique d'association ou de dissociation d'un récepteur tandis qu'un agent compétiteur du ligand endogène n'aura pas d'effet sur ces cinétiques.

On appelle "ligand, marqueur du site du ligand endogène" un ligand qui se lie au site du ligand endogène par opposition à un éventuel effecteur allostérique, qui se lie à un site distinct de celui du ligand endogène. Le marqueur du site du ligand endogène interagit de manière compétitive avec le ligand endogène (liaison mutuellement exclusive), tandis que l'effecteur allostérique interagit de manière non compétitive avec le ligand endogène.

On appelle "effecteur allostérique" ou "modulateur allostérique" une molécule ou un ligand ou une substance biologiquement active qui module les propriétés de liaison du ligand ainsi que les propriétés fonctionnelles du récepteur, sans entrer en compétition avec ledit ligand.

Par définition, on rappelle qu'on nomme ligand toute molécule susceptible de se lier à un récepteur de manière non covalente en se liant au même site que le ligand endogène, et qu'un ligand peut être un agoniste ou un antagoniste, un agoniste étant capable, en se liant, de déclencher une réponse biologique et un antagoniste ne déclenchant pas de réponse biologique et étant capable de bloquer l'effet de l'agoniste.

Un effecteur allostérique est aussi un ligand du récepteur mais celui-ci se lie avec le récepteur sur un autre site de liaison. Dans le présent contexte, sera utilisé le terme "effecteur allostérique" ou "modulateur allostérique" et non pas "ligand".

La modulation allostérique est le mécanisme par lequel une molécule, appelée effecteur allostérique ou modulateur allostérique, augmente ou diminue la réponse d'un récepteur activé par un agoniste. L'effecteur allostérique interagit avec le récepteur au niveau d'un site de liaison qui est distinct de celui auquel le ligand compétitif se lie sur le récepteur. Le fait qu'une modulation allostérique soit possible réside dans :
1) l'existence de différentes conformations ou états du récepteur. Chaque conformation est dotée de propriétés pharmacologiques et fonctionnelles distinctes. Il existe ainsi des conformations actives et inactives sur le plan biologique. Chaque conformation lie ses ligands avec des affinités différentes. Par exemple, un agoniste, tout comme un effecteur allostérique positif, exhibe une affinité plus grande pour la (les) conformation(s) biologiquement active(s) du récepteur, tandis qu'un antagoniste, tout comme un effecteur allostérique négatif, présente une affinité plus élevée pour la (les) conformation(s) inactive(s) du récepteur ;
   Les propriétés pharmacologiques d'une conformation sont la capacité de lier des ligands et modulateurs avec une affinité définie. Chaque conformation lie différents ligands avec une affinité intrinsèque propre à chaque ligand. L'ensemble des ligands se liant à une conformation avec une affinité propre constitue ce que l'on appelle profil pharmacologique. Les propriétés pharmacologiques d'une conformation sont assimilables à son profil pharmacologique.
   Les propriétés fonctionnelles d'une conformation d'un récepteur correspondent à sa capacité ou non de stimuler une réponse biologique. On distinguera plusieurs propriétés fonctionnelles d'un récepteur :
   - l'état de repos, par définition non actif, car non couplé à une réponse, mais activable, c'est à dire, capable de s'interconvertir dans une conformation active ou couplée à une réponse ;
   - le ou les état(s) actif(s), responsable(s) d'une réponse biologique ; dans le cas où il existe plusieurs états actifs, l'une au moins des propriétés de la réponse sera différente (par exemple, réponse calcique versus cAMP, ou alors même réponse mais sensibilité différente aux agonistes, ou encore durée de réponses différentes etc....) ; d'autre part, chaque état actif peut donner lieu à une ou plusieurs réponses, distinctes par leur nature ou d'un point de vue quantitatif; de plus, un récepteur peut adopter plusieurs conformations correspondant à plusieurs états actifs auxquels sont associées des réponses de nature différente ;
   - le(s) état(s) désensibilisé(s) qui sont non actifs et non activables ;
2) l'existence, sur les protéines, de sites multiples qui permettent l'interaction simultanée de plusieurs molécules avec un seul récepteur ; les ligands compétitifs interagissent avec un site commun (de manière mutuellement exclusive) qui lui-même est distinct du (des) site(s) d'interaction des effecteurs ou modulateurs allostériques ;
3) les transitions conformationnelles du récepteur impliquent la totalité de la molécule de récepteur et ne sont pas restreintes à la région de la molécule qui lie les ligands ; elles affectent toute la structure tertiaire, voire quaternaire, de la protéine, et sont discrètes ; ainsi, lors de la transition conformationnelle de l'état de repos vers l'état actif du récepteur, la totalité des sites de liaison ainsi que celle du site "biologiquement actif" voient leur structure changer d'une manière concertée.

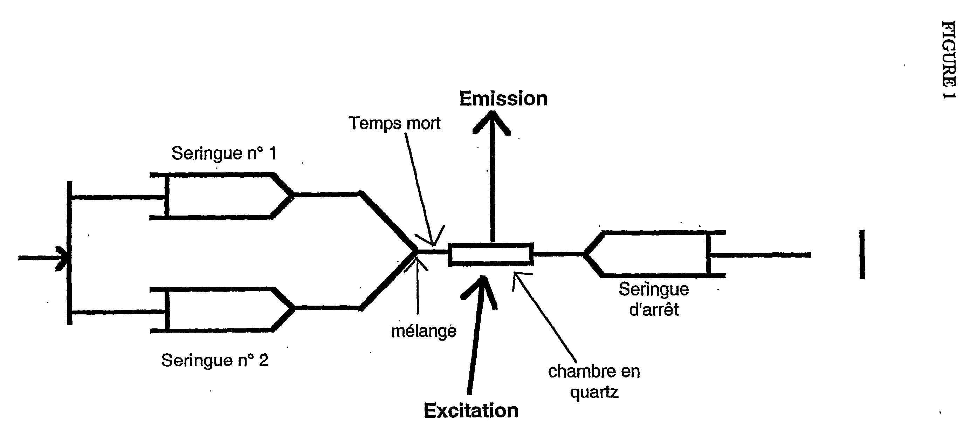

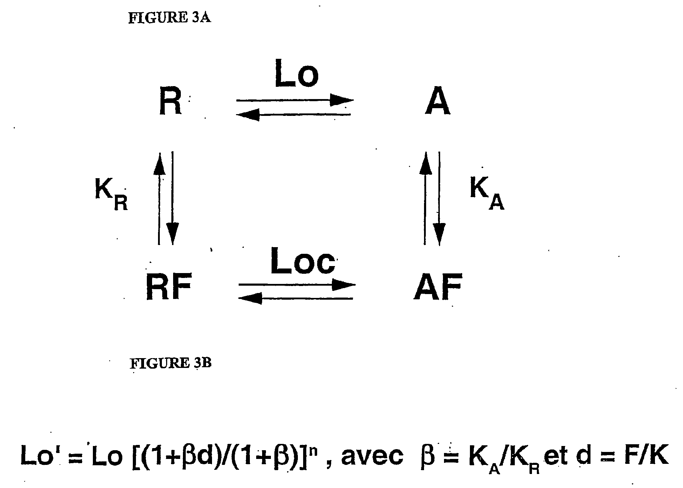

Ces différents aspects de la régulation fonctionnelle d'une protéine sont illustrés dans la figure 1 (Monod et al., 1965 ; Galzi et al., 1996 ; Rubin et Changeux, 1966). La description du mode de fonctionnement d'un récepteur pouvant exister dans deux conformations (état de repos et état actif) est illustrée en figure 3 pour un récepteur. Une version équivalente de ce schéma pour un récepteur couplé aux protéines G pouvant activer une protéine Gs, elle-même responsable de l'activation d'une adénylate cyclase, est décrite dans Tucek et Proska (1995) et dans Hall (2000).

Dans ces schémas, la protéine, en particulier le récepteur, existe dans deux conformations qui sont en équilibre spontané l'une avec l'autre. Cet équilibre est décrit par la constante L₀ dont la valeur est donnée par le rapport de concentration fractionnelle [R]/[A], R étant l'état de repos et A, l'état actif. Les ligands se lient à l'état R et à l'état A avec des affinités décrites par les constantes de dissociation K_{R} et K_{A}, respectivement. Si le rapport c = K_{A}/K_{R} est < 1, c'est à dire que l'affinité du ligand est meilleure pour A que pour R, ledit ligand se comporte comme un agoniste du récepteur et la concentration fractionnelle de récepteur dans l'état A sera décrite par le produit L₀c qui sera lui-même plus petit que L₀. Inversement, si le rapport d'affinité c est > 1, le ligand se liera préférentiellement à l'état R et aura tendance à se comporter comme un antagoniste. Le produit L₀c sera plus grand que L₀. Les effecteurs allostériques, en se liant à des sites distincts de celui des ligands compétitifs, se comportent de la même manière que les ligands compétitifs en ce qu'ils modifient la valeur de la constante d'équilibre entre les états R et A. Le formalisme mathématique de l'effet des effecteurs allostériques est donné dans Rubin et Changeux (1966). La présence d'un effecteur allostérique modifie la valeur de la constante L₀ en L'o selon L'₀ = L₀ [(1+βd)/(1+β)]ⁿ avec β = K_{A}/K_{R} et d = F/K_{A}, F correspondant à la concentration d'effecteur allostérique. L'ajout d'un agoniste ou d'un antagoniste entraîne alors le changement des concentrations fractionnelles des états du récepteur en L'₀c.

On rappelle les définitions suivantes :

On désigne par "récepteur" toute molécule de nature protéique susceptible d'entrer en interaction non covalente avec un agent pharmacologique. Préférentiellement, dans l'invention, on utilise un récepteur de neurotransmetteur, d'hormone, de facteur de croissance etc.... capable de produire, après interaction avec un ligand pharmacologique, une réponse de transduction de signal mesurable *in vivo* et *in vitro.*

On désigne par "interaction compétitive" une interaction de deux molécules avec un récepteur prenant place au niveau d'un site unique, et un ligand est dit "compétitif' lorsqu'il interfère avec la liaison d'un autre ligand de manière stérique.

Parallèlement, on désigne par "interaction non compétitive" une interaction de deux molécules avec un récepteur prenant place au niveau de sites topologiquement distincts, et un ligand est dit "non compétitif' lorsqu'il interfère avec la liaison d'un autre ligand, les deux ligands interagissant avec des sites topologiquement distincts du récepteur.

On rappelle également que l' "association" dans l'expression "association du complexe" est l'action pour un ligand de se lier à une protéine réceptrice.

Si la protéine réceptrice peut adopter plusieurs conformations ou états, il est parfois possible de discerner la liaison d'un ligand aux différentes conformations par des différences de cinétiques d'association.

On désigne par "cinétique d'association" le décours temporel d'une réaction d'association. Les cinétiques peuvent être soit monoexponentielles, soit pluriexponentielles. Dans le cas où elles sont pluriexponentielles, elles se décomposent en une somme de relaxations monoexponentielles caractérisées chacune par une vitesse d'association et une amplitude.

La "vitesse d'association" est mesurée au moyen de la constante de vitesse de la réaction d'association obtenue par ajustement d'une courbe expérimentale à l'aide d'une expression monoexponentielle de la forme y = λ exp (-kₐₚₚ × T) où λ est l'amplitude, kₐₚₚ est la constante de vitesse apparente de la réaction et T est le temps. Dans le cas ou l'association reflète plusieurs évènements de liaison simultanés, on utilise une expression multiexponentielle de type y = λ₁ exp (-k1ₐₚₚ × T) + λ₂ exp (-k2ₐₚₚ × T)+ ...

On rappelle que l'expression mathématique de la constante de vitesse apparente (kₐₚₚ) dépend de la nature linéaire ou non de la variation de kₐₚₚ avec la concentration de ligand [L]. Ainsi, si kₐₚₚ varie de manière linéaire avec [L], la réaction est bimoléculaire du type R + L ↔ RL avec k₁ pour la formation et k₋₁ pour la dissociation du complexe. kₐₚₚ est alors égale à k₁ × [L] + k₋₁. Si la relation est non linéaire, on applique le schéma réactionnel suivant : R + L ↔ RL ↔ R'L où K_{D} est la constante de dissociation du complexe RL, k₂ est la vitesse d'interconversion RL → R'L et k-₂ est la constante de vitesse de la conversion R'L → RL, kₐₚₚ est alors égal à k₂ × ([L]/([L] + K_{D}))+ k₋₂.

On rappelle également que la "dissociation" dans l'expression "dissociation du complexe" est l'action pour un ligand de quitter un site récepteur. La dissociation peut être obtenue d'au moins deux manières : a) en diluant fortement le mélange récepteur-ligand de façon à privilégier la dissociation par rapport à l'association ou 2) en ajoutant en fort excès un ligand compétitif qui occupera de manière privilégiée un site laissé vacant par le ligand qui s'est dissocié.

Si la protéine réceptrice peut adopter plusieurs conformations ou états, il est parfois possible de discerner la dissociation entre un ligand et son récepteur présentant différentes conformations, par des différences de cinétique de dissociation entre les différentes conformations.

On désigne par cinétique de dissociation le décours temporel d'une réaction de dissociation. Les cinétiques peuvent être soit monoexponentielles, soit pluriexponentielles. Dans le cas où elles sont pluriexponentielles, elles se décomposent en un somme de relaxations monoexponentielles caractérisées par une vitesse de dissociation et une amplitude.

La "vitesse de dissociation" est mesurée au moyen de la constante de vitesse de la réaction de dissociation obtenue par ajustement d'une courbe expérimentale à l'aide d'une expression exponentielle de la forme y = λ exp(-k × T) où λ est l'amplitude, k est la constante de vitesse intrinsèque de la réaction (k = k₋₁) et T est le temps. Dans le cas ou la dissociation reflète plusieurs évènements de dissociation simultanés, on utilise une expression multiexponentielle de type y = λ₁ exp (-k₁ × T) + λ₂ exp (-k₂ × T)+ ...

On rappelle que l'expression "amplitude de la liaison" désigne l'amplitude du signal enregistré, qui est lui-même proportionnel au taux d'occupation des sites récepteurs. À concentration saturante de ligand, l'amplitude de liaison est constante. En deçà, elle évolue de manière non linéaire, et selon la loi d'action de masse. Elle peut être décrite par l'équation empirique de Hill : RL = R₀/(1+K_{D}/L)ⁿ où R₀ est la quantité totale de sites récepteurs présents dans l'essai, K_{D} est la constante de dissociation, L est la concentration du ligand, et où n est le coefficient de proportionnalité aussi appelé coefficient de Hill.

On appelle "réponse biologique" toute variation de métabolisme de cellules, tissus ou organismes. Pour des cellules, par exemple, on pourra déterminer des variations de pH, de concentration ionique, de formation de métabolites tels que le GTP ou l'AMPc, d'expression de gènes, de morphologie cellulaire (mesure du pourcentage de cellules qui ont changé de forme), de prolifération cellulaire, entre autres. Des exemples sont donnés plus loin.

Par "conditions physiologiques appropriées", on désigne toutes conditions de pH, de concentrations et de composition ioniques, de compléments nutritifs aussi proches que possibles de celles qui sont rencontrées dans l'organisme entier. Ces conditions seront choisies de manière à ce que l'expérience effectuée soit réalisée dans des conditions aussi proches que possibles de celles que l'on pourrait obtenir en effectuant la mesure chez l'organisme entier.

L'expression "capable de moduler au moins l'une des réponses" signifie que dans un ensemble de réponses mesurables et stimulables par un récepteur, l'une au moins doit être modifiée par l'effecteur. La modification ou modulation peut porter sur le délai d'établissement de la réponse, sa fréquence, son amplitude, sa durée, sa vitesse d'extinction, ainsi que sa sensibilité à l'agoniste.

S'agissant de la réponse de transduction de signal pour les récepteurs couplés aux protéines G, le test général consiste à déterminer l'activation de la protéine G par mesure de la fixation de GTP (Befort et al., 1996). D'autres mesures plus spécifiques font par exemple intervenir des déterminations de concentrations intracellulaires d'AMPc, d'inositol phosphates, de calcium, des mesures d'activation de la transcription de gènes ou d'activité oncogénique, suivant le type de couplage spécifique du récepteur considéré.

Pour les récepteurs-canaux, les mesures les plus directes sont des déterminations de courants ioniques (Hille, 1992). D'autres mesures peuvent, par exemple, faire intervenir des déterminations de transcription de gènes ou des activations d'enzymes.

Pour les récepteurs de facteurs de croissance, les tests généraux sont ceux de la prolifération, de différentiation ou de la survie cellulaire, fréquemment aussi des tests de phosphorylations de substrats spécifiques (Honneger et al., 1988) de chaque récepteur et repérage par des anticorps spécifiques de phosphoaminoacides.

Pour les récepteurs nucléaires, les tests de transduction de signal sont ceux de la transcription de gènes dans lesquels des gènes rapporteurs, par exemple "chromogéniques" sont placés sous le contrôle de promoteurs spécifiques des voies de transduction du récepteur étudié (Ko et al., 2002).

Ainsi, pour les réponses d'accumulation d'AMPc, on pourra employer divers protocoles, notamment une mesure radioimmunologique telle que décrite dans Palanché et al. (2001) ou Hausdorff et al. (1990).

La liaison de GTP à la protéine G pourra être enregistrée par exemple comme décrit dans Fawzi et al. (2001) ou Vuong et Chabre (1990).

La prolifération cellulaire sera par exemple analysée selon le protocole décrit dans Burstein et al (1997).

La régulation de l'expression de gènes pourra par exemple être étudiée selon le protocole décrit dans Baulmann et al. (2000).

L'activation de protéines kinases pourra par exemple être étudiée comme décrit dans Vollmer et al. (1999) ou Yuan et al. (2000).

La variation de pH pourra être mesurée (Nicolini et al., 1995).

Une "protéine autofluorescente" est une protéine naturelle ou synthétique dans laquelle le chromophore se forme par une réaction autocatalytique entre des acides aminés de la protéine sans nécessiter l'addition d'un groupe prosthétique (chromophore), et dont les propriétés de fluorescence sont intrinsèques au monomère.

L'expression "transfert d'énergie de fluorescence" correspond à un processus physique, dépendant de la distance, par lequel de l'énergie est transmise de manière non radiative d'un chromophore excité, le donneur, à un autre chromophore, l'accepteur, par interaction dipôle-dipôle (Förster, 1951 ; Wu and Brand, 1994; Clegg, 1995). Le transfert d'énergie peut être observé soit par une diminution de l'amplitude de l'émission du donneur, soit par une augmentation de l'amplitude de l'excitation et de l'émission de l'accepteur. Si l'accepteur n'est pas fluorescent, mais présente un spectre d'excitation recouvrant au moins en partie le spectre d'émission du donneur, le transfert d'énergie pourra être détecté sous la forme d'une réduction d'amplitude de l'émission du donneur.

Dans le cas de l'application du transfert d'énergie à des échantillons biologiques en interaction non covalentes, le signal de transfert ne peut pas persister si les conditions expérimentales ne permettent pas l'interaction entre le ligand fluorescent et le récepteur fluorescent. De même, si l'un des deux partenaires interagissant n'est pas porteur d'un chromophore adéquat, les éventuelles variations de fluorescence observées pour l'autre partenaire ne pourront pas être attribuées à un processus de transfert d'énergie.

Les termes "changement" ou "variation de fluorescence", définis dans le contexte du transfert d'énergie, se réfèrent à toutes modifications de 1) l'amplitude du signal de fluorescence de l'accepteur, de 2) l'amplitude du spectre d'excitation ou 3) l'amplitude du signal d'émission du donneur. Les variations ou changements de fluorescence ne doivent pas être observés si l'un des deux partenaires ne porte pas de chromophore ou de fluorophore adéquat (voir infra) ou si l'interaction entre les partenaires fluorescents est inhibée, par exemple par un excès d'un agent compétiteur.

De façon plus précise, la réaction de transfert d'énergie de fluorescence requiert deux groupes, l'un appelé donneur, nécessairement fluorescent, et l'autre accepteur, soit fluorescent, soit colorant. Cette réaction se produit lorsque deux conditions sont réunies :
1) le spectre d'absorption de l'accepteur et le spectre d'émission du donneur doivent se recouvrir, au moins en partie ; le recouvrement se calcule à partir de données expérimentales et d'une équation donnant une valeur en cm³M⁻¹ (Lakey et al., 1991) ;
2) le donneur et l'accepteur doivent être proches dans l'espace (de 10 à 100 angströms) afin que le transfert d'énergie puisse avoir lieu.

La première condition a pour conséquence le fait que l'excitation du donneur entraîne alors de manière concomitante une diminution de l'amplitude de l'émission du donneur et l'apparition d'un signal d'émission de l'accepteur. Ceci permet de détecter les interactions entre le donneur et l'accepteur et/ou de mesurer leur distance.

L'expression "proches dans l'espace" signifie que la distance entre le donneur et l'accepteur est inférieure à 2 Ro, Ro représentant le rayon de Forster (op.cit.) (Lakey et al., 1991).

Selon un mode de réalisation avantageux de l' invention, lorsque la variation de l'amplitude de la liaison formée entre le susdit récepteur et l'un de ses ligands en présence dudit effecteur allostérique, par rapport à l'amplitude de la liaison formé entre ledit récepteur et ledit ligand, en l'absence dudit effecteur, est négative, il convient de détecter l'existence d' une variation de la cinétique de dissociation et/ ou d' association du complexe formé entre le susdit récepteur et l'un de ses ligands en présence dudit effecteur allostérique, par rapport à la cinétique de dissociation et/ou d' association du complexe formé entre ledit récepteur et ledit ligand, en l'absence dudit effecteur, et avantageusement de quantifier cette variation de cinétique, afin de distinguer un effecteur allostérique d'un agent compétitif.

La présente invention concerne un procédé de mise en évidence d'un effecteur allostérique d'un récepteur, par détermination de la variation :
- de la cinétique de dissociation et/ou d'association du complexe formé entre le susdit récepteur et l'un de ses ligands en présence dudit effecteur allostérique, par rapport à la cinétique de dissociation et/ou d'association du complexe formé entre ledit récepteur et ledit ligand, en l'absence dudit effecteur,
- et/ou de l'amplitude de la liaison formée entre le susdit récepteur et l'un de ses ligands en présence dudit effecteur allostérique, par rapport à l'amplitude de la liaison formée entre ledit récepteur et ledit ligand, en l'absence dudit effecteur,
   ledit récepteur et ledit ligand étant impliqués dans au moins une réponse biologique dans des conditions physiologiques appropriées, et l'effecteur allostérique étant capable de moduler au moins l'une des réponses,
   ledit récepteur étant marqué par une protéine fluorescente choisie parmi :
   - la protéine fluorescente verte (GFP ou EGFP), la protéine fluorescente cyan (CFP ou ECFP), la protéine fluorescente jaune (YFP ou EYFP) ou GFPUV,
   ou,
   - des variants dérivés de la GFP, de la CFP, de la YFP ou de la GFPUV, par addition, délétion ou substitution d'un ou plusieurs acides aminés, sous réserve que ces variants conservent la propriété de fluorescence,
   - ou des fragments de la GFP, de la CFP, de la YFP ou de la GFPUV, ou de fragments des susdits variants, sous réserve que ces fragments conservent la propriété de fluorescence,
   ledit ligand étant marqué par un marqueur constitué :
   - soit par une molécule susceptible d'absorber la lumière émise par la protéine fluorescente,
   - soit par une substance fluorescente,
   lesdites déterminations de variation de cinétique de dissociation et/ou d'association et de variation d'amplitude étant effectuées par transfert d'énergie de fluorescence :
   - entre la protéine fluorescente telle que définie ci-dessus et la susdite substance fluorescente, la substance fluorescente étant telle que soit elle est excitable à la longueur d'onde d'émission de la protéine fluorescente, soit elle émet à la longueur d'onde d'excitation de la protéine fluorescente, ou
   - entre la protéine fluorescente telle que définie ci-dessus et la susdite molécule susceptible d'absorber la lumière émise par la protéine fluorescente.

Selon un mode de réalisation préféré de l'invention, lorsque la variation de la susdite amplitude de la liaison formée entre le susdit récepteur et l'un de ses ligands en présence dudit effecteur allostérique, par rapport à l'amplitude de la liaison formée entre ledit récepteur et ledit ligand, en l'absence dudit effecteur, est négative, on détecte également l'existence de la variation de la susdite cinétique.

Le récepteur est marqué par voie génétique par une protéine fluorescente choisie parmi :
- la protéine fluorescente verte (GFP) (Ward et al., 1980 ; Chalfie, 1995), ou EGFP (Heim & Tsien, 1996 ; Miyawaki et al., 1997),
- la protéine fluorescente cyan (CFP ou ECFP) (Heim & Tsien, 1996 ; Miyawaki et al., 1997),
- la protéine fluorescente jaune (YFP ou EYFP) (Cormack et al., 1995 ; Heim, Cubitt et Tsien, 1995 ; Ehrig et al., 1995) (Miyawaki et al., 1997),
- GFPUV (Crameri et al., 1996 ; Ehrig et al., 1995),
ou leurs mutants dans lesquels les codons sont optimisés pour l'expression dans les cellules humaines, bactériennes ou végétales,
ou leurs mutants présentant des longueurs d'excitation ou d'émission plus élevées ou plus faibles que celles associées aux protéines définies ci-dessus, sous réserve que leur coefficient d'extinction moléculaire soit supérieur à environ 14000M⁻¹cm⁻¹ et. leur rendement quantique de fluorescence soit supérieur à environ 0;38.

L'expression "codons optimisés" indique la substitution de codons de la protéine sauvage par leurs homologues préférés de l'organisme hôte, sans changement de code donc sans changement de séquence protéique.

La GFP sauvage (WT) de longueur d'onde, d'excitation et d'émission 395/470-509 est décrite dans Ward et al. (1980) et Chalfie (1995).

La GFP UV présentant les mutations suivantes: F99S, M153T, V163A de longueur d'onde, d'excitation et d'émission respectivement de 395-510 est décrite dans Crameri et al. (1996), ou avec la mutation T203I et la longueur d'onde respectivement d'excitation et d'émission 400-512 est décrite dans Ehrig et al. (1995).

La EGFP présente les mutations suivantes :

| | | |
|---|---|---|
| F64L | S65T | H231L |

La EYFP présente les mutations suivantes :

| | | | |
|---|---|---|---|
| S65G | V68L | S72A | T203Y |

La ECFP présente les mutations suivantes :

| | | | |
|---|---|---|---|
| F64L | S65T | Y66W | N146I |
| M153T | V163A | N212K | |

Les différents mutants de GFP peuvent en outre être optimisés (par l'introduction de mutations silencieuses optimisant l'usage de codons spécifiques à chaque espèce) pour l'expression dans des cellules :
- humaines (Haas et al., 1996 ; Yan et al., 1996 ; Zolotukhin et al., 1996)
- bactériennes (Crameri et al., 1996 ; Cormack et al., 1996, pour *Escherichia coli*),
- végétales (Reichel et al., 1996).

Le terme GFP indique une protéine qui une fois exprimée dans des cellules émet une fluorescence. Les GFPs présentant des substitutions, additions ou délétions d'acides aminés influençant soit les propriétés de fluorescence, soit le taux d'expression de la GFP sont appelés mutants de GFP.

On donne ci-après les principales caractéristiques des protéines fluorescentes avantageusement utilisées dans le procédé de l'invention :

| Protéine | λ-excitation maximale | λ-émission | coefficient d'extinction | rendement quantique |
|---|---|---|---|---|
| EYFP | 514 | 527 | 36500 | 0,63 |
| ECFP | 432 | 480 | 18000 | 0,67 |
| GFPUV | 395 | 509 | 21000 | 0,77 |
| EGFP | 489 | 511 | 39000 | 0,66 |

La protéine autofluorescente BFP est de préférence exclue car elle ne répond pas aux conditions définies ici pour les protéines autofluorescentes de cnidaires, à savoir coefficient d'extinction moléculaire supérieur à 14000 M⁻¹cm⁻¹ et rendement quantique de fluorescence supérieur à 0,38.

Selon un mode de réalisation avantageux de l'invention, le récepteur est marqué par une protéine fluorescente (n° 1) et le ligand est marqué
* soit par une substance fluorescente, le marquage étant :
   - soit effectué par voie chimique, la substance fluorescente étant alors un composé chimique,
   - soit effectué par voie recombinante, la substance fluorescente étant alors un peptide ou une protéine fluorescente (n° 2), et pouvant être notamment choisie parmi les protéines fluorescentes issues ou dérivées de protéines autofluorescentes de cnidaires, dont le coefficient d'extinction moléculaire est supérieur à environ 14000M⁻¹cm⁻¹ et le rendement quantique de fluorescence est supérieur à environ 0,38, notamment choisie parmi :
      - la protéine fluorescente verte (GFP), ou
      - des variants dérivés de la GFP, par addition, délétion ou substitution d'un ou plusieurs acides aminés, sous réserve que ces variants conservent la propriété de fluorescence,
      - ou des fragments de la GFP, ou de fragments des susdits variants, sous réserve que ces fragments conservent la propriété de fluorescence,
* soit par une substance non fluorescente appartenant au groupe des acides violets [Acid Violet 5, CAS 10130-48-0 ; Acid Violet 7, CAS 4321-69-1 ; Acid Violet 17, CAS 4129-84-4], acides rouges [Acid Red 1, CAS 3734-67-6 ; Acid Red 8, CAS 4787-93-3 ; Acid Red 37, CAS 6360-07-2 ; Acid Red 40, CAS 12167-45-2 ; Acid Red 106, CAS 6844-74-2; Acid Red 114, CAS 6459-94-5], les alizarines, l'aluminon, l'azocarmine B [CAS 25360-72-9], la fuschine basique [Basic Red 9, CAS 569-61-9], le Bordeaux R [Acid Red 17, CAS 5858-33-3], 1a Carmine [CAS 1390-65-4].

"CAS" correspond à Chemical Abstracts.

Par marquage d'un récepteur ou d'un ligand, on entend :
- pour le récepteur, la fusion de son gène ou ADNc, ou partie du gène ou de l'ADNc, avec le gène ou ADNc, ou partie du gène ou ADNc, de la GFP;
- pour le ligand, il peut s'agir d'un couplage chimique entre le ligand et un groupe fluorescent, ou bien de la fusion de son gène ou ADNc, ou partie du gène ou de l'ADNc, avec le gène ou ADNc, ou partie du gène ou ADNc, de la GFP.

L'invention concerne l'utilisation d'une protéine fluorescente selon l'invention dans laquelle le récepteur et le ligand sont marqués par voie génétique, la protéine fluorescente et la substance fluorescente étant respectivement choisies parmi les couples de composés suivants :
GFPUV - EYFP
EYFP - GFPUV
ECFP - EYFP
EYFP - ECFP
ECFP - EGFP
EGFP - ECFP
EGFP - EYFP
EYFP - EGFP
et notamment dans laquelle le récepteur est marqué par la protéine EYFP ou EGFP et le ligand est marqué par la protéine ECFP, ou le récepteur est marqué par la protéine ECFP et le ligand est marqué par la protéine EYFP ou EGFP.

Selon un mode de réalisation avantageux de l'invention, la protéine fluorescente est EGFP et :
- soit la EGFP est donneur d'énergie de fluorescence et le marqueur absorbant la lumière émise par la EGFP est une substance fluorescente ou non, et le marqueur étant choisi parmi des substances, dont le spectre d'excitation chevauche le spectre d'émission de la EGFP,
   et, dans le cas où le marqueur est une substance fluorescente, il est choisi parmi: le 4,4-difluoro-4-bora-3a,4a-diaza-s-indacene (Bodipy), l'éosine, la lissamine, l'érythrosine, la tétraméthylrhodamine, la sulforhodamine 101 commercialisée par Molecular probe sous la dénomination Texas Red, et leurs dérivés permettant d'une part le greffage et, d'autre part, dont le spectre d'excitation recouvre le spectre d'émission de EGFP,
   et, dans le cas où le marqueur n'est pas une substance fluorescente, il est choisi parmi le groupe des acides violets [Acid Violet 5, CAS 10130-48-0 ; Acid Violet 7, CAS 4321-69-1 ; Acid Violet 17, CAS 4129-84-4], des acides rouges [Acid Red 1, CAS 3734-67-6 ; Acid Red 8, CAS 4787-93-3 ; Acid Red 37, CAS 6360-07-2 ; Acid Red 40, CAS 12167-45-2 ; Acid Red 106, CAS 6844-74-2 ; Acid Red 114, CAS 6459-94-5], les alizarines, l'aluminon, l'azocarmine B [CAS 25360-72-9], la fuschine basique [Basic Red 9, CAS 569-61-9], le Bordeaux R [Acid Red 17, CAS 5858-33-3], la Carmine [CAS 1390-65-4],
- soit la EGFP est accepteur d'énergie de fluorescence et la substance fluorescente est donneur d'énergie de fluorescence et est choisie parmi des substances, dont le spectre d'émission chevauche le spectre d'excitation de la EGFP, et notamment parmi : les coumarines, la fluorescamine, le 6-(N-méthylanilino)naphtalène, (mansyl) et leurs dérivés permettent d'une part le greffage et, d'autre part, dont le spectre d'émission recouvre le spectre d'excitation de EGFP,

Selon un mode de réalisation avantageux de l'invention, la protéine fluorescente est ECFP et :
- soit la ECFP est donneur d'énergie de fluorescence et la substance fluorescente est accepteur d'énergie et est choisie parmi la fluoresceïne et le 7-nitrobenz-2-oxa-1,3-diazole,
- soit la ECFP est accepteur d'énergie de fluorescence et la substance fluorescente est donneur d'énergie et est choisie parmi le pyrène ou la coumarine ou leurs dérivés permettant d'une part le greffage, et, d'autre part, dont le spectre d'émission chevauche le spectre d'excitation de la ECFP.

S'agissant du récepteur, il peut être choisi parmi :
- les récepteurs membranaires couplés à la protéine G, notamment dans Supplément Trends in Pharmacological Sciences, 1997 (*Receptor and ion Channel Nomenclature*) et dans les bases de données ensembl.org et GPCRdb,
- les récepteurs des facteurs de croissance, notamment ceux qui sont structurellement reliés au récepteur de l'insuline (Yarden, Y. and Ullrich, A., 1988) ou au récepteur de l'interféron γ (Brisco et al., 1996 ; Ihle, 1995) et ceux décrits dans les bases de données ensembl.org et GPCRdb,
- les récepteurs canaux, notamment dans Supplement Trends in Pharmacological Sciences, 1997 (*Receptor and ion Charcnel Nomenclature*) et ceux décrits dans les bases de données ensembl.org et GPCRdb,
- les récepteurs nucléaires intracellulaires, notamment ceux qui sont structurellement reliés au récepteur des stéroïdes (Mangelsdorf et al., 1995 ; Wurtz et al., 1996) et ceux décrits dans les bases de données ensembl.org et GPCRdb.

Selon un mode de réalisation avantageux, le récepteur est choisi parmi les récepteurs membranaires couplés à la protéine G.

Comme défini ci-dessus, on rappelle que par "récepteur" on désigne toute molécule de nature protéique susceptible d'entrer en interaction non covalente avec un agent pharmacologique, et, préférentiellement, un récepteur de neurotransmetteur, d'hormones, de facteur de croissance etc..., capable de produire, après interaction avec un ligand pharmacologique, une réponse de transduction de signal mesurable *in vivo* et/ou *in vitro.*

Par réponse de transduction de signal, on désigne toute réponse, ou inhibition de réponse, mesurable *in vivo* et/ou *in vitro,* résultant de l'interaction d'un récepteur avec ses agents pharmacologiques spécifiques et conduisant à des activations ou inhibitions du métabolisme cellulaire par effet sur des messagers secondaires, des enzymes, ou des courants ioniques.

A titre d'exemples de récepteurs membranaires couplés aux protéines G, on peut citer les récepteurs de purines et nucléotides, des amines biogéniques, des peptides et protéines, des eicosanoides, des lipides et dérivés, des acides aminés excitateurs et des ions, des molécules olfactives ainsi que les récepteurs orphelins (ci-après une liste assez exhaustive).

A titre d'exemple de récepteurs de facteurs de croissance, on peut citer les cytokines, le facteur de croissance épidermique, l'insuline, le facteur de croissance dérivé des plaquettes, le facteur de croissance de transformation.

A titre de récepteurs canaux, on peut citer notamment les récepteurs de l'ATP, de la sérotonine, du GABA, de la glycine, de l'acétylcholine, du glutamate.

A titre d'exemple de récepteurs nucléaires, on peut citer notamment les récepteurs des hormones thyroïdiennes, des oestrogènes, des glucocorticoïdes, des rétinoïdes.

A titre de ligands des récepteurs couplés à la protéine G on peut citer :
- Purines et Nucléotides
   - Adénosine
   - cAMP
   - ATP
   - UTP
   - ADP
- Amines Biogéniques (et ligands naturels reliés).
   - 5-hydroxytryptamine
   - Acétylcholine
   - Dopamine
   - Adrénaline
   - Histamine
   - Mélatonine
   - Noradrénaline
   - Tyramine/Octopamine
   - autres composés reliés
- Peptides
   - Hormone adrénocorticotrophique (ACTH)
   - Hormone stimulatrice de mélanocyte (MSH)
   - Mélanocortines
   - Neurotensine (NT)
   - Bombésine et peptides voisins
   - Endothélines
   - Cholecystokinine
   - Gastrine
   - Neurokinine B (NKB)
   - Récepteur des tachykinines
   - Substance K (NKA)
   - Substance P (SP)
   - Neuropeptide Y (NPY)
   - Facteur de libération de la thyrotropine
   - Nociceptine
   - Bradykinine
   - Angiotensine II
   - Beta-endorphine
   - C5a anaphalatoxine
   - Calcitonine
   - Chemokines (également appelés intercrines)
   - Facteur de libération corticotrophique (CRF)
   - Dynorphine
   - Endorphine
   - Peptides formylés
   - Follitropine (FSH)
   - Phéromones de maturation fongique
   - Galanine
   - Récepteur du polypeptide inhibiteur gastrique (GIP)
   - Peptides analogues du glucagon (GLPs)
   - Glucagon
   - Hormone de libération de gonadotropine (GmRH)
   - Hormone de libération de l'hormone de croissance (GHRM)
   - Hormone diurétique d'insecte
   - Interleukine
   - Leutropine (LH/HCG)
   - MET -enképhaline
   - Peptides opioïdes
   - Oxytocine
   - Hormone parathyroïde (PTH) et (PTHrP)
   - Peptides activant l'adényl cyclase pituitaire (PACAP)
   - Secretine
   - Somatostatine
   - Thrombine
   - Thyrotropine (TSH)
   - Peptide instestinal vasoactif (VIP)
   - Vasopressine
   - Vasotocine
- Eicosanoïdes
   - IP - Prostacyclines
   - PG - Prostaglandines
   - TX - Thromboxanes
- Composés à base de rétinal
   - 11-cis rétinal de vertébré
   - 11-cis rétinal d'invertébré
- Lipides et composés à bases de lipides
   - Cannabinoïdes
   - Anandamide
   - Acide lysophosphatidique
   - Facteur d'activation des plaquettes
   - Leukotriènes
- Amino acides excitateurs et ions
   - Ion Calcium
   - Glutamate
- Récepteurs orphelins

La présente invention concerne un procédé tel que défini ci-dessus, caractérisé en ce que l'on détermine la variation :
- de la cinétique de dissociation du complexe formé entre le susdit récepteur et l'un de ses ligands en présence dudit effecteur allostérique, par rapport à la cinétique de dissociation du complexe formé entre ledit récepteur et ledit ligand, en l'absence dudit effecteur,
- et/ou de l'amplitude de la liaison formée entre le susdit récepteur et l'un de ses ligands en présence dudit effecteur allostérique, par rapport à l'amplitude de la liaison formée entre ledit récepteur et ledit ligand, en l'absence dudit effecteur.
   La présente invention concerne un procédé tel que défini ci-dessus, caractérisé en ce que l'on détermine uniquement la cinétique de dissociation du complexe formé entre le susdit récepteur et l'un de ses ligands en présence dudit effecteur allostérique, par rapport à la cinétique de dissociation du complexe formé entre ledit récepteur et ledit ligand, en l'absence dudit effecteur.
   Dans tous les cas, la mesure de la variation de la cinétique est suffisante afin de détecter un effecteur allostérique.

- L'intérêt d'observer seulement la cinétique de dissociation réside dans le fait qu'à des concentrations élevées (saturantes) de ligand fluorescent, il est possible qu'aucune variation d'amplitude ne soit détectable car tous les sites de liaison sont saturés.

La présente invention concerne un procédé tel que défini ci-dessus, caractérisé en ce que l'on détermine uniquement l'amplitude de la liaison formée entre le susdit récepteur et l'un de ses ligands en présence dudit effecteur allostérique, par rapport à l'amplitude de la liaison formée entre ledit récepteur et ledit ligand, en l'absence dudit effecteur.

Dans certains cas, la mesure seule de l'amplitude est suffisante afin de détecter un effecteur alllostérique, tandis que dans d'autres, elle doit être couplée à la mesure de la variation de la cinétique.

Selon un mode de réalisation avantageux de la présente invention, le procédé tel que défini ci-dessus est caractérisé en ce que le ligand est un antagoniste.

Comme défini ci-dessus, on rappelle que par "antagoniste", on entend toute molécule inhibant l'effet de l'agoniste en se liant sur le même récepteur que ce dernier.

Selon un mode de réalisation avantageux de la présente invention, le procédé tel que défini ci-dessus est caractérisé en ce que le ligand est un agoniste.

Comme défini ci-dessus, on rappelle que par "agoniste", on entend toute molécule se liant au site du ligand endogène naturel et capable d'activer la réponse biologique.

Tout système biologique complexe peut être décrit par un modèle partiel comprenant un nombre d'états réduits suffisants pour décrire le phénomène observé. Dans le cas des modulateurs ou effecteurs allostériques, on emploie un modèle partiel comprenant deux états : l'état de repos (R) et l'état actif (A) du récepteur.

On a ainsi un équilibre R ↔ A.

Dans ce modèle, les ligands présentent des affinités distinctes pour les états R et A. De manière qualitative, on peut décrire les affinités relatives K_{R} et K_{A} pour respectivement R et A de la manière suivante, avec c = K_{A} / K_{R} :

| | | | |
|---|---|---|---|
| Affinité pour | État de repos | État actif | c |
| Agoniste | moins bonne | meilleure | c < 1 |
| Antagoniste compétitif | meilleure | moins bonne | c ≥ 1 |

Dans cette description, un agoniste déplace l'équilibre R ↔ A en faveur de A car son affinité est meilleure pour A que pour R. Inversement, l'antagoniste compétitif déplace l'équilibre vers R. Les écarts d'affinité entre l'état R et l'état A varient de 1 fois (c = 1)(pas de différence d'affinité) à plus de 100 fois selon le ligand (c ≥ 100 ou c ≤ 0,01). On obtient ainsi, pour un ensemble de ligands d'une cible, une palette de molécules agonistes (c < 1) ou antagonistes (c > 1) d'efficacité variable.

En pratique, l'association d'un ligand à un site récepteur est contrôlée par la diffusion de ce ligand dans le milieu biologique. Des différences d'affinité pour un ligand donné, et pour un site donné, résultent d'une vitesse de dissociation différente dudit ligand pour le site dans chacune des conformations qu'il peut adopter. Il en est de même pour les agonistes, les antagonistes compétitifs et les effecteurs allostériques. Il résulte de ceci que l'effecteur allostérique, en stabilisant par exemple une conformation de haute affinité pour l'agoniste, va augmenter la proportion des récepteurs dans la conformation de haute affinité, et donc entraîner une diminution de la vitesse de dissociation de l'agoniste lié à son site. Dans une mesure expérimentale de vitesse de dissociation, on obtiendra une courbe de dissociation en fonction du temps dont l'allure générale sera plus lente si l'effecteur stabilise des états de haute affinité pour le ligand fluorescent. Ces courbes sont correctement analysées selon un modèle multiexponentiel dans lequel les vitesses de dissociation sont identiques en présence et en absence d'effecteur, mais dont les amplitudes (et donc les concentrations fractionnelles des divers états conformationnels) différent. On obtiendra ainsi des rapports de concentration [Repos]/[Actif] qui différeront lorsque l'agoniste seul est présent et lorsque l'agoniste et l'effecteur allostérique sont présents tous deux. De la même manière, l'expérience peut être conduite avec un antagoniste : dans ce cas, l'effet des effecteurs allostériques sera de signe opposé à celui qui est observé pour un agoniste.

Si on effectue une dissociation après association à l'équilibre, on pourra révéler cette différence d'affinité entre R et A par une cinétique de dissociation présentant un décours temporel monoexponentiel (0,01 ≤ L₀c ≤ 100) ou biexponentiel pour L₀c > 100 ou L₀c < 0,01. Dans le cas où la cinétique est biexponentielle, elle est décrite à l'aide de 2 constantes de vitesse et de 2 amplitudes, et le rapport des 2 amplitudes correspond à la concentration fractionnelle de chacun des états. Lorsque la cinétique de dissociation est biexponentielle, on enregistre la somme des évènements de dissociation de chacun des états conformationnels.

De même que précédemment, dans le cas des effecteurs, on peut décrire les affinités relatives K_{R} et K_{A} pour respectivement R et A de la manière suivante, avec c = K_{A} / K_{R}:

| | | | |
|---|---|---|---|
| Affinité pour | État de repos | État actif | c |
| Effecteur positif | moins bonne | meilleure | c < 1 |
| Effecteur négatif | meilleure | moins bonne | c ≥ 1 |

Dans cette description, un effecteur positif déplace l'équilibre R ↔ A en faveur de A car son affinité est meilleure pour A que pour R. Inversement, l'effecteur négatif déplace l'équilibre vers R.

Ainsi, dans le cas où il existe deux états, à savoir un état actif et un état de repos, la présente invention concerne un procédé tel que défini ci-dessus, caractérisé en ce que la cinétique de dissociation du complexe formé entre ledit récepteur et l'un de ses ligands (agoniste), en présence dudit effecteur, est plus lente que la cinétique de dissociation du complexe formé entre ledit récepteur et ledit ligand, en l'absence dudit effecteur, ce qui implique que l'effecteur allostérique est un effecteur positif. Dans ce mode de réalisation préféré de l'invention, le ligand est un agoniste : il déplace donc l'équilibre R ↔ A vers A (état actif du récepteur) pour lequel il a une affinité plus élevée et donc une vitesse de dissociation plus lente, tandis que l'effecteur allostérique déplace aussi l'équilibre vers A, ce qui a pour effet d'augmenter l'amplitude de la dissociation lente et de diminuer l'amplitude de la dissociation rapide.

Dans le cas où il existe deux états, à savoir un état actif et un état de repos, la présente invention concerne un procédé tel que défini ci-dessus, caractérisé en ce que la cinétique de dissociation du complexe formé entre ledit récepteur et l'un de ses ligands (agoniste), en présence dudit effecteur, est plus rapide que la cinétique de dissociation du complexe formé entre ledit récepteur et ledit ligand, en l'absence dudit effecteur, ce qui implique que l'effecteur allostérique est un effecteur négatif. Dans ce mode de réalisation préféré de l'invention, le ligand est un agoniste : il déplace donc l'équilibre R ↔ A vers A (état actif du récepteur) pour lequel il a une affinité plus élevée et donc une vitesse de dissociation plus lente, tandis que l'effecteur allostérique déplace aussi l'équilibre vers R, ce qui a pour effet de diminuer l'amplitude de la dissociation lente et d'augmenter l'amplitude de la dissociation rapide.

Dans le cas où il existe deux états, à savoir un état actif et un état de repos, la présente invention concerne un procédé tel que défini ci-dessus, caractérisé en ce que la cinétique de dissociation du complexe formé entre ledit récepteur et l'un de ses ligands, en présence dudit effecteur, est plus lente que la cinétique de dissociation du complexe formé entre ledit récepteur et ledit ligand, en l'absence dudit effecteur, ce qui implique que l'effecteur allostérique est un effecteur négatif. Dans ce mode de réalisation préféré de l'invention, le ligand est un antagoniste : il déplace donc l'équilibre R ↔ A vers R (état de repos du récepteur) pour lequel il a une affinité plus élevée et donc une vitesse de dissociation plus lente, tandis que l'effecteur allostérique stabilise l'état de repos, ce qui a pour effet d'augmenter l'amplitude de la dissociation lente, tout en diminuant l'amplitude de la dissociation rapide.

Dans le cas où il existe deux états, à savoir un état actif et un état de repos, la présente invention concerne un procédé tel que défini ci-dessus, caractérisé en ce que la cinétique de dissociation du complexe formé entre ledit récepteur et l'un de ses ligands, en présence dudit effecteur, est plus rapide que la cinétique de dissociation du complexe formé entre ledit récepteur et ledit ligand, en l'absence dudit effecteur, ce qui implique que l'effecteur allostérique est un effecteur positif. Dans ce mode de réalisation préféré de l'invention, le ligand est un antagoniste : il déplace donc l'équilibre R ↔ A vers R (état de repos du récepteur) pour lequel il a une affinité plus élevée et donc une vitesse de dissociation plus lente, tandis que l'effecteur allostérique stabilise l'état actif, ce qui a pour effet de diminuer l'amplitude de la dissociation lente, et d'augmenter l'amplitude de la dissociation rapide.

Dans le cas où le récepteur peut adopter un nombre de conformations supérieur à deux, il convient d'identifier les conformations occupées par le ligand fluorescent afin de déterminer sur quels équilibres conformationnels porte l'effet de l'agent allostérique supposé.

Ainsi, s'il existe trois états conformationnels R (repos), A (actif) et D (désensibilisé), l'agoniste peut occuper les états A et D tandis que l'antagoniste se liera de manière préférentielle à l'état R ou à l'état D. Il en résultera des effets de nature différente de l'agent allostérique selon que l'on utilisera un agoniste fluorescent ou une antagoniste fluorescent dans les expériences de mesure de liaison. L'effecteur positif (qui stabilise A) réduira la liaison de l'agoniste à l'état D. Il y aura augmentation de la vitesse de dissociation par disparition d'une fraction des récepteurs dans l'état D qui généralement lie l'agoniste avec une affinité plus grande que l'état A.

De la même manière, si il existe deux états actifs A1 et A2, un effecteur stabilisant A1 aura pour effet de réduire la liaison à A2 (et diminuera les réponses qui y sont associées) tandis qu'il augmentera la liaison à A1 (et potentialisera les réponses qui y sont associées). Dans ce cas, on observera une augmentation de la vitesse de dissociation de l'agoniste fluorescent.

L'analyse des cinétiques d'association du ligand est plus complexe. Elle dépend en effet du système expérimental analysé. Pour certains récepteurs, et dans certaines conditions expérimentales, il est possible d'observer des cinétiques d'association multiexponentielles. Dans ces cas, la cinétique la plus rapide reflète l'interaction bimoléculaire du ligand (agoniste ou antagoniste) avec le récepteur, tandis que les cinétiques plus lentes peuvent refléter des interconversions conformationnelles qui prennent place plus lentement. Si cela est observé expérimentalement, il est alors possible d'analyser les amplitudes des cinétiques lentes et d'observer des variations de ces amplitudes en présence d'un effecteur allostérique. Ces variations reflèteront la stabilisation différentielle des divers états conformationnels par l'effecteur allostérique. Dans d'autres cas défavorables, les interconversions conformationnelles sont cinétiquement invisibles car plus rapides que la cinétique d'association bimoléculaire du ligand avec le récepteur. L'effet d'un effecteur allostérique sur la vitesse d'association n'est alors pas détectable expérimentalement.

L'analyse des réponses est une manière de suivre l'état actif du récepteur. En effet, on définit l'état actif comme un état conformationnel doté de la capacité de produire la réponse biologique. L'analyse des réponses doit porter sur plusieurs paramètres :

L'un des paramètres les plus simplés à évaluer est l'amplitude de la réponse. Celle-ci sera plus grande en présence d'un effecteur allostérique positif et plus faible lorsque l'effecteur allostérique est négatif.

Le deuxième paramètre est celui du délai : il est parfois possible de détecter un délai d'établissement plus court pour des réponses potentialisées, et inversement un délai plus long pour des réponses inhibées, en comparaison à une réponse contrôle. Ceci est en effet observable lorsque le récepteur active des effecteurs secondaires eux-mêmes responsables de la réponse. Dans ce cas, qui est celui des récepteurs couplés aux protéines G, l'accumulation transitoire de protéines de relais activées (la protéine G) ou de messagers secondaires (inositols triphosphates, AMPc...), l'apparition de la réponse peut survenir avec un délai moindre lorsqu'il y a potentialisation.

Enfin, lorsque plusieurs réponses peuvent être déclenchées par un récepteur, l'analyse comparative des amplitudes (et/ou des délais) des diverses réponses peut apporter des indications sur la nature de l'effet modulateur.

La présente invention concerne également un procédé tel que défini ci-dessus, par détermination de :
- la variation de l'amplitude de la liaison formée entre le susdit récepteur et l'un de ses ligands en présence dudit effecteur allostérique, par rapport à l'amplitude de la liaison formé entre ledit récepteur et ledit ligand, en l'absence dudit effecteur,
- et éventuellement de la variation de la cinétique de dissociation du complexe formé entre le susdit récepteur et l'un de ses ligands en présence dudit effecteur allostérique, par rapport à la cinétique de dissociation du complexe formé entre ledit récepteur et ledit ligand, en l'absence dudit effecteur.

La présente invention concerne un procédé tel que défini ci-dessus, caractérisé en ce que l'on détermine uniquement la variation de l'amplitude de la liaison formée entre le susdit récepteur et l'un de ses ligands en présence dudit effecteur allostérique, par rapport à l'amplitude de la liaison formé entre ledit récepteur et ledit ligand, en l'absence dudit effecteur, lorsque ladite variation est positive.

La présente invention concerne un procédé tel que défini ci-dessus, caractérisé en ce que l'on détermine :
- la variation de l'amplitude de la liaison formée entre le susdit récepteur et l'un de ses ligands en présence dudit effecteur allostérique, par rapport à l'amplitude de la liaison formé entre ledit récepteur et ledit ligand, en l'absence dudit effecteur,
et que ladite variation est négative, ce qui nécessite la détermination de :
- la variation de la cinétique de dissociation du complexe formé entre le susdit récepteur et l'un de ses ligands en présence dudit effecteur allostérique, par rapport à la cinétique de dissociation du complexe formé entre ledit récepteur et ledit ligand, en l'absence dudit effecteur.

Ce mode de réalisation permet ainsi de distinguer un effecteur allostérique d'un agent compétitif.

Selon un mode de réalisation avantageux de la présente invention, le procédé de l'invention est caractérisé en ce que ladite variation de cinétique de dissociation est positive ou négative, ce qui implique que le composé testé est un effecteur allostérique. Si ladite variation de cinétique de dissociation est nulle, cela implique que le composé testé est un compétiteur.

Dans le cas des récepteurs couplés aux protéines G on discerne des cas complexes car il peut exister plusieurs états actifs d'un récepteur (Palanché et al., 2001 ; Lefkowitz, 1998). Dans ce cas, les différents états actifs sont pourvus de propriétés fonctionnelles distinctes, c'est-à-dire qu'ils régulent des réponses qui peuvent différer les unes des autres. L'effecteur allostérique peut alors avoir un comportement discriminant les états actifs entre eux. Ainsi, en privilégiant la stabilisation d'un état actif pris parmi plusieurs, l'effecteur peut se comporter comme modulateur positif d'une réponse donnée tout en se comportant comme un modulateur négatif des réponses associées aux autres états actifs. On définira alors le caractère positif et/ou négatif par rapport à une réponse biologique donnée.

L'effecteur peut moduler une réponse inconnue du récepteur. Ainsi, dans le cas où on n'observe pas d'effet sur une réponse connue sur le récepteur, la présente invention permet de rechercher d'autres réponses du récepteur.

Des produits répondant à l'une des formules suivantes ont été identifiés : 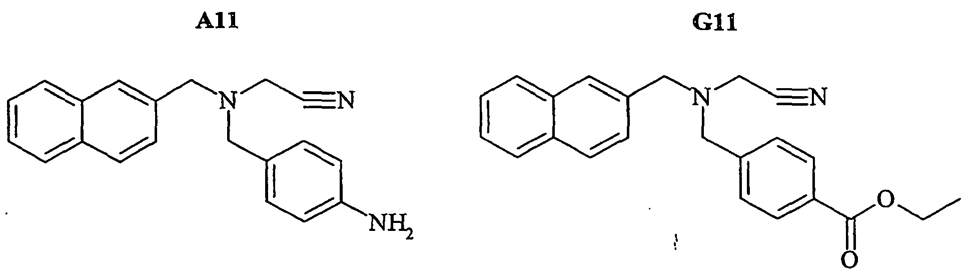 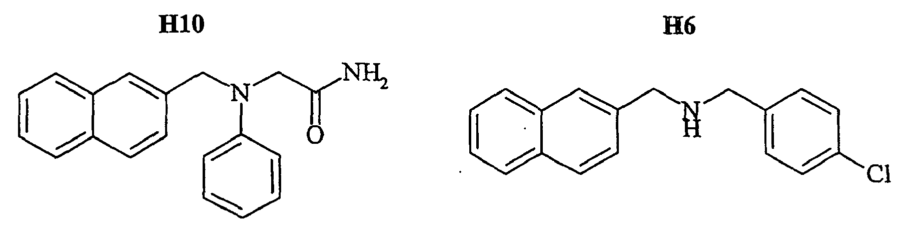 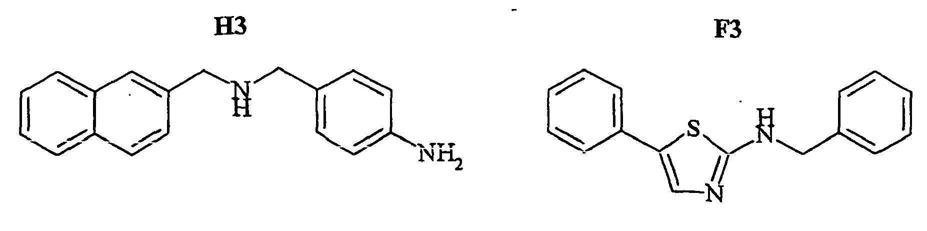 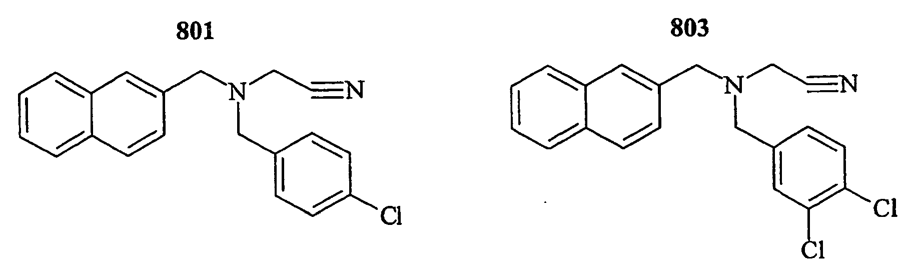 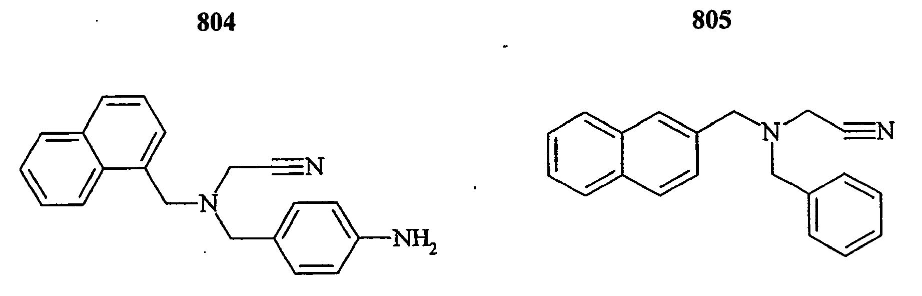 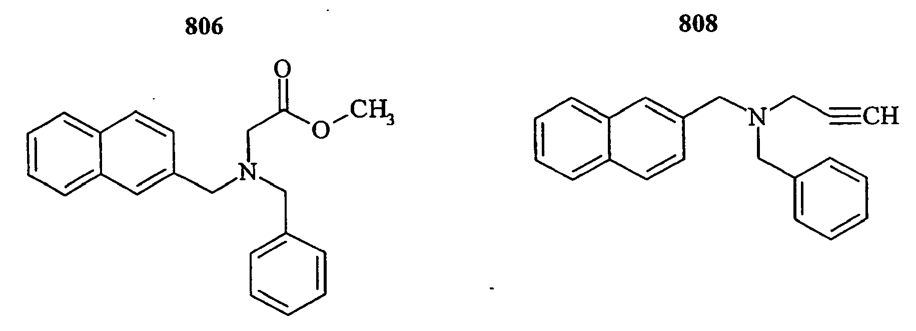 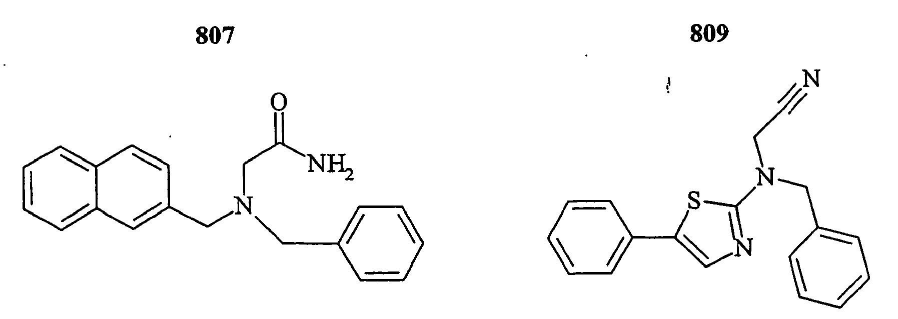 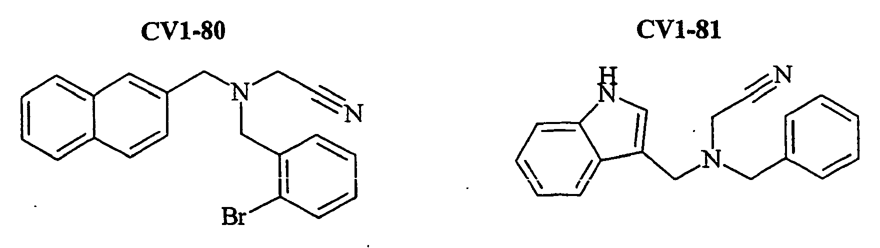 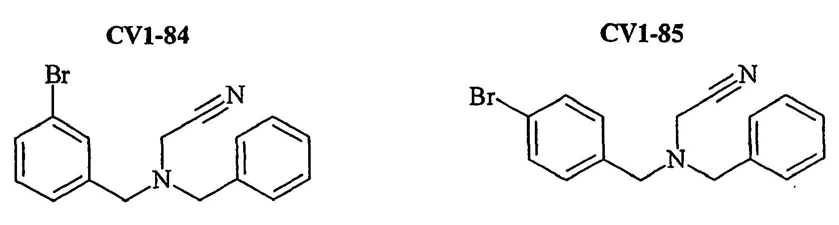 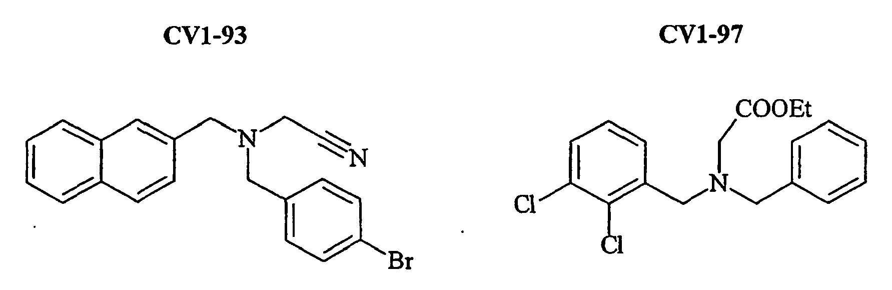 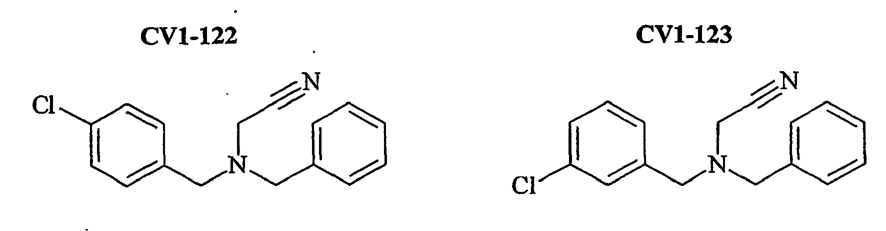 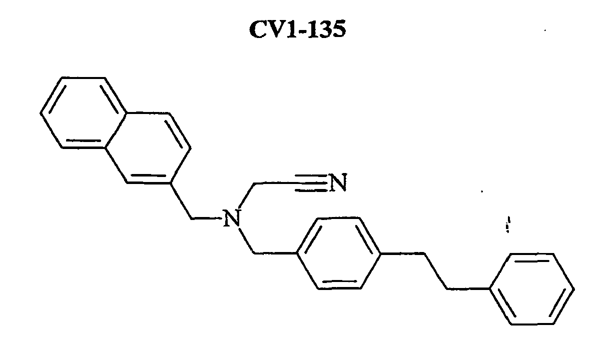 lesdits produits étant des composés tels que détectés par le procédé indiqué ci-dessus.

Les produits de formule A11, G11, H10, H6, H3, F3, 801, 802, 803, 804, 805, 806, 807, 808, 809, CV1-80, CV1-81, CV1-84, CV1-85, CV1-93, CV1-97, CV1-122, CV1-123, CV1-131 et CV1-135 sont nouveaux en tant que tels.

Les tableaux 1 et 2 suivants résument l'ensemble des données expérimentales acquises pour les molécules G6, A11, G11, F3, H6, F9, H3, F7, H10, NP234 (ou 801), NP 246 (ou 803), 804, 805, 806, 808, 807, 809, CV1-80, CV1-81, CV1-84, CV1-85, CV1-93, CV1-97, CV1-122, CV1-123, et CV1-135, dont les structures sont indiquées en colonnes.

Dans le tableau 1, la colonne "code" correspond au nom d'usage ; la colonne "EC50" correspond à la concentration de composé provoquant une augmentation de 50% (par rapport à la valeur maximale) du pourcentage de l'amplitude de la vitesse de dissociation rapide ; la colonne "%a1 (max) " correspond à la valeur maximale observée pour l'augmentation d'amplitude de la vitesse de dissociation rapide ; la colonne "IC50" correspond à la concentration de composé pour laquelle on observe une diminution de 50% de la liaison de 20 nM de NKA-Bo au récepteur EGFP-NK2R ; la colonne "Rep Ca" correspond à la capacité de chaque composé à évoquer une réponse calcique sur des cellules n'exprimant pas le récepteur EGFP-NK2R et la colonne "inhib AMPc" indique la capacité de chaque composé à inhiber la réponse de production d'AMPc évoquée par la NKA.

Dans le tableau 2, la colonne "code" correspond au nom d'usage ; la colonne "EC50" correspond à la concentration de composé provoquant une augmentation de 50% (par rapport à la valeur maximale) du pourcentage de l'amplitude de la vitesse de dissociation rapide ; la colonne "% al (à 10 µM) " signifie que les composés ont été testés uniquement à 10 µM et comparés aux valeurs déterminées pour 805 (T = 22 ± 1 représente l'amplitude de dissociation rapide en absence d'effecteur) ; la colonne "% Réversion de liaison (à 10 µM) " signifie que les composés ont été testés uniquement à 10 µM et comparés aux valeurs déterminées pour 805.

Parmi tous les composés, 805 a fait l'objet d'une étude de la potentialisation des réponses calciques évoquées par la NKA ou la NKA tronquée sur les récepteurs NK2R humain sauvage, NK1R humain sauvage, ou EGFP-NK2R.

**Tableau 1**

| **Code** | **Structure** | **Nom IUPAC** | **EC50 (µM)** | **% al (max)** | **IC50 (µM)** | **Rép. Ca** | **Inhib AMPc** |
|---|---|---|---|---|---|---|---|
| G6 | 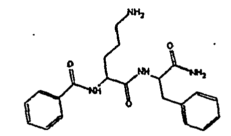 | N-(4-amino-1-(1-carbamoyl-2-phenyl- ethylcarbamoyl)-butyl)-benzamide | ns | ns | 46 | nt | nt |
| A11 | 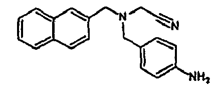 | [(4-Aminobenzyl)-naphthalen-2- ylmethyl-amino]-acetonitrile | 38-44 | ≥90 | 100 | ns | ++ |
| G11 | 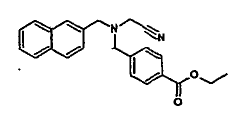 | 4-[(Cyanomethyl-naphthalen-2-ylmethyl-amino)- methyl]-benzoic acid ethyl ester | .10-30 | 70 | >100 | ns | nt |
| F3 | 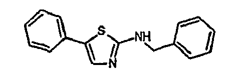 | Benzyl-(5-phenyl-thiazol-2-yl)-amine | 1-5 | 65 | 100 | ++ | + |
| H6 | 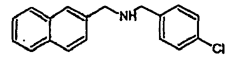 | (4-Chloro-benzyl)-naphthalen-2-ylmethyl-amine | 50-80 | 80 | >100 | ++ | ++ |
| F9 | 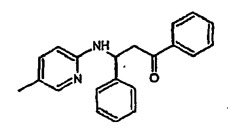 | 3-(5-Methyl-pyridin-2-ylamino)-1,3- diphenyl-propan-1-one | ns | ns | >50 | ++ | nt |
| H3 | 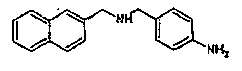 | 4-{[(Naphthalen-2-ylmethyl)-amino]-methyl}- phenylamine | ns | ns | >50 | ns | nt |
| F7 | 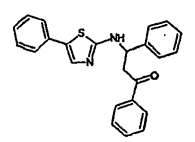 | 1,3-Diphenyl-3-(5-phenyl-thiazol-2-ylamino)-propan- 1-one | ≥50 | ≥60 | 90 | ns | nt |
| H10 | 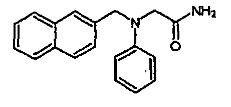 | 2-(Naphthalen-2-ylmethyl-phenyl-amino)-acetamide | >50 | >60 | ≥100 | ns | nt |
| NP234 ou 801 | 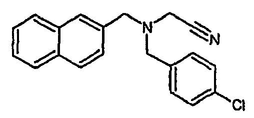 | [(4-chlorobenzyl)(2-naphthylmethyl)a mino]acetonitrile | 5-10 | 60-70 | 110 | ns | ++ |
| NP246 ou 803 | 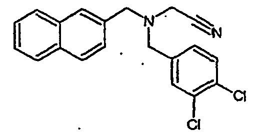 | [(3,4-dichlorobenzyl)(2-naphthylmethyl)a mino]acetonitrile | 10-30 | 60-70 | >500 | ns | nt |
| 804 | 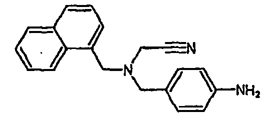 | [(4-aminobenzyl)(1-naphthylmethyl)a mino]acetonitrile | 20-30 | ≥80 | >100 | ns | nt |
| 805 | 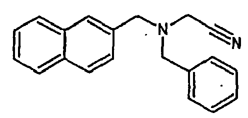 | [benzyl(2-naphthylmethyl)a mino]acetonitrile | 3-8 | 80 | >500 | ns | ++ |
| 806 | 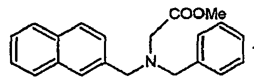 | methyl [benzyl(2-naphthylmethyl)a mino]acetate | 10-20 | 90 | >200 | ns | nt |
| 808 | 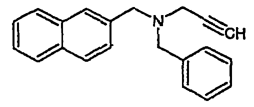 | *N*-beozyl-*N*-(2-naphthylmethyl)-*N*-prop-2- ynylamine | 20-30 | ≥70 | >200 | + | nt |
| 807 | 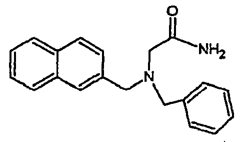 | 2-[benzyl(2-naphthylmethyl)a mino]acetamide | ? | ≥ 90 | 100 | + ? | nt |
| 809 | 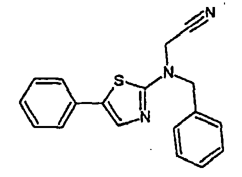 | [benzyl(5-phenyl-1,3-thiazol-2-yl)amino]acetonitr ile | 1-20 | ≥75 | >100 | nt | nt |

| | | | | | | | |
|---|---|---|---|---|---|---|---|
| *ns : non significatif; nt : non testé ;* ? : *non interprétable* | | | | | | | |

**Tableau 2**

| **Code** | **Structure** | **Nom IUPAC** | **EC50 (µM)** | **% al (à 10µM) T=22±1** | **% Reversion liaison (à 10µM)** | **Rép. Ca** |
|---|---|---|---|---|---|---|
| 805 | | | 8 | 29 (10µM) 45 (50µM) | 1 (10µM) 9 (50µM) | ns |
| CV1-80 | 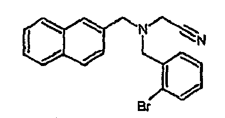 | [(2-bromobenzyl)(2-naphthylmethyl)a mino]acetonitrile | nt | 25 | 16 | nt |
| CV1-81 | 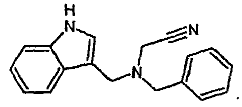 | [benzyl(1*H*-indol-3 ylmethyl)amino]ac etonitrile | nt | 22 | 15 | nt |
| CV1-84 | 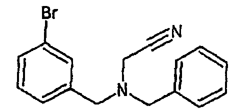 | [benzyl(3-bromobeazyl)amin o]acetonitrile | nt | 25 | 4 | nt |
| CV1-85 | 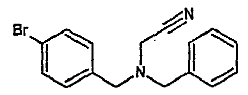 | [benzyl(4-bromobenzyl)amin o]acetonitrile | nt | 28 | 4 | nt |
| CV1-93 | 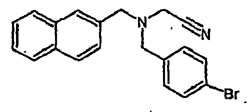 | [(4-bromobenzyl)(2- naphthylmethyl)a mino]acetonitrile | nt | 26 | 3 | nt |
| CV1-97 | 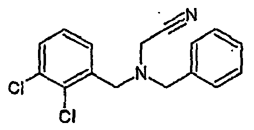 | [benzyl(2,3-dichlorobenzyl)am ino] acetonitrile | nt | 34 | 15 | nt |
| CV1-122 | 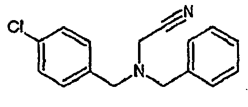 | [benzyl(4-chlorobenzyl)amin o]acetonitrile | nt | 31 | 17 | nt |
| CV1-123 | 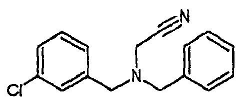 | [benzyl(3-chlorobenzyl)amin o]acetonitrile | nt | 26 | 7 | nt |
| CV1-135 | 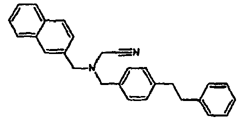 | | nt | 22 | 6 | nt |

| | | | | | | |
|---|---|---|---|---|---|---|
| *ns : non significatif; nt : non testé ;* ? : *non interprétable* | | | | | | |

- Les composés susmentionnés sont regroupés dans deux grandes familles : famille I et famille II.

La famille I regroupe les composés A11, G11, H10, 801, 803, 804, 805, 806, 808, 807, CV1-80, CV1-81, CV1-84, CV1-85, CV1-93, CV1-97, CV1-122, CV1-123 et CV1-135.

Ces composés répondent à la formule générale suivante : 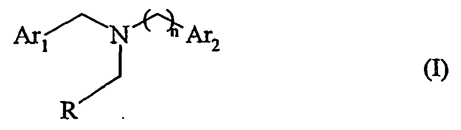 dans laquelle n est égal à 0 ou 1, Ar₁ et Ar₂ représentent des groupes aromatiques substitués monocycles ou bicycles et R représente un groupe électroattracteur tel que CN, COOMe ou CONR₁R₂ ou un groupe insaturé tel qu'un groupe alcène ou alcyne.

| **composé** | **n** | **Ar**_{**1**} | **Ar**_{**2**} | **R** |
|---|---|---|---|---|
| **A11** | 1 | β-naphtyle | 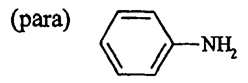 | C≡N |
| **G11** | 1 | β-naphtyle | 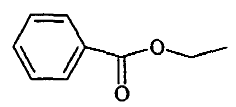 | C≡N |
| **H10** | 0 | β-naphtyle | 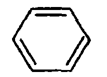 | CONH_{**2**} |
| **801** | 1 | β-naphtyle | 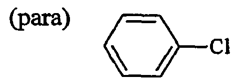 | C≡N |
| **803** | 1 | β-naphtyle | 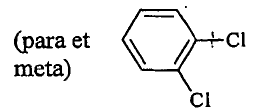 | C≡N |
| **804** | 1 | α-naphtyle | 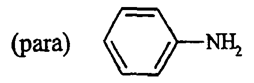 | C≡N |
| **805** | 1 | β-naphtyle | 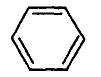 | C≡N |
| **806** | 1 | β-naphtyle | 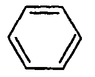 | COOCH₃ |
| **808** | 1 | β-naphtyle | 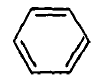 | C≡CH |
| **807** | 1 | β-naphtyle | 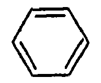 | CONH₂ |
| **CV1-80** | 1 | β-naphtyle | 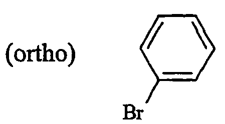 | C≡N |
| **CV1-81** | 1 | 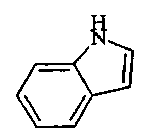 | 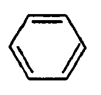 | C≡N |
| **CV1-84** | 1 | 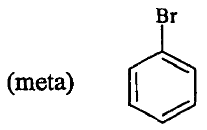 | 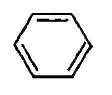 | C≡N |
| **CV1-85** | 1 | 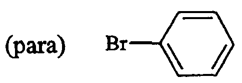 | 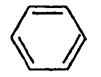 | C≡N |
| **CV1-93** | 1 | β-naphtyle | 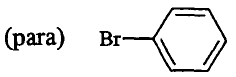 | C≡N |
| **CV1-97** | 1 | 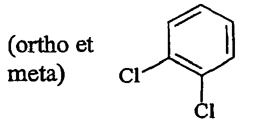 | 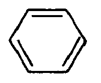 | COOEt |
| **CV1-122** | 1 | 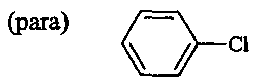 | 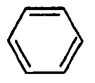 | C≡N |
| **CV1-123** | 1 | 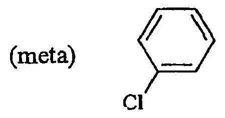 | 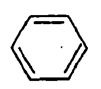 | C≡N |
| **CV1-135** | 1 | β-naphtyle | 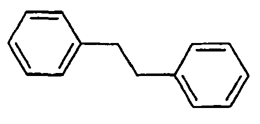 | C≡N |

Les composés H6 et H3 sont des intermédiaires de synthèse permettant l'obtention des composés de la famille I susmentionnée.

La famille II regroupe les composés F3, F7 et 809.

Ces composés répondent à la formule générale suivante : 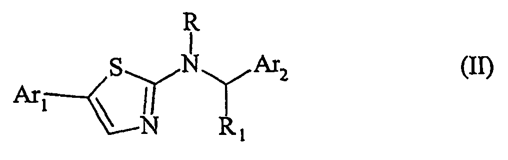 dans laquelle Ar₁ et Ar₂ représentent des groupes aromatiques, R représente soit un atome d'hydrogène soit un groupe -CH₂CN; et R₁ est un atome d'hydrogène ou un groupe CH₂COΦ.

Les composés F7 et F9 sont déjà connus et sont décrits dans l'article de Moutou et al. (1994).

Par ailleurs, le composé G6 du tableau 1 est déjà connu et décrit dans la demande internationale WO 02/24192.

### Modes de préparation des composés susmentionnés :

### 1 - Mode de préparation des composés de la famille I :

### A) Première étape : préparation des amines secondaires :

A une solution de l'aldéhyde aromatique approprié (1 éq) dans le méthanol, on ajoute l'amine primaire (1 éq)(voir tableau ci-après). Le milieu réactionnel est chauffé à 60°C pendant deux heures puis refroidi rapidement à 0°C. On ajoute ensuite du NaBH₄ (2 éq) et on agite pendant quelques minutes puis on remet le milieu réactionnel à température ambiante pendant une heure. Après évaporation de la solution, on extrait avec de l'AcOEt en lavant avec de l'eau puis avec une solution saturée en NaCl. On sèche avec Na₂SO₄, on filtre sur fritté et on évapore à sec. L'amine secondaire, ainsi isolée, le plus généralement sous la forme d'une huile, est utilisée telle quelle sans aucune purification.

L'amine secondaire ainsi obtenue répond à la formule suivante : 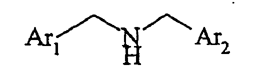 dans laquelle Ar₁ et Ar₂ sont tels que définis ci-dessus.

Un exemple de réaction correspondant à l'étape 1 (ici obtention du composé H3) est :

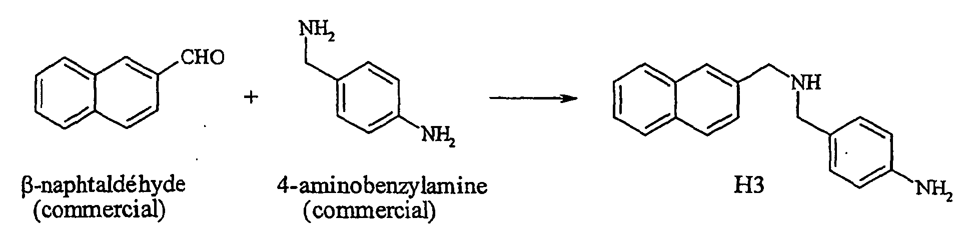

Tableau récapitulatif des produits de départ :

| **Produit final obtenu à l'issue de l'étape 2** | **Aldéhyde de départ** | **Amine de départ** |
|---|---|---|
| **H3** | β-naphtaldéhyde | 4-aminobenzylamine |
| **A11** | β-naphtaldéhyde | 4-aminobenzylamine |
| **G11** | β-naphtaldéhyde | 4-éthoxycarbonylbenzylamine |
| **H6** | β-naphtaldéhyde | 4-chlrobenzylamine |
| **H4** | β-naphtaldéhyde | benzylamine |
| **H10** | β-naphtaldéhyde | aniline |
| **801** | β-naphtaldéhyde | 4-chlorobenzylamine |
| **803** | β-naphtaldéhyde | 3,4-dichlorobenzylamine |
| **804** | α-naphtaldéhyde | 4-aminobenzylamine |
| **805** | β-naphtaldéhyde | benzylamine |
| **806** | β-naphtaldéhyde | benzylamine |
| **808** | β-naphtaldéhyde | benzylamine |
| **807** | β-naphtaldéhyde | benzylamine |
| **CV1-80** | β-naphtaldéhyde | 2-bromobenzylamine |
| **CV1-81** | β-naphtaldéhyde | indol 3-méthylamine |
| **CV1-84** | 3-bromobenzaldéhyde | benzylamine |
| **CV1-85** | 4-bromobenzaldéhyde | benzylamine |
| **CV1-93** | β-naphtaldéhyde | 4-bromobenzylamine |
| **CV1-97** | 3,4-dichlorobenzaldéhyde | benzylamine |
| **CV1-122** | 4-chlrobenzaldéhyde | benzylamine |
| **CV1-123** | 3-chlrobenzaldéhyde | benzylamine |

### B) Deuxième étape : préparation des amines tertiaires :

L'amine secondaire (1,1 éq) obtenue à l'étape précédente est dissoute dans le diméthylformamide, et on ajoute de l'halogénure d'alkyle (chloroacétonitrile, chlorure de propargyle, etc.)(voir plus loin). La solution est chauffée à reflux pendant 12 heures puis on la laisse revenir à température ambiante. On extrait ensuite avec de l'éther (10 fois plus en volume que le DMF) et on lave avec de l'eau puis avec une solution saturée de NaCl. La phase organique est séchée avec Na₂SO₄ puis elle est évaporée à sec. Le brut est purifié par chromatographie sur gel de silice (AcOEt 1/Hex 9). Les chlorhydrates sont préparés par barbotage de HCl gazeux dans l'AcOEt.

On obtient alors les composés susmentionnés, sous forme de chlorhydrate, de formule telle que définie ci-dessous :

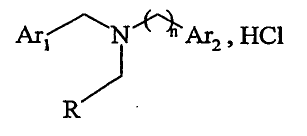

Plus précisément, cette deuxième étape correspond au schéma réactionnel suivant :

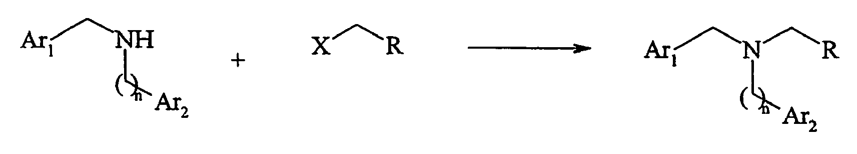

Les amines secondaires sont donc mises à réagir avec différents halogénures commerciaux.

Pour les composés A11, G11, 801, 803, 804, 805, CV1-80, CV1-81, CV1-84, CV1-85, CV1-93, CV1-122, CV 1-123 et CV1-135, on utilise du chloroacétonitrile.

Pour les composés 806 et CV1-97, on utilise du bromo ou du chloroacétate d'éthyle ou de méthyle.

Pour les composés H4, H10 et 807, on utilise du bromo ou du chloroacétamide.

Pour le composé 808, on utilise du bromure ou du chlorure de propargyle.

Concernant le composé CV1-135, il est obtenu selon un mode opératoire particulier, tel que décrit ci-après :

On dissout dans l'acétonitrile le 4-bromobenzonitrile de formule : 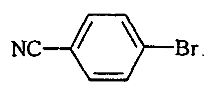 ainsi que le catalyseur PdCl₂(PPh₃)₂, de l'iodure de cuivre CuI, la base NEt₃ et du phénylacétylène. Le milieu réactionnel est chauffé pendant une nuit à 50-60°C. On évapore en fin de réaction et on purifie par chromatographie sur colonne de silice.

Ce mode opératoire correspond au schéma réactionnel suivant :
1) 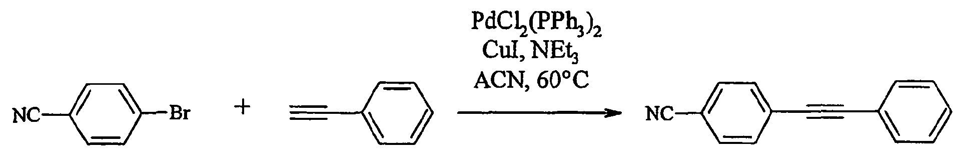
2)

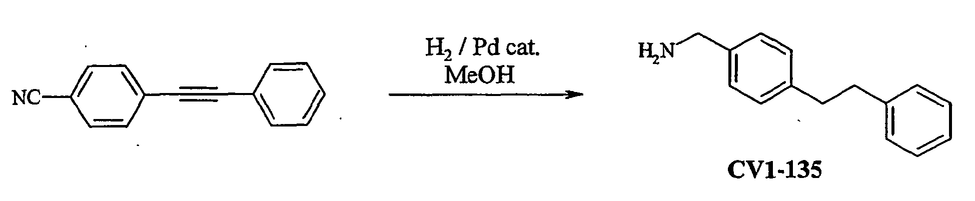

### II - Mode de préparation des composés de la famille II :

Le 5-phényl 2-benzylaminothiazole est obtenu au départ du 5-phényl-2-aminothiazole par condensation avec le benzaldéhyde, suivie d'une hydrogénation. Le composé obtenu et ensuite alkylé par le chloroacétonitrile (voir mode opératoire I).

Le composé F3 est obtenu selon le schéma réactionnel suivant :

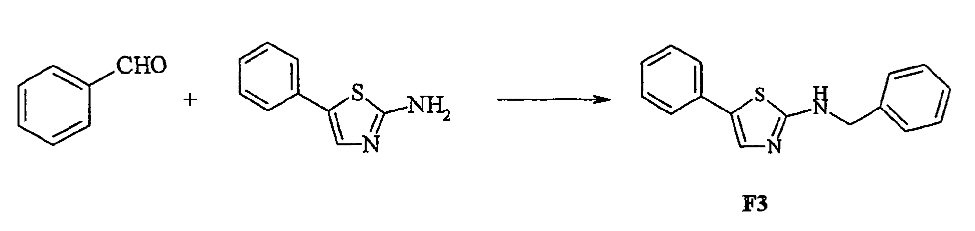

A une solution de 5-phényl-2-aminobenzylthiazole (1 éq) dans le DMF refroidi à 0°C est ajouté du NaH (1,3 éq). Le milieu réactionnel est agité pendant 15 minutes puis le chloroacétonitrile (1 éq) est ajouté gouttes à gouttes. L'agitation est poursuivie à température ambiante pendant 8 heures, la solution est ensuite diluée par l'eau. Après extraction à l'acétate d'éthyle, la phase organique est séchée sur sulfate de sodium anhydre et est évaporée. Le brut réactionnel est purifié par chromatographie sur gel de silice (AcOEt 1 / Hexane 2).

On obtient donc ainsi par exemple le composé 809 : ((5-phényl-thiazol-2-yl)-*N*-benzyl)acétonitrile C₁₈H₁₅N₃S (305,40 g.mol⁻¹) sous la forme d'une poudre beige, selon le schéma réactionnel suivant : 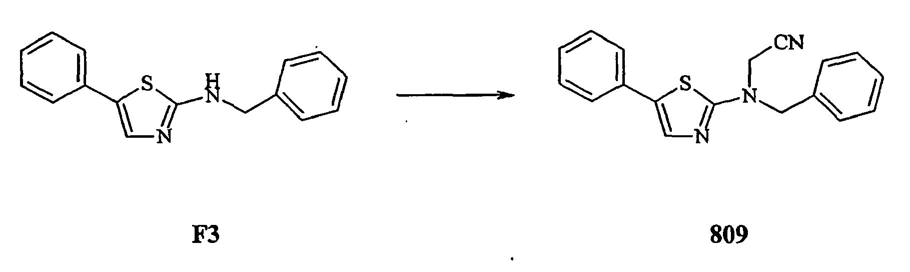 RMN ¹H (CDCl₃, 200 MHz) : 4,42 (s, 2H) ; 4,70 (s, 2H) ; 7,30-7,51 (m, 11H)

### DESCRIPTION DÉTAILLÉE DE L'INVENTION

Dans sa réalisation préférée, le développement de l'invention utilise l'ADNc codant pour la protéine fluorescente verte (Prasher et al., 1992) de la méduse aequorea victoria, préférentiellement les mutants EYFP, EGFP et ECPF de cette protéine optimisée pour leur expression dans les organismes hôtes préférés, les cellules mammifères.

L'ADNc peut être modifié pour coder pour un variant dans lequel un ou plusieurs acides aminés sont substitués, insérés ou délétés afin de permettre sa fusion, en N ou en C-terminal avec le gène codant pour un récepteur.

Le récepteur peut être choisi parmi :
1) les récepteurs de neurotransmetteurs couplés à des protéines G structurellement reliés aux récepteurs adrénergiques et récepteurs métabotropiques du glutamate tels que présentés dans la liste actualisées annuellement et éditée dans les bases de données GPCRdb (http://www.gcrdb.uthscsa.edu ou http://www.gpcr.org), ensembl (http://www.ensembl.org) entre autres.
2) les récepteurs-canaux structurellement reliés aux récepteurs nicotiniques, aux récepteurs de glutamate et aux récepteurs de l'ATP, tels que présentés dans la liste actualisées annuellement et éditée dans les bases de données GPCRdb (http://www.gcrdb.uthscsa.edu ou http://www.gpcr.org), ensembl (http://www.ensembl.org) entre autres.
3) les récepteurs nucléaires possédant un domaine d'interaction avec l'ADN structurellement reliés au récepteur des stéroïdes (Mangelsdorf et al., 1995 ; Wurtz et al., 1996).
4) les récepteurs de la membrane plasmique à activité tyrosine kinase structurellement reliés au récepteur de l'insuline (Yarden, Y. and Ullrich, A., 1988).
5) les récepteurs membranaires couplés aux protéines tyrosine kinases (STATs, TYK2, Jak) structurellement reliés au récepteur de l'interféron γ (Brisco et al., 1996 ; Ihle, 1995).

Dans le cas où la fusion est effectuée entre la EGFP et un récepteur couplé aux protéines G (groupe 1), la fusion peut être notamment effectuée :
1) du coté N-terminal du récepteur, et donc du côté C-terminal de la EGFP,
2) du coté C-terminal du récepteur et donc du côté N-terminal de la EGFP,
3) dans la séquence du récepteur, en particulier dans la première ou la troisième boucle intracellulaire, éventuellement en introduisant une ou plusieurs copies d'une séquence espaceur, notamment -GGGGS-.

Dans le cas où la fusion est effectuée entre la EGFP et un récepteur-canal (groupe 2), la fusion peut être notamment effectuée :
1) dans la région homologue à la "région immunogénique majeure" de la sous-unité α du récepteur nicotinique de Torpille (résidus 67-76), éventuellement en introduisant une ou plusieurs copies d'une séquence espaceur, notamment -GGGGS-.

Dans le cas ou la fusion est effectuée entre la EGFP et un récepteur nucléaire (groupe 3), la fusion peut être notamment effectuée :
1) du coté N-terminal du récepteur, et donc du côté C-terminal de la EGFP,
2) du coté N-terminal du récepteur, tronqué dans sa partie N-terminale en amont du domaine de liaison à l'ADN, et donc du côté C-terminal de la EGFP.

Dans le cas où la fusion est effectuée entre la EGFP et un récepteur soit à activité tyrosine kinase, soit couplé à une tyrosine kinase (groupes 4 et 5), la fusion peut être notamment effectuée :
1) du coté N-terminal du récepteur, et donc du coté C-terminal de la EGFP.

Tout gène codant pour une protéine fluorescente, notamment la GFP, couplée à un récepteur, et dérivant d'organismes exprimant la GFP ou des protéines similaires pourrait être utilisé dans cette invention.

Les séquences d'ADN codant pour la GFP et les protéines cibles, notamment des récepteurs, peuvent être d'origine génomique ou être des ADNc, et peuvent être obtenus à partir de l'ADN de n'importe quelle espèce animale ou végétale, eucaryote ou procaryote, par exemple en préparant des banques génomiques ou des banques d'ADNc et en criblant ces banques pour identifier les séquences codantes par hybridation avec des sondes oligonucléotidiques par les techniques standard (Current Protocols in Molecular Biology, op. cit.).

Les constructions d'ADN codant pour la GFP et les protéines cibles peuvent aussi être obtenues par synthèse totale par les méthodes standards, notamment la méthode des phosphoramidites (Beaucage and Caruthers, 1981) et l'emploi d'appareils de synthèse d'ADN automatisés, les polynucléotides obtenus étant ensuite purifiés, ligués et clonés dans les vecteurs appropriés. Pour la plupart des applications, les gènes codant pour la GFP et les protéines cibles seront préférentiellement obtenus par criblage de banques, tandis que les bras espaceurs ainsi que les oligonucléotides requis pour la mutagenèse seront préférentiellement obtenus par synthèse.

Les constructions d'ADN pourront être de nature mixte, synthétique et génomique, par ligation de fragments synthétiques avec des élément d'ADN génomique, selon des procédures standard (Current Protocols in Molecular Biology, op.cit.).

Les constructions d'ADN peuvent aussi être obtenues par PCR ("polymerase chain reaction") en employant des amorces spécifiques, comme par exemple décrit dans PCR protocol 1990, Academic press, San Diego, California, USA.

Enfin, les constructions d'ADN peuvent être modifiées par d'autres méthodes incluant par exemple des réactions chimiques, de la mutagenèse aléatoire ou dirigée, par insertion, délétion ou substitution de nucléotides, ces modifications pouvant altérer des propriétés de l'une ou l'autre protéine, notamment, la GFP et les protéines cibles.

Les constructions d'ADN peuvent être insérées dans un vecteur recombinant. Ce vecteur peut être n'importe quel vecteur approprié pour les procédures employées avec des vecteurs recombinants. Le choix du vecteur sera souvent effectué en fonction de la cellule hôte dans laquelle la construction d'ADN voudra être introduite. Le vecteur pourra ainsi être un vecteur capable de se répliquer de manière autonome, c'est-à-dire extrachromosomal, et indépendante de la réplication chromosomale, par exemple un plasmide. Alternativement, le vecteur pourra être élaboré de manière à intégrer tout ou partie de l'ADN qu'il contient dans le génome de la cellule hôte, et se répliquera en même temps que le(s) chromosome(s) dans lequel il sera intégré.

Le vecteur est préférentiellement un vecteur d'expression dans lequel la GFP fusionnée au récepteur ou la GFP fusionnée au ligand est sous le contrôle d'autres segments d'ADN requis pour la transcription. En général, le vecteur d'expression dérive d'ADN plasmidique ou viral ou peut contenir des éléments de l'un et de l'autre.

Le terme "sous le contrôle" indique que les segments d'ADN sont disposés sur le vecteur de manière à fonctionner de concert pour servir l'objectif voulu, par exemple, la transcription est initiée dans le promoteur et se poursuit tout au long de la séquence codant pour le récepteur fusionné à la GFP ou le ligand fusionné à la GFP.

Le promoteur peut être n'importe quelle séquence d'ADN susceptible de promouvoir une activité transcriptionnelle dans la cellule hôte choisie et peut être dérivé de gène homologues ou hétérologues à la cellule hôte.

Des exemples de promoteurs convenant pour l'expression du récepteur fusionné avec le GFP ou du ligand fusionné avec la GFP dans des cellules mammifères sont le promoteur du virus simien SV40 (Subramani et al., 1981), le promoteur du virus du sarcome de Rous (RSV), le promoteur du cytomegalovirus (CMV) ou le promoteur tardif majeur de l'adénovirus (AdMLP).

### Exemples de promoteurs pour cellules d'insectes :

Promoteur de la polyhédrine (US 4,745,051 ; Vasuvedan et al., 1992) le promoteur P10 (Vlack et al., 1988), le promoteur du gène précoce 1 du baculovirus (US 5,155,037; US 5,162,222).

### Exemples de promoteurs pour levures :

Promoteurs des gènes de la glycolyse (Hitzeman et al., 1980 ; Alber et Kawasaki, 1982), des gènes des alcool deshydrogénases (Young et al., 1982).

### Exemples de promoteurs pour bactéries :

Des exemples de promoteurs pour l'expression dans la bactérie peuvent être des promoteurs constitutifs comme le promoteur de la polymérase T7, ou des promoteurs inductibles comme par exemple le promoteur pL du phage lambda (Current Protocols in Molecular Biology, op.cit.).

### Exemples de promoteurs pour champignons filamenteux :

Les promoteurs utilisables sont par exemple le promoteur ADH3 (McKnight et al., 1985) ou le promoteur tpiA. D'autres promoteurs utiles peuvent être dérivés des gènes codant pour l'aspartate protéinase de *Rhizomucor miehei,* l'alpha-amylase neutre de *Aspergillus niger,* de l'acétamidase de *Aspergillus nidulans,* de la TAKA amylase de *Aspergillus oryzae* ou le promoteur de la glucoamylase de *Aspergillus awamori.*

Le vecteur pourra par ailleurs contenir :
- des séquences de polyadénylation, comme par exemple celles de SV40 ou de la région Elb 5 de l'adénovirus,
- des séquences activatrices (enhancer) de la transcription (l'activateur de SV40),
- des séquences de réplication comme par exemple les séquences de réplication de SV40 ou du virus Epstein Barr, pour les cellules mammifères ou l'origine et les gènes de réplication REP 1-3 du plasmide 2 µ, pour les levures,
- des marqueurs de sélection, à savoir des gènes conférant une résistance à un antibiotique (néomycine, zéocine, hygromycine, ampicilline, kanamycine, tétracycline, chloramphénicol...) ou permettant la compensation d'un défaut (gène codant pour la dihydrofolate réductase permettant la résistance au méthotrexate, ou gène TPI de S. pombe décrit par Russell (1985).

La cellule hôte peut être n'importe quelle cellule capable d'exprimer la construction d'ADN insérée dans un vecteur approprié.

Les cellules peuvent être notamment des bactéries, des levures, des champignons et des cellules eucaryotes supérieures comme par exemple des cellules mammifères.

Des exemples de cellules bactériennes capables d'exprimer les constructions d'ADN sont :
- des bactéries gram-positives telles que les souches de Bacillus comme *B. subtilis, B. licheniformis, B. lentus, B. brevis, B. strearothermophilus, B. thurigiensis* ou des souches de *Streptomyces* comme *S. lividans, S murinus,*
- des bactéries gram-négatives telles *Escherichia coli.*
   La transformation des bactéries peut être effectuée par transformation protoplastique ou par transformation de bactéries compétentes (Current Protocols in Molecular Biology, op.cit.).

### Exemples de cellules eucaryotes :

Lignées cellulaires HEK 293, HeLa, cultures primaires, cellules COS (par exemple ATCC CRL 1650), BHK (par exemple ATCC CRL 1632) CHO (par exemple ATCC CCL 61).

Les méthodes d'introduction de l'ADN dans ces cellules (transfection, lipofection, électroporation etc.) sont décrites dans Current Protocols in Molecular Biology, op.cit.

### Exemples de cellules de levures :

*Saccharomyces, S. cerevisiae, S. kluyveri,*
*Kluiveromyces, K. lactis,*
*Hansenula, H. polymorpha,*
*Pichia, P. pastoris,*
transformées par introduction d'ADN hétérologue selon les protocoles décrits dans Current Protocols in Molecular Biology, op.cit.

Les cellules transformées sont sélectionnées par un phénotype déterminé par un marqueur de résistance, généralement à une drogue, ou par leur capacité à proliférer en l'absence d'un nutriment particulier.

### - Exemples de champignons filamenteux :

Les souches *Aspergillus* (*A. oryzae, A. nidulans, A. niger*), *Neurospora, Fusarium, Trichoderma.* L'utilisation de *Aspergillus* pour l'expression de protéines est décrite dans EP 272 277 ou EP 230 023 ou EP 184 438.

### Exemples de cellules d'insectes :

On peut citer les lignées de *Lepidoptera* e.g. *Spodoptera frugiperda* (Sf9) ou *Trichoplusia ni* (Tni). Les méthodes de transformation (infection en particulier) sont décrites dans Current Protocols in Molecular Biology (op.cit.).

### LES LIGANDS

Les ligands interagissant avec le récepteur peuvent être de n'importe quelle origine (naturelle, synthétique, semi-synthétique, recombinante), et de n'importe quelle structure (chimique, peptidique, protéique). Ils peuvent être naturellement fluorescents (ou porteurs d'un chromophore) ou peuvent nécessiter soit une réaction chimique permettant le greffage d'un groupe fluorescent (ou d'un précurseur de groupe fluorescent) ou d'un chromophore, soit une construction d'ADN conduisant à la fusion du ligand avec la GFP et permettant l'expression du ligand ainsi rendu fluorescent.

Des exemples de réactions chimiques sont :
- couplage d'amines ou de thiols avec des réactifs de type halogénure d'alkyle, halogénures d'aryles, halogénures d'acyles, halogénures d'acides, le groupe isothiocyanate, le groupe maléimide, les époxydes, dans un solvant organique en présence d'une base ou en milieu aqueux,
- couplage d'acides avec des amines activées par des groupes tels que les succinimides.

Selon le procédé de l'invention, la fluorescence des cellules transformées peut être mesurée dans un spectrofluorimètre à l'aide duquel les propriétés spectrales des cellules, en suspension ou adhérentes, peuvent être déterminées par l'acquisition de leurs spectres d'excitation et d'émission. Les interactions avec le ligand fluorescent sont ensuite détectées par les changements des spectres d'excitation et/ou d'émission du donneur et de l'accepteur d'énergie, et les ligands sont définis comme pharmacologiquement significatifs si leurs interactions avec le récepteur sont inhibées par l'addition d'un excès de ligand non fluorescent empêchant l'interaction entre le récepteur fluorescent et le ligand fluorescent.

### LES MESURES DE LIAISON

Les mesures des cinétiques d'association et/ou de dissociation sont effectuées par tous les moyens permettant d'enregistrer la formation ou la dissociation du complexe entre le ligand marqué et le récepteur marqué, de manière continue ou discontinue, de telle sorte que l'on parvient à déterminer les paramètres cinétiques de l'association et de la dissociation, à savoir la (les) constantes de vitesse(s), ainsi que la ou les amplitudes relatives associées à chaque étape cinétique d'association et/ou de dissociation.

Dans sa réalisation préférée, les cinétiques dont les constantes de vitesse apparente sont supérieures à 0,1 s⁻¹ seront enregistrées à l'aide d'un appareil de mélange rapide connecté à un dispositif d'excitation et de détection de fluorescence (figure 1). Les échantillons à mélanger seront disposés dans des seringues (ou autres conteneurs) de mélange, puis mélangés rapidement. Dans ce contexte, "rapidement" veut dire que le contenu des seringues est propulsé avec un débit supérieur ou égal à 4 ml/sec pour une chambre d'observation de 100 µl, ainsi que décrit par exemple dans Palanché et al. (2001).

Dans le cas où les constantes de vitesse apparentes sont inférieures à 0,1 s⁻¹, l'utilisation d'un appareil de mélange rapide n'est pas indispensable. On pourra dans ce cas enregistrer les variations de fluorescence du donneur et/ou de l'accepteur dans un spectrofluorimètre standard pourvu d'un dispositif d'agitation magnétique de l'échantillon, d'une régulation de température et d'une fonction d'enregistrement en temps. Les échantillons sont alors disposés dans une cuvette munie d'un agitateur et le mélange sera obtenu par addition manuelle des composants ou solutions désirés. Les échantillons contenant le récepteur peuvent être soit des cellules, soit des fragments de cellules. Ils seront préférentiellement disposés en premier dans la cuvette et les agents modulateurs ainsi que le ligand marqué seront ajoutés à cette solution. L'effet de ces ajouts pourra ainsi être enregistré et toute variation de fluorescence pourra être caractérisée au plan physique et pharmacologique pour déterminer si elles relèvent ou non du processus de transfert d'énergie et répondent ou non aux critères de la spécificité pharmacologique recherchée. Dans sa réalisation préférée, une mesure de dissociation est obtenue par addition d'un excès de ligand compétitif. Ceci peut être obtenu :
- par addition d'un volume faible (≤ 5% du volume final) d'une solution concentrée de ligand compétitif (environ 500-1000 fois son K_{d} dans la solution finale, le Kₐ correspondant à la concentration conduisant à 50% d'occupation des sites) dans une cuvette contenant un mélange de ligand fluorescent, de récepteur fluorescent et éventuellement d'effecteur allostérique, ou
- par mélange rapide du contenu d'une seringue contenant le ligand fluorescent, le récepteur fluorescent et éventuellement l'effecteur allostérique avec celui d'une seringue contenant le ligand compétitif à la concentration désirée ainsi que l'effecteur allostérique à une concentration égale à celle de l'autre seringue (pour un mélange volume à volume) afin d'éviter de le diluer au cours de l'opération de mélange.

Dans la réalisation préférée du procédé, les mesures d'association sont effectuées par mélange du ligand fluorescent avec des cellules, membranes ou extraits, contenant le récepteur fluorescent avec le ligand fluorescent, en suivant la variation de fluorescence due au transfert d'énergie. L'effet de l'effecteur peut être étudié après mélange préalable avec le récepteur ou dans un protocole de mélange simultané avec le ligand fluorescent. Le mélange peut être effectué au moyen d'un appareil de mélange rapide ou au moyen d'un mélange manuel dans une cuvette de spectrofluorimètre.

### LES MESURES DE RÉPONSE

Etant donné la diversité des récepteurs et le nombre variable des états conformationnels qu'ils peuvent adopter, c'est la mesure de la modulation des réponses qui permet de définir le caractère positif ou négatif de l'effecteur allostérique.

On rappelle que dans ce contexte, le caractère positif ou négatif d'un effecteur allostérique est défini par sa capacité à potentialiser (effecteur positif) ou à déprimer (effecteur négatif) une réponse physiologique propre au récepteur étudié. L'effecteur allostérique positif sera considéré comme tel, quel que soit son effet (accélération ou ralentissement) sur la cinétique d'association et/ou de dissociation du ligand fluorescent marqueur, que ce dernier soit agoniste ou antagoniste.

Les différents tests utilisés pour mesurer les réponses ont été donnés plus haut.

Tous les paramètres d'une réponse peuvent être affectés par un effecteur allostérique positif. Ces paramètres incluent entre autres, la vitesse d'établissement de ia réponse, son amplitude, sa durée, sa fréquence, sa sensibilité à l'agoniste et le niveau de base de la réponse.

Dans sa réalisation préférée, le procédé est destiné à l'étude des récepteurs couplés aux protéines G. Pour ces récepteurs, les réponses pouvant faire l'objet d'une étude sont variées car ils sont couplés à de multiples voies de transduction de signal. On peut citer par exemple la liaison de GTP à la protéine G, la production d'AMPc, d'inositols phosphates, d'acide arachidonique, la phosphorylation de protéines, la libération de calcium intracellulaire, la modification de pH cellulaire, la modification de la vitesse de prolifération cellulaire, l'altération de la morphologie cellulaire, la polymérisation d'actine, ou encore la régulation de canaux ioniques ou celle de l'expression de gènes.

Chacune de ces réponses peut être enregistrée et les effets de l'effecteur peuvent être déterminés.

Dans les cas les plus répandus, on se focalisera en premier sur les réponses calciques et la production d'AMPc.

Pour les réponses calciques, on pourra employer une sonde optique sensible au calcium ou un enregistrement électrophysiologique de la régulation des courants. L'utilisation d'une sonde calcique permet de déterminer le délai d'établissement de la réponse, la durée de la réponse, son intensité ou sa sensibilité à l'agoniste. L'enregistrement électrophysiologique permet en outre l'analyse de la fréquence d'ouverture de canaux (Mulle et al., 1992). Un effecteur allostérique positif pourra diminuer le délai d'établissement de la réponse et augmenter les autres paramètres (amplitude, durée, sensibilité ...). L'effecteur allostérique négatif aura des effets contraires.

### DESCRIPTION DES FIGURES

La **figure 1** montre le schéma d'un appareil de mélange rapide permettant d'effectuer les mesures de cinétique rapide. L'appareil est constitué de deux seringues, d'une chambre de mélange et d'une chambre d'observation. L'avancée des pistons des seringues permet le mélange du contenu des seringues au niveau de la chambre de mélange. Après un temps mort, le mélange aboutit dans la chambre d'observation munie d'un dispositif d'excitation et de détection de la fluorescence. L'arrêt de la poussée des pistons, ou l'arrivée en butée de la seringue d'arrêt, stoppe l'étape de mélange au terme de laquelle on enregistre l'évolution du mélange.

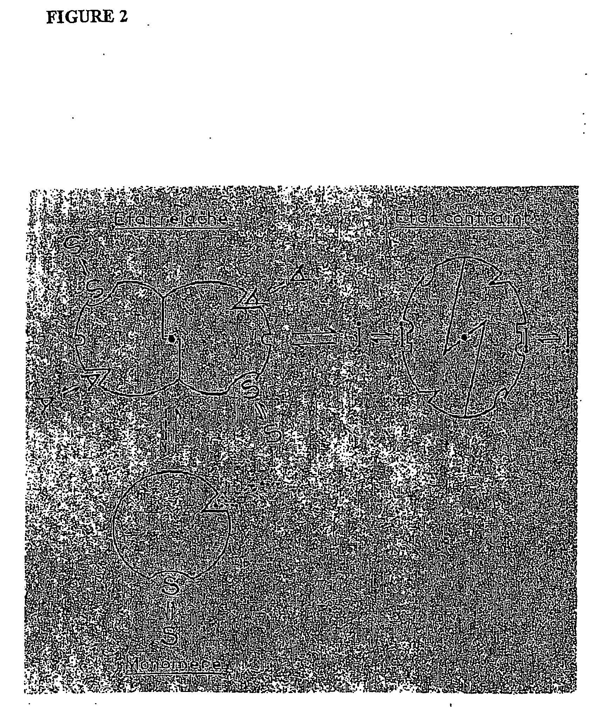

La **figure 2** (empruntée à Monod, Wyman et Changeux, 1965) représente un schéma de protéine régulatrice existant dans deux états conformationnels ou deux états d'oligomérisation. Dans ce modèle, la protéine peut exister dans plusieurs états discrets, en nombre fini, qui correspondent à des états thermodynamiquement stables qui diffèrent entre eux par leur structure tertiaire et quaternaire. L'interconversion entre chaque état peut s'opérer spontanément et est décrite par le paramètre d'isomérisation.

Les conformations diffèrent par leurs propriétés pharmacologiques. Ainsi, les ligands stabilisent les conformations pour lesquelles ils exhibent l'affinité la plus élevée. Les conformations diffèrent par leurs propriétés fonctionnelles. Ainsi les agonistes stabilisent préférentiellement l'état actif, tandis que les antagonistes stabilisent préférentiellement l'état inactif.

Dans ce schéma, l'état relâché correspond à l'état conformationnel "actif" de la protéine. Cette conformation peut lier avec une haute affinité les agonistes (A) si c'est un récepteur ou les substrats (S) si c'est une enzyme. Les états contraint et/ou monomère sont moins (ou pas) actifs en comparaison à l'état relâché, qui est le plus actif. L'état contraint lie avec une haute affinité les inhibiteurs ou antagonistes. L'état monomérique lie avec une affinité modérée les agonistes ou les substrats.

Les **figures 3A et 3B** présentent un schéma quantifié des relations entre les états conformationnels et les interactions avec les ligands.

Dans la **figure 3A**, la protéine existe dans deux conformations correspondant à un état de repos (non actif au plan biologique) et un état actif. L'isomérisation spontanée entre les états R et A est décrite par la constante d'isomérisation L₀ égale au rapport des concentrations relatives de chaque état (L₀ = [R]/[A]). Les ligands de la protéine se lient à l'état R avec une constante de dissociation égale à K_{R} et à l'état A avec une constante de dissociation égale à K_{A}. Le rapport c = K_{A}/K_{R} décrit le rapport d'affinité pour un ligand donné entre les conformations. Pour c est inférieur à 1, le ligand est agoniste (il augmente la concentration fractionnelle de A). Si c est inférieur ou égal à 1, le ligand est antagoniste (il augmente la concentration fractionnelle de R). L'équilibre entre les conformations R et A liées au ligand (RF ↔ AF) est décrit par le produit de L₀ par c.

En présence d'un effecteur allostérique (voir **figure 3B**), la constante d'isomérisation L₀ est altérée en L₀' selon L₀' = L[(1+(βd) / (1+β)]ⁿ où β (homologue de c) décrit le rapport d'affinité de l'effecteur pour les deux conformations (β = K_{A}/K_{R}) et d est la concentration d'effecteur normalisée par rapport à l'affinité pour l'état A (d = [effecteur]/ K_{A}). Les effecteurs dont β>1 sont inhibiteurs, ceux dont β<1 sont potentialisateurs.

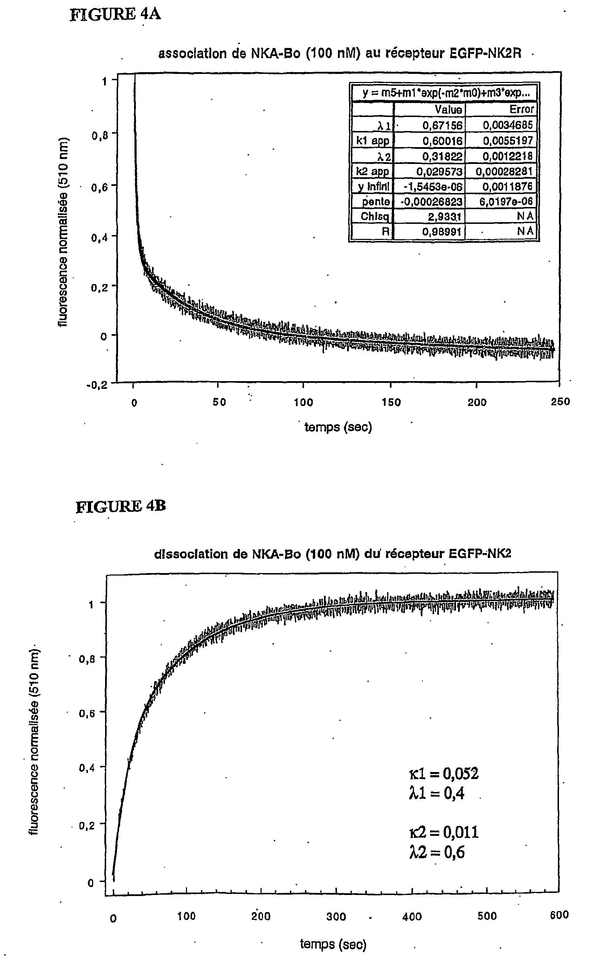

La **figure 4** représente un enregistrement en temps réel de la cinétique d'association (A) et de dissociation (B) de NKA-Bo (NKA marquée par Bodipy) au récepteur EGFP-NK2R (NK2R marqué par la GFP). L'enregistrement de la cinétique d'association est effectué après mélange rapide de cellules HEK 293 exprimant le récepteur EGFP-NK2R. La cinétique de dissociation est enregistrée après mélange manuel des cellules exprimant le récepteur avec la NKA-bo (pré-incubation de 15 minutes, 100 nanomolaires) puis avec l'antagoniste compétitif SR 4896 (10 µM final).

L'axe des abscisses représente le temps en secondes et l'axe des ordonnées représente la fluorescence (unités arbitraires) à 510 nm. Les valeurs λn et κn correspondent respectivement aux amplitudes et aux constantes de vitesses déterminées par un ajustement aux moindres carrés à deux exponentielles.

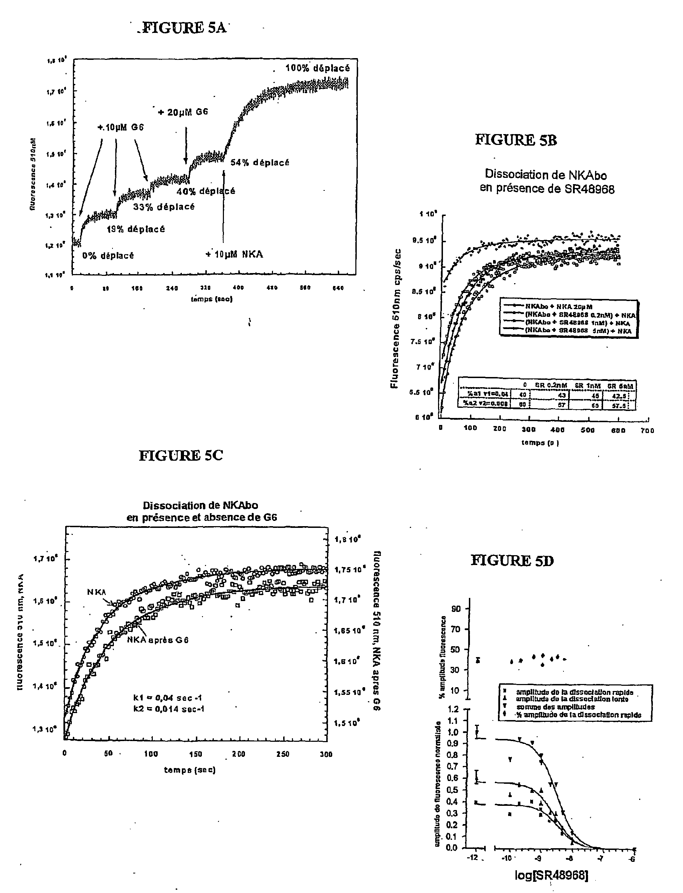

**Les figures 5A, 5B, 5C et 5D** indiquent la procédure d'identification d'un ligand interagissant de manière compétitive avec le ligand fluorescent sur un site récepteur.

Le complexe formé entre le récepteur EGFP-NK2R et la NKA-Bo est renversé par des concentrations croissantes de la molécule G6 (voir tableau 1) puis par la NKA non fluorescente.

La **figure 5A** indique qu'une partie du complexe récepteur-ligand est renversé lors de chaque ajout de molécule G6 (10 ou 20 µM), ainsi que lors de l'addition d'une concentration saturante de NKA (10 µM). Dans cette figure, l'axe des abscisses correspond au temps en secondes et l'axe des ordonnées à la fluorescence mesurée à 510 nm.

La **figure 5B** montre que la cinétique de dissociation de la NKA-Bo, déterminée après addition d'une concentration saturante de NKA n'est pas modifiée par la présence de la molécule G6.

Les deux traces expérimentales sont ajustées à l'aide de 2 exponentielles rapide (0,04 s⁻¹) et lente (0,014 s-¹) dont les amplitudes (66% pour la rapide et 33% pour la lente) ne sont pas affectées par la présence de 50 µmoles de molécule G6.

Dans cette figure, l'axe des abscisses correspond au temps en secondes et l'axe des ordonnées à la fluorescence mesurée à 510 nm.

La **figure 5C** représente une étude quantitative de la cinétique de dissociation de NKA-Bo en présence d'un antagoniste compétitif connu, le SR 48968. La dissociation de NKA-Bo est enregistrée en présence de 0,2 ; 1 et 5 nM de SR 48968. Dans les trois conditions expérimentales, la vitesse de dissociation peut être décrite comme une somme de deux exponentielles dont les vitesses sont 0.04 sec⁻¹ et 0.008 sec⁻¹, et dont les amplitudes relatives restent constantes aux différentes doses de SR 48968 (45% de dissociation rapide (0,04 s⁻¹) et 55% de dissociation lente (0,008 s⁻¹)).

Dans cette figure, l'axe des abscisses correspond au temps en secondes et l'axe des ordonnées à la fluorescence mesurée à 510 nm.

La **figure 5D** représente les valeurs des amplitudes de dissociation rapide (carrés) et lente (triangles vers le haut) de NKA-Bo en présence de SR 48968, ainsi que l'amplitude totale de la liaison de NKA-Bo (triangles vers le bas).

Dans cette figure, l'axe des abscisses correspond au logarithme de la concentration en SR 48968 et l'axe des ordonnées à l'amplitude de la fluorescence normalisée:

La partie supérieure de la figure 5D représente le rapport de l'amplitude de la dissociation lente (A2) par rapport à l'amplitude totale de dissociation (A totale)(courbe avec les losanges). On note que ce rapport est constant. Dans ce graphique, l'axe des abscisses correspond au logarithme de la concentration en SR 48968 et l'axe des ordonnées au pourcentage de l'amplitude de la fluorescence.

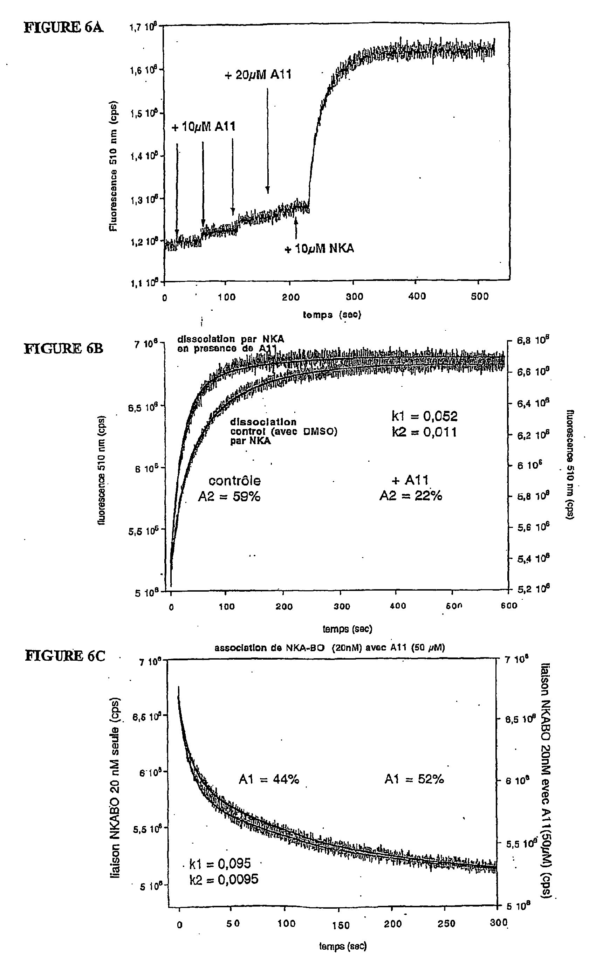

- Les **figures 6A, 6B et 6C** indiquent la procédure d'identification d'un ligand interagissant de manière non compétitive avec le ligand fluorescent sur un site récepteur.

Dans la **figure 6A**, on a représenté la fluorescence mesurée à 510 nm en fonction du temps en secondes.

Le complexe formé entre le récepteur EGFP-NK2R et la NKA-Bo est renversé par des concentrations croissantes de la molécule A11 puis par la NKA non fluorescente. La figure 6A indique qu'une faible portion du complexe récepteur - ligand est renversé lors de chaque ajout de molécule A11, tandis que la NKA (10 µM) renverse la majorité du complexe. La cinétique de la dissociation par NKA n'est pas de même forme que dans le contrôle.

Dans la **figure 6B,** on a représenté la fluorescence mesurée à 510 nm en fonction du temps en secondes. La figure 6B illustre la modification de cinétique de dissociation en présence de molécule A11. Les enregistrements de dissociation de NKA-Bo de son récepteur EGFP-NK2R sont effectués en présence et en absence de A11. La dissociation est initiée par l'ajout de NKA non fluorescente à une concentration de 20 µM finale. L'analyse de la cinétique de dissociation fait apparaître deux relaxations monoexponentielles dont les constantes de vitesses (k1 et k2) sont identiques en présence et en absence de A11 mais dont les amplitudes relatives changent de manière à ce que la dissociation lente représente 59% du signal total dans le contrôle et 22% du signal total en présence de A11.

Dans la **figure 6C,** on a représenté l'association de NKABo (20 nanomolaires) au récepteur EGFP-NK2R, en fonction du temps en secondes. La figure 6C représente des enregistrements de liaison (association) résolue dans le temps de NKA-Bo à son récepteur EGFP-NK2R, en absence et en présence de la molécule A11. L'analyse de la cinétique de liaison fait apparaître deux relaxations monoexponentielles caractérisées par des constantes de vitesses (k1 et k2) ne changeant pas en présence de A11, mais d'amplitudes variables selon que les cellules sont incubées ou non avec A11.

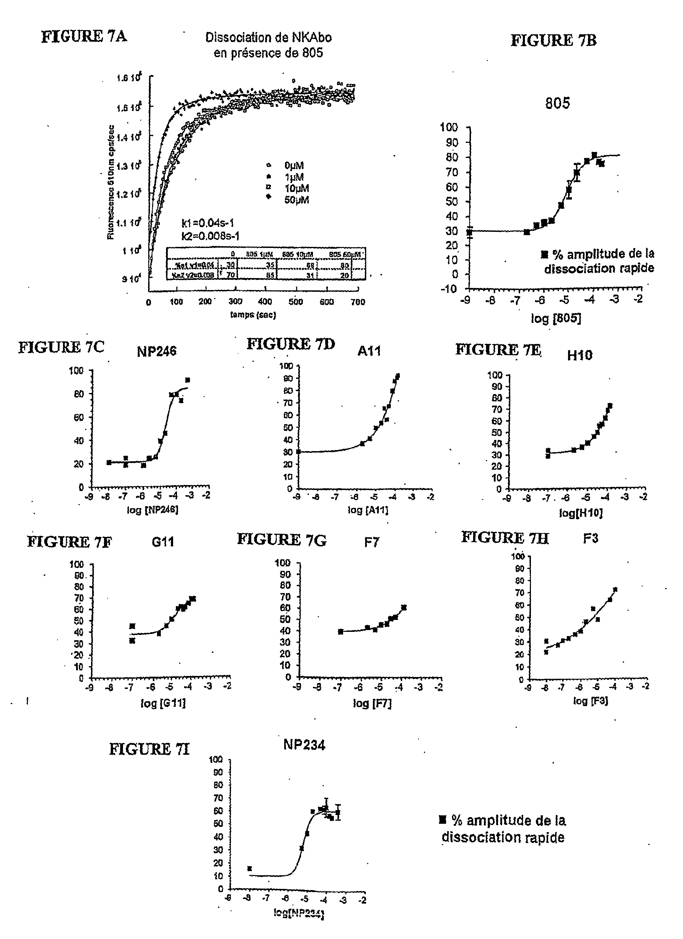

**Les figures 7A à 7I** illustrent l'analyse des effets de diverses molécules sur la dissociation de NKA-Bo liée à son récepteur fluorescent EGFP-NK2R.

La **figure 7A** représente les enregistrements de dissociation de NKA-Bo en présence de concentrations variables (1, 10 et 50 µM) de la molécule 805 (voir tableau 1). On a représenté la fluorescence mesurée à 510 nm en fonction du temps en secondes. L'analyse quantitative de la cinétique de dissociation est obtenue à l'aide de deux exponentielles rapide (0.04 sec⁻¹) et lente (0.008 sec⁻¹) dont les amplitudes relatives (rapide/lente) varient de 30/70 à 80/20 quand la concentration de 805 augmente.

Dans les **figures 7B** à **7I,** on a représenté le pourcentage d'amplitude de la dissociation rapide en fonction du logarithme de la concentration des composés testés. Ces figures donnent les résultats de l'analyse quantitative de la dissociation de NKA-Bo en présence des molécules 805 (figure 7B), NP246 (figure 7C), A11 (figure 7D), H10 (figure 7E), G11(figure 7F), F7 (figure 7G), F3 (figure 7H) et NP 234 (figure 7I). La dissociation de NKA-Bo a été déterminée en présence de chaque molécule aux concentrations indiquées sur l'axe des abscisses. L'effet d'augmentation de la vitesse de dissociation est représenté par l'augmentation relative de l'amplitude (%λ1) de la dissociation rapide.

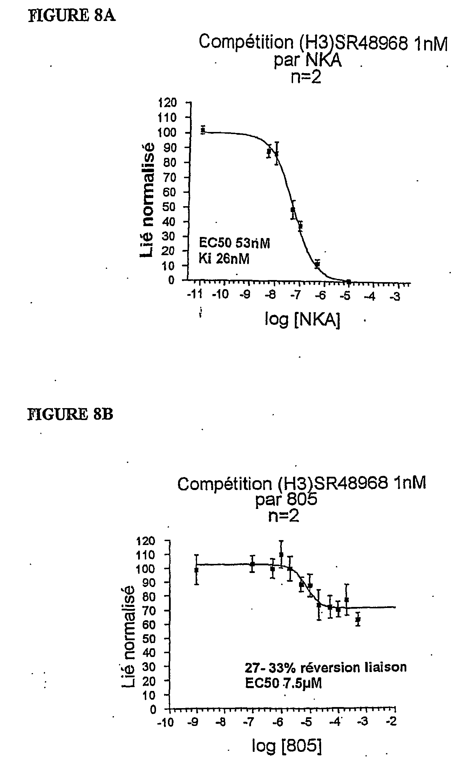

Les **figures 8A** et **8B** présentent une quantification du déplacement de [³H]SR 48968, lié au récepteur EGFP-NK2R, par la NKA et la molécule 805.

Dans ces figures, on a représenté la liaison de 1 nanomolaire [³H]SR 48968 (Amersham, Life Sciences) en fonction du logarithme de la concentration en NKA (figure 8A) ou en fonction de la concentration en molécule 805 (figure 8B).

On note que la NKA (ligand compétitif) renverse 100% de la liaison de [³H]SR 48968 tandis que 805 (ligand non compétitif) ne renverse qu'environ 35% de la liaison de SR 48968.

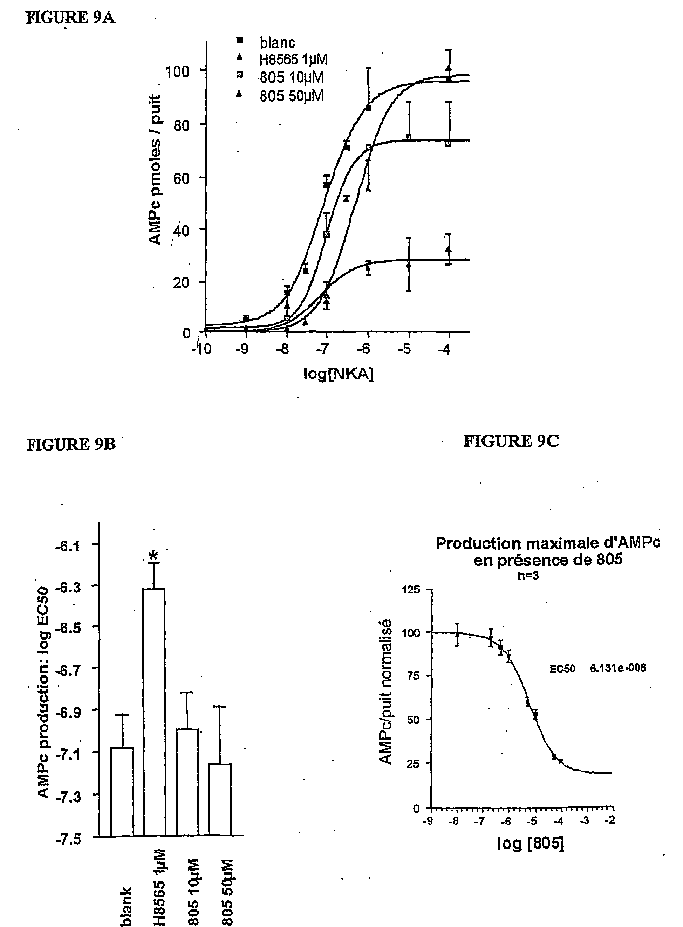

Les **Figures 9A, 9B** et **9C** présentent les résultats de stimulation de production d'AMPc par la NKA dans des cellules HEK 293 exprimant le récepteur EGFP-NK2R.

La **figure 9A** représente l'effet de la présence d'antagoniste compétitif (H8565) et d'effecteur allostérique (805). Dans cette figure, on a représenté l'accumulation d'AMPc intracellulaire (en pmoles/puits) en fonction du logarithme de la concentration en NKA. La courbe avec les carrés noirs correspond au blanc ; la courbe avec les triangles noirs correspond à l'antagoniste H8565 à une concentration de 1 µM; la courbe avec les carrés gris correspond au ligand 805 à une concentration de 10 µM et la courbe avec les triangles gris correspond au ligand 805 à une concentration de 50 µM.

L'accumulation d'AMPc intracellulaire est déterminée à différentes concentrations de NKA. L'antagoniste H8565 déplace la courbe concentration-réponse de NKA vers des valeurs plus élevées sans affecter l'intensité de la réponse maximale (triangles noirs). Le ligand 805 (carrés et triangles gris) diminue l'intensité de la réponse maximale sans affecter l'EC₅₀ (concentration efficace à 50%) de la NKA pour la réponse.

La **figure 9B** représente la concentration efficace à 50% de la réponse maximale d'AMP cyclique à la NKA. Cette EC₅₀ augmente en présence d'antagoniste compétitif tandis qu'elle n'est pas modifiée de manière significative en présence de 10 et 50 µM de 805.

La **figure 9C** représente l'inhibition de la réponse maximale d'AMPc induite par la NKA en présence de concentration croissante de 805. L'abscisse représente le logarithme de la concentration en 805 et l'ordonnée la valeur maximale de la production d'AMP cyclique. La constante d'inhibition KI est de l'ordre de 6 µM.

Les **Figures 10A, 10B, 10C, 10D** et **10E** illustrent l'effet de 805 sur les réponses calciques associée au récepteur EGFP-NK2R dans les cellules HEK 293.

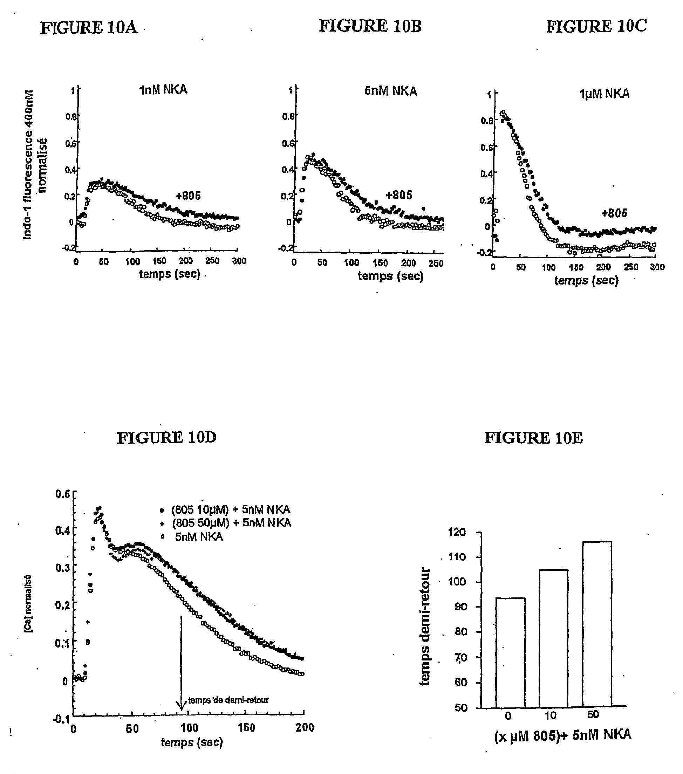

Dans les **figures 10A, 10B** et **10C,** on a représenté la fluorescence de l'INDO-1 à 400 nm en fonction du temps en secondes.

Les réponses sont enregistrées à l'aide de la sonde fluorescente sensible au calcium INDO-1. Pour chaque concentration d'agoniste NKA (1nM, 5 nM et 1 µM) on mesure une réponse contrôle (cercle blanc) et une réponse en présence de 50 µM de 805 (cercle noir)(figures 10A, 10B et 10C). Dans tous les cas, on note une augmentation de la durée de la réponse calcique en présence de 805, sans effet significatif sur l'amplitude ou sur la cinétique de mise en place de la réponse.

La **figure 10D** indique les variations de réponses calciques obtenues pour une concentration d'agoniste fixe (5 nM de NKA) et des concentrations variables (10 et 50 µM) de 805. On a représenté la concentration en calcium normalisée en fonction du temps en secondes. La courbe avec les cercles blancs correspond à une mesure effectuée en présence de 5 nM de NKA et sans 805 ; la courbe avec les cercles noirs correspond à une mesure effectuée en présence de 5 nM de NKA et de 10 µM de 805 ; et la courbe avec les carrés noirs correspond à une mesure effectuée en présence de 5 nM de NKA et de 50 µM de 805.

Dans tous les cas on note une augmentation de la durée de la réponse calcique en présence de 805.

- La **figure 10E** présente une quantification du temps de demi retour (temps requis pour que la concentration de calcium soit la moitié de sa valeur maximale) à la valeur initiale de la réponse calcique enregistrée en présence de 0, 10 et 50 µM de 805.

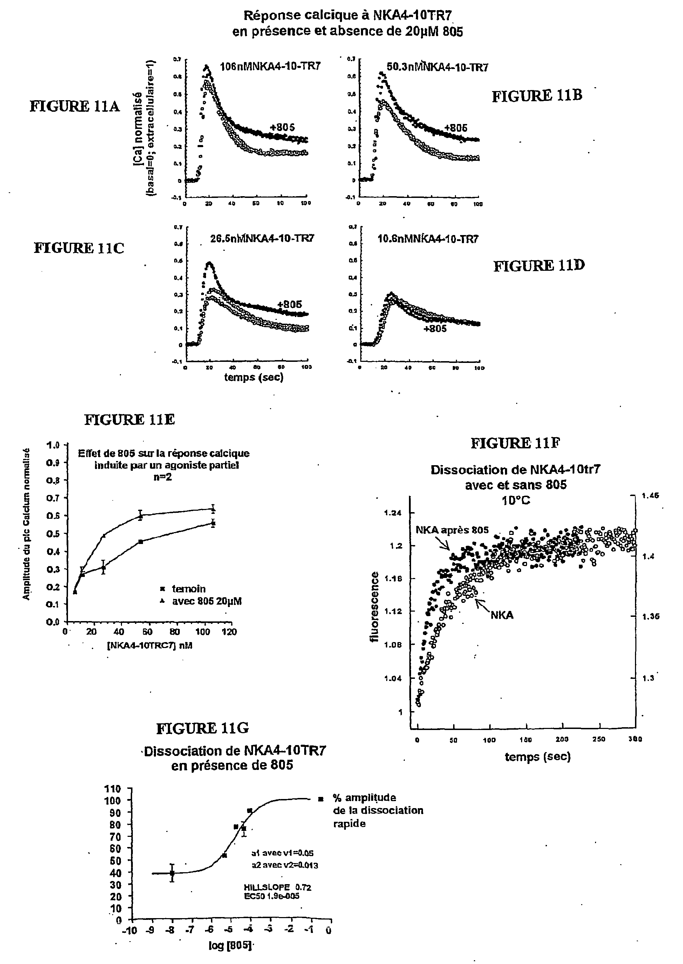

Les **figures 11A, 11B, 11C, 11D, 11E, 11F et 11G** illustrent l'effet de 805 sur la liaison de neurokinine tronquée fluorescente (NKA4-10 TR7) marquée avec le groupe fluorescent Texas red et la réponse calcique associée.

Dans les **figures 11A à 11D,** les réponses calciques à 106 (figure 11A), 50,3 (figure 11B), 26,5 (figure 11C) et 10,6 nM (figure 11D) de NKA4-10 TR7 (NKA4-10 marquée en position 7 par le Texas Red) sont enregistrées en double, en absence (courbes avec les cercles blancs) et en présence de 20 µM de 805 (courbes avec les cercles noirs). On a représenté la concentration en calcium normalisée en fonction du temps en secondes. On note que 805 provoque une augmentation de l'amplitude de la réponse calcique, et une persistance du signal.

**Le figure 11E** représente la relation entre l'amplitude de la réponse calcique et la concentration d'agoniste. On a représenté l'amplitude du pic calcium normalisé par rapport à la concentration de calcium extracellulaire, en fonction de la concentration en NKA4-10 TR7. La courbe avec les carrés noirs correspond à la mesure témoin et (sans 805) et la courbe avec les triangles correspond à la mesure effectuée en présence de 20 µM de 805.

La **figure 11F** représente la dissociation de la liaison de NKA 4-10 TR7. L'axe des abscisses représente le temps en secondes et l'axe des ordonnées représente la fluorescence. Le complexe formé entre le récepteur EGFP-NK2R et la NKA4-10 TR7 (106 nM) est renversé (à 10°C) par la NKA non fluorescente (20 µM) en absence et en présence de 10 µM de 805. L'analyse de la cinétique de dissociation fait apparaître deux relaxations monoexponentielles dont les constantes de vitesses (0,05 sec⁻¹ et 0,013 sec⁻¹) sont identiques en présence et en absence de 805, mais dont les amplitudes relatives changent de manière à ce que la dissociation lente représente 42% du signal total dans le contrôle et 12% du signal total en présence de 805.

Dans la figure 11G, on a représenté le pourcentage d'amplitude de la dissociation rapide de NKA 4-10 TR7 en fonction du logarithme de la concentration en 805. L'amplitude de la dissociation rapide de NKA 4-10 TR7 est mesurée en présence de concentrations variables de 805. La dissociation rapide représente environ 40% de la dissociation totale en absence de 805 et jusqu'à 90% de l'amplitude de dissociation à 100 µM. L'affinité apparente pour la variation d'amplitude est de l'ordre de 20 µM.

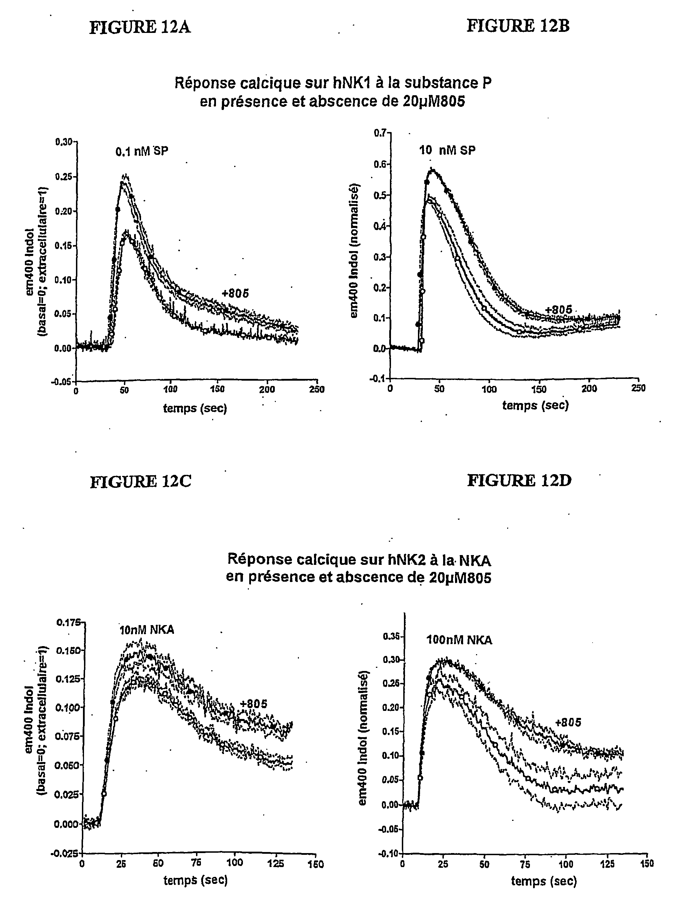

Les **figures 12A, 12B, 12C et 12D** représentent les réponses calciques évoquées par la substance P (ligand endogène) sur le récepteur NK1 humain (figure 12A et 12B) et par la neurokinine A sur le récepteur NK2 humain (figures 12C et 12D), en présence et en absence de 805.

Dans les **figures 12A et 12B,** une suspension (1 × 10⁶ cellules/ml) de cellules HEK 293 exprimant le récepteur NK1 humain sauvage, et chargées en indo-1 (3 µM) est stimulée par 0,1 nM (figure 12A) ou 10 nM (figure 12B) de substance P (SP) en présence (cercles noirs) et en absence (cercles blancs) de molécules 805 (20 µM).

Dans les **figures 12C et 12D**, une suspension (1 × 10⁶ cellules/ml) de cellules HEK 293 exprimant le récepteur NK2 humain sauvage, et chargées en indo-1 (3 µM) est stimulée par 10 nM (figure 12C) ou 100 nM (figure 12D) de neurokinine A (NKA) en présence (cercles noirs) et en absence (cercles blancs) de molécules 805 (20 µM).

Dans tous les cas, on note que la molécule 805 provoque une augmentation de l'amplitude et de la durée de la réponse calcique évoquée par chaque agoniste.

### EXEMPLES

### EXEMPLE 1 : Mesure de la vitesse d'association et de dissociation de neurokinine A marquée par le fluorophore bodipy au récepteur NK2 des tachykinines marqué avec le EGFP, par transfert d'énergie de fluorescence

Association (Figure 4A) : les cellules HEK 293 exprimant le récepteur EGFP-NK2R (NK2R marqué avec la GFP) sont mises en suspension à une concentration de 2 000 000 cellules/ml dans du tampon physiologique Hepes (en mM : 137,5 NaCl ; 1,25 MgCl₂; 1,25 CaCl₂ ; 6 KCl ; 5,6 glucose ; 10 Hepes ; 0,4 NaH₂PO₄; 1% BSA (w/v) ; pH 7,4) et disposées dans l'un des réservoirs de l'appareil de mélange rapide. Une solution de NKA-BO (neurokinine A marquée avec le Bodipy)(200 nM) dans le même tampon est disposée dans l'autre réservoir de l'appareil de mélange rapide. La chambre d'observation est disposée dans un spectrofluorimètre SPEX fluorolog 3. La température des seringues réservoir ainsi que de la chambre de mélange et d'observation est fixée à 21°C. La longueur d'onde d'excitation de la chambre d'observation est fixée à 470 nm, et la longueur d'onde d'émission de fluorescence est fixée à 510 nm. La fréquence d'échantillonnage des points expérimentaux est fixée à 50 Hz (1 point toutes les 20 msec). Le contenu des réservoirs est propulsé vers la chambre de mélange et d'observation à l'aide d'un dispositif pneumatique permettant un débit de 4 ml/sec et l'évolution du mélange est enregistrée en continu à partir du moment d'arrêt de la poussée des pistons des réservoirs. La réaction d'association de NKA-Bo avec le récepteur EGFP-NK2R est détectée sous la forme d'une diminution de l'intensité de fluorescence de la GFP porte par le récepteur NK2R. La trace expérimentale est ajustée par une courbe multiexponentielle de type y = λ₁ exp (-k1ₐₚₚ × T) + λ₂ exp (-k2ₐₚₚ × T) + "droite", où λ₁ et λ₂ sont les amplitudes des relaxations rapide et lente, k1ₐₚₚ et k2ₐₚₚ sont les constantes de vitesse apparente d'association rapide et lente, T est le temps et "droite" est une correction mathématique de la dérive du signal. Les valeurs de λ₁, λ₂, k1ₐₚₚ, k2ₐₚₚ pour une concentration de NKA-BO finale de 100 nM sont respectivement 67% ; 32% ; 0,6 sec⁻¹ et 0,03 sed⁻¹. Le coefficient de corrélation de l'ajustement de la courbe expérimentale est R = 0,989.

- Dissociation (figure 4B) : Les cellules HEK 293 exprimant le récepteur EGFP-NK2R sont mises en suspension à une concentration de 1 000 000 cellules/ml dans du tampon physiologique Hepes (en mM : 137,5 NaCl ; 1,25 MgCl₂ ; 1,25 CaCl2 ; 6 KCl ; 5,6 glucose ; 10 Hepes ; 0,4 NaH₂PO₄ ; 1% BSA (w/v) ; pH 7,4) et disposées dans une cuvette de 1 ml munie d'un barreau d'agitation magnétique sur le porte cuve d'un spectrofluorimètre SPEX fluorolog 3. On y ajoute 20 nM (concentration finale) de NKA-Bo et on laisse l'association atteindre l'équilibre pendant 10 minutes. La mesure de la cinétique de dissociation de NKA-Bo est initiée par l'ajout manuel de 10 µM (concentration finale) de l'antagoniste compétitif SR 48968 et enregistrée à 510 nm (excitation : 470 nm) à raison de 1 point toutes les 100 msec. L'ajustement de la courbe expérimentale est obtenu à l'aide d'une somme de deux exponentielles ayant pour constantes de vitesse k₁ = 0,052 sec⁻¹ et k₂ = 0,011 sec⁻¹ et pour amplitudes λ₁ = 40% et λ₂ = 58%, respectivement.

### EXEMPLE 2 : Identification de la molécule G6 se comportant comme un ligand compétitif, par transfert d'énergie de fluorescence entre la NKA-Bo et le récepteur EGFP-NK2R.

10⁶ cellules HEK 293 exprimant le récepteur EGFP-NKR2 sont incubées, dans un millilitre de tampon Hepes, avec 20 nm de NKA-Bo pendant 15 minutes. Le mélange est ensuite placé dans une cuvette de spectrofluorimétrie et la réversion de l'interaction entre EGFP-NK2R et NKA-Bo est évaluée après addition de molécule G6 (voir tableau 1) en concentration croissante (Figure 5A). La quantification de la fraction de complexe renversé est ensuite effectuée après addition d'une quantité saturante de NKA (10 µM) qui donne accès à la valeur de 100% de réversion de la liaison.

La comparaison de la cinétique de dissociation de NKA-Bo par la NKA, en présence et en absence de molécule G6 (50 µM), montre que la molécule G6 n'altère pas la vitesse de dissociation de NKA-Bo (figure 5B). Les deux courbes expérimentales sont correctement ajustées à l'aide de deux exponentielles avec une constante de vitesse rapide k₁ = 0,04sec⁻¹ et une constante de vitesse lente k₂ = 0,01 sec⁻¹.

L'expérience contrôle présentée en figure 5C indique qu'une molécule connue, compétitive pour l'interaction de la NKA-Bo avec son site, ie 5R48968, provoque une dissociation de NKA-Bo avec une cinétique identique à celle qui est enregistrée lorsque la NKA-Bo est déplacée par le NKA. Aux différentes concentrations testées de SR 48968, on note que la dissociation de NKA-Bo se fait selon un processus biphasique incluant une étape de dissociation rapide (k₁ = 0,04 sec⁻¹) et lente (k₂ = 0,008 sec⁻¹). L'amplitude relative de chaque étape reste constante pour chaque concentration de SR 48968, ainsi qu'en attestent les rapports d'amplitudes rapide/lent voisins de 45/55.

L'analyse quantitative de la dissociation de NKA-Bo en présence d'une gamme de concentrations croissantes de SR 48968 révèle que pour chaque concentration de SR 48968, la dissociation se produit non seulement selon un processus biphasique avec deux constantes de vitesses (voir figure 5C) mais en outre que le rapport d'amplitude de la dissociation rapide et de la dissociation lente reste constant (figure 5D). La dissociation lente représente toujours environ 43% du signal "dissociation rapide + dissociation lente".

### EXEMPLE 3 : Identification d'une molécule se comportant comme un effecteur allostérique du récepteur NK2 des tachykinines

10⁶ cellules HEK 293 exprimant le récepteur EGFP-NKR2 sont incubées, dans un millilitre de tampon Hepes, avec 20 nM de NKA-Bo pendant 15 minutes. Le mélange est ensuite placé dans une cuvette de spectrofluorimétrie et la réversion de l'interaction entre EGFP-NK2R et NKA-Bo est évaluée après addition de molécule A11 en concentration croissante (Figure 6A). L'estimation de la fraction de complexe renversé est ensuite mesurée après addition d'une quantité saturante de NKA (10 µM). On voit que la molécule A11 ne déplace qu'une faible fraction (29%) de la NKA-Bo liée à son site. Par contre, on constate que lors de la dissociation de NKA-Bo par la NKA non fluorescente, la présence de A11 a pour effet d'accélérer la vitesse de dissociation de NKA-Bo. Ce phénomène est illustré sur la figure 6B où la cinétique de dissociation de NKA-BO par la NKA est mesurée en présence et en absence de molécule A11. Les deux cinétiques de dissociation sont mieux décrites par une somme de deux relaxations exponentielles dont les vitesses sont respectivement k₁ = 0,052 sec⁻¹ pour l'étape rapide et k₂ = 0,011 sec⁻¹ pour l'étape lente. Compte tenu de la vitesse d'association de NKA-Bo à son récepteur (kₒₙ = 5 × 10⁶ M⁻¹ sec⁻¹) on déduit que la NKA-Bo se lie à deux populations de sites dont les constantes de dissociation (K_{D}= k_{off}/kₒₙ) sont respectivement, 10 nM (dissociation rapide - 0,011 s⁻¹) et 2 nM (dissociation lente - 0,052 s⁻¹). L'amplitude de chaque relaxation est alors donnée par l'ajustement des traces expérimentales. Pour la dissociation contrôle (sans A11), la relaxation rapide représente 41% et la lente 59%. Après addition de A1 (50 µM), la relaxation rapide représente 78% du signal et la lente 22%. La molécule A11 a donc pour effet de stabiliser une fraction des récepteurs dans un état de plus faible affinité que celui dans lequel la NKA-Bo seule le stabilise. La molécule A11 quant à elle se lie de manière préférentielle à la conformation du récepteur qui présente une affinité plus modeste pour la NKA-Bo. La liaison de NKA-Bo n'est pas inhibée par A11. Elle se produit sur les mêmes états conformationnels du récepteur, mais les proportions de ces deux états conformationnels sont modifiées par la présence de A11. Il en résulte que la cinétique de dissociation de NKA-Bo est plus rapide en présence de A11 qu'en son absence. Cette accélération de la cinétique de dissociation de NKA-Bo ne résulte pas d'un changement des affinités intrinsèques des conformations pour la NKA-Bo, mais d'une modification des proportions relatives des états de haute et de faible affinité.

L'analyse de l'effet de la molécule A11 sur la cinétique d'association de NKA-Bo (Figure 6C) révèle une accélération modeste mais détectable. La liaison rapide de NKA-Bo, enregistrée à une concentration de 20 nM à l'aide de l'équipement de mélange rapide, se décompose en une somme de deux exponentielles dont les vitesses (kₐₚₚ) différent d'un facteur 10. La liaison rapide de NKA-Bo (kₐₚₚ = 0,095 sec⁻¹) représente 44% du signal total de liaison, en absence de A11 (50 µM), tandis qu'elle représente 52% du signal total en présence de A11. La liaison lente s'effectue dans les deux cas avec une vitesse apparente (kₐₚₚ) égale à 0,0095 sec⁻¹, et une amplitude égale à 66% en absence de A11, et 48% en présence de A11. Ces résultats s'interprètent selon un modèle décrit dans Palanché et al. (2001) dans les termes suivants :
- la liaison rapide tout comme la liaison lente s'effectuent d'une manière qui est contrôlée par la diffusion de NKA-Bo dans le milieu,
- la vitesse apparente de la liaison rapide augmente de manière linéaire avec la concentration de NKA-Bo. Il s'agit donc d'une liaison contrôlée exclusivement par la diffusion,
- la vitesse apparente de la liaison lente augmente de manière non linéaire avec la concentration de NKA-Bo et sature à des concentrations de ligand de l'ordre de 500 nM. Cette cinétique lente reflète l'interconversion lente d'un état conformationnel de faible affinité pour la NKA-BO vers un état de plus haute affinité dont on détecte l'occupation.

Ainsi, le récepteur existe dans au moins deux états conformationnels dont l'un préexiste à l'addition d'agoniste et l'autre apparaît lentement au cours du temps en présence d'agoniste.

La molécule A11, en stabilisant les états de faible affinité du récepteur, augmente la proportion de liaison rapide (de faible affinité) au détriment de la liaison lente qui reflète la stabilisation d'un état de plus haute affinité

### EXEMPLE 4 : Identification, par analyse de la liaison de NKA-Bo, d'autres molécules se comportant comme des effecteurs allostériques du récepteur NK2R des tachykinines.

L'identification d'autres effecteurs allostériques du récepteur NK2R des tachykinines est faite par l'analyse de la cinétique de dissociation de NKA-Bo liée au récepteur.

10⁶ cellules HEK 293 exprimant le récepteur EGFP-NK2R sont mises en suspension dans du tampon Hepes (voir plus haut) et incubées avec de la NKA-Bo (20 nM) et l'une des molécules suivantes : 805, NP246, A11, H10, G11, F7, F3 et NP 234. Les molécules A11 et l'antagoniste compétitif connu SR 48968 sont introduits dans l'expérience comme contrôles positifs. La dissociation de NKA-Bo est enregistrée, dans une cuvette de spectrofluorimètre munie d'une agitation magnétique pendant 700 secondes, après avoir ajouté 10 µM de NKA non fluorescente pour chasser la NKA-Bo liée. La cinétique de dissociation de NKA-Bo est enregistrée en présence de concentrations croissantes de chacune des molécules précitées.

La figure 7A montre une expérience typique de mesure de la vitesse de dissociation de NKA-Bo en présence de concentrations croissantes de la molécule 805. Ces déterminations permettent de mesurer plusieurs paramètres expérimentaux : i) la vitesse de dissociation de NKA-Bo qui sera ensuite décomposée en une somme de relaxations exponentielles et ii) l'amplitude relative de chaque relaxation exponentielle qui donnera la mesure de la concentration fractionnelle de chacun des états conformationnels détectés dans l'expérience. On détermine ainsi que la dissociation de NKA-Bo se produit en deux étapes caractérisées par des constantes de vitesse k₁ = 0,04 sec⁻¹ et k₂ = 0,008 sec⁻¹ et des amplitudes λ1/λ2 variables : λ1/λ2= 30/70 (sans -805) ; λ1/λ2= 35/65 (1 µM 805) ; λ1/λ2= 9/31 (10 µM 805) et λ1/λ2= 80/20 (50 µM 805).

Les figures 7B à 7I montrent, pour chaque molécule testée, la variation d'amplitude de liaison de NKA-Bo et la variation d'amplitude de la dissociation rapide. Ces figures montrent que toutes les molécules indiquées se comportent comme des effecteurs allostériques car elles provoquent une augmentation de l'amplitude de la dissociation rapide de NKA-Bo.

L'augmentation d'amplitude de la dissociation rapide reflète la capacité des molécules à stabiliser une fraction plus importante des récepteurs dans l'état de faible affinité. On voit ainsi que la fraction initiale de récepteurs dans l'état de faible affinité passe d'environ 25% en absence d'effecteur à 60-75% en présence des molécules 805, NP234, A11, G11, F7, F3, et H10 (à 100 µM d'effecteur) et à près de 100% en présence de A11 et NP246.

### EXEMPLE 5 : Etude de l'inhibition de la liaison de l'antagoniste compétitif SR 48968 radioactif par l'agoniste NKA et l'effecteur allostérique 805.

25 000 cellules exprimant le récepteur EGFP-NK2R sont incubées pendant 3 heures à 4°C dans un volume final de 500 µl de tampon Hepes en présence de 1 nM de [³H] SR48968 et de concentrations variables de NKA ou de 805. Au terme de l'incubation, les mélanges réactionnels sont filtrés sur des filtres GF/C (Whatmann) en fibre de verre préalablement incubés dans du tampon Hepes complémenté de 1 % (W/V) de lait en poudre semi-écrémé, et rincés trois fois par 5 ml de tampon hepes à 4°C. Les filtres sont ensuite disposés dans des fioles de comptage à scintillation dans lesquelles on ajoute 3 ml de cocktail scintillant. La radioactivité est mesurée après 16 h dans un compteur de radioactivité. La figure 8A montre que l'agoniste NKA déplace quantitativement la liaison de SR 48968 avec une affinité apparente Kᵢ = 22 nM. En revanche, l'effecteur 805 est incapable de déplacer la totalité du SR 48968 lié, même lorsque sa concentration est de l'ordre du millimolaire. Il renverse seulement 27-33% de la liaison du radioligand avec une affinité apparente Kᵢ = 5 µM. L'effet de 805 sur la liaison de SR 48968 ne reflète pas une interaction compétitive mais plutôt de nature non-compétitive.

### - EXEMPLE 6 : Quantification de l'effet de la molécule 805 sur la réponse d'accumulation de cAMP dans des cellules HEK 293 exprimant le récepteur EGFP-NK2R

Des boîtes de culture cellulaire à 6 puits de 30 mm sont traitées au collagène puis ensemencées avec 50 000 cellules exprimant le récepteur EGFP-NK2R. Après deux à trois jours de culture, le milieu est remplacé par du tampon hepes contenant de l'inhibiteur de phosphodiestérase IBMX (isobutyl-methyl-xhantine) et incubées pendant 30 minutes avec des concentrations croissantes de NKA en présence ou en absence i) d'inhibiteur compétitif H8565 (1 µM) ou ii) d'effecteur 805 (10 ou 50 µM). La quantité de cAMP produit (exprimé en pmoles/puits) est déterminée selon un procédé radioimmunologique décrit dans Gicquiaux et al. (2002). Il en résulte une mesure de la courbe concentration-effet de NKA (Figure 9A).

La NKA seule est capable de stimuler la production de cAMP dans les cellules exprimant le récepteur EGFP-NK2R avec une EC₅₀ voisine de 100 nM.

En présence d'une concentration fixe de l'antagoniste compétitif H8565, on note une diminution de l'affinité apparente de la NKA (EC₅₀ = 300 nM) sans que le niveau maximal de la réponse ne soit affecté (réponse max. = 100%).

En présence de l'effecteur 805 à 10 ou 50 µM, on ne détecte aucune variation de l'EC₅₀ de la NKA (EC₅₀ =100 nM) mais la réponse maximale n'atteint jamais la valeur du contrôle (Rₘₐₓ = 0,7 pour [805]= 10 µM et Rₘₐₓ = 0,3 pour [805] = 50 µM). La valeur de la concentration de 805 qui conduit à 50% de diminution de la réponse maximale d'AMPc est de l'ordre de 6 µM (figure 9C). Ainsi, l'affinité apparente pour la réponse, mais pas son amplitude maximale, est diminuée en présence d'antagoniste compétitif, tandis que l'amplitude maximale de la réponse, mais pas son affinité apparente, est diminuée par un effecteur allostérique négatif.

### EXEMPLE 7 : Quantification de l'effet de la molécule 805 sur la réponse d'accumulation de calcium évoquée par la NKA-Bo dans des cellules HEK 293 exprimant le récepteur EGFP-NK2R

Les réponses calciques évoquées par l'agoniste NKA-Bo sur des cellules exprimant le récepteur EGFP-NK2R sont enregistrées après chargement des cellules avec la sonde fluorescente sensible au calcium : indo-1, selon le protocole décrit dans Vollmer et al. (1999). Les cellules sont mises en suspension à raison de 500 000 cellules/ml dans du tampon Hepes et disposées dans une cuve de spectrofluorimètre. La longueur d'onde d'excitation est fixée à 338 nm et l'émission est enregistrée à 400 et 470 nm. Les réponses tracées sont les rapports des signaux 400/470. Les figures 10A, 10B et 10C montrent que le composé 805 (50 µM) augmente la durée des réponses calciques évoquées par 1, 5 et 1000 nM de NKA. De la même manière, le composé 805, aux concentrations de 10 et 50 µM, augmente la durée de la réponse calcique évoquée par 5 nM de NKA (figure 10D).

Le composé 805 se comporte donc comme un effecteur allostérique positif des réponses calciques évoquées par l'agoniste NKA-Bo qui augmente la durée de la réponse. Ceci est indiqué en figure 10E où l'on peut voir que le temps nécessaire pour retourner à 50% de la réponse maximale passe de 91 secondes en absence de 805 à 105 puis 115 s lorsque la concentration de 805 est de 10 puis 50 µM.

### EXEMPLE 8 : Quantification de l'effet de la molécule 805 sur la réponse d'accumulation de calcium évoquée par la NKA 4-10 TR7, dans des cellules HEK 293 exprimant le récepteur EGFP-NK2R.

Les réponses calciques évoquées par l'agoniste tronqué NKA4-10 TR7 sur des cellules exprimant le récepteur EGFP-NK2R sont enregistrées après chargement des cellules avec la sonde fluorescente sensible au calcium : indo-1 selon le protocole décrit dans Vollmer et al. (1999). Les cellules sont mises en suspension à raison de 500 000 cellules/ml dans du tampon Hepes et disposées dans une cuve de spectrofluorimètre. La longueur d'onde d'excitation est fixée à 355 nm et l'émission est enregistrée à 375 et 405 nm. Les réponses tracées sont les rapports des signaux 375 nm/405 nm. Les figures 11A à 11D montrent que le composé 805 (20 µM) augmente l'amplitude de la réponse évoquée par NKA 4-10 TR7 (106, 50.3, 26.5 et 10.6 nM). La variation d'amplitude par 805 est illustrée dans le panneau B de la figure 11 pour des concentrations de 5, 10, 25, 50 et 106 nM de NKA 4-10 TR7. Le composé 805 s'avère être un effecteur allostérique positif des réponses calciques évoquées par NKA 4-10 TR7 qui diminue la dose efficace à 50 % d'environ 20 nM à environ 15 nM.

### EXEMPLE 9 : Quantification de l'effet de la molécule 805 sur la dissociation de NKA4-10 TR7 du récepteur EGFP-NK2R.

La NKA 4-10 correspond à une forme tronquée de la NKA que l'on trouve dans les fluides des tumeurs de l'intestin moyen. La NKA 4-10 fluorescente est modifiée en position 7 pour introduire une cystéine qui est ensuite marquée par le groupe fluorescent Texas Red. La NKA 4-10 fluorescente est, tout comme la NKA fluorescente, un agoniste du récepteur EGFP-NK2R, à ceci près qu'elle est incapable d'évoquer des réponses d'accumulation d'AMPc (Palanche et al., 2001).

La liaison de NKA 4-10 fluorescente se fait selon un processus monoexponentiel auquel est associé le développement d'une réponse calcique dans les cellules HEK 293 exprimant le récepteur EGFP-NK2R (Palanche et al., 2001).

A 10°C, la dissociation de NKA 4-10 rendue fluorescente à l'aide du groupe Texas red introduit en position 7 du peptide (NKA4-10 TR7) se déroule selon un processus biphasique caractérisé par les constantes de vitesse rapide et lente égales à k₁ = 0,5 sec⁻¹ et k₂ = 0,015 sec⁻¹, et des amplitudes respectives de 58% pour la dissociation rapide et 42 % pour la dissociation lente (figure 11F). En présence de 10 µM de 805, on observe une accélération de la dissociation caractérisée par une modification des amplitudes des étapes rapides (88%) et lentes (12%) de dissociation. On note une relation entre l'augmentation de vitesse de dissociation de NKA4-10 TR7 et la concentration de 805 (figure 11G). La concentration de 805 qui provoque une augmentation de 50 % de l'amplitude de la dissociation rapide de NKA4-10 TR7 est de l'ordre de 20 µM.

### EXEMPLE 10 : Potentialisation des réponses calciques des récepteurs NK1 et NK2 humains sauvages par la molécule 805.

Les réponses calciques évoquées par les agonistes suivants : substance P (SP) et neurokinine A (NKA), sur des cellules exprimant le récepteur NK1 humain sauvage ou NK2 humain sauvage sont enregistrées après chargement des cellules avec la sonde fluorescente sensible au calcium : indo-1 (3 µM) selon le protocole décrit dans Vollmer et al. (1999). Les cellules sont mises en suspension à raison de 1.000.000 cellules/ml dans du tampon Hepes (op. cit.) et disposées dans une cuve de spectrofluorimètre. La longueur d'onde d'excitation est fixée a 338 nm et l'émission est enregistrée à 400 nm. Les réponses tracées sont les signaux enregistrés à 400 nm. Les figure 12A et 12B montrent que le composé 805 (20 µM) augmente l'amplitude de la réponse évoquée par la substance P (0.1 ou 10 nM). Les figures 12C et 12D montrent que le composé 805 (20 µM) augmente l'amplitude de la réponse évoquée par la neurokinine A (10 ou 100 nM).

Le composé 805 s'avère être un effecteur allostérique positif des réponses calciques évoquées par la neurokinine A sur le récepteur NK2 humain sauvage et des réponses évoquées par la substance P sur le récepteur NK1 humain sauvage.

### EXEMPLE 11 : Identification d'effecteurs allostériques du récepteur muscarinique M1 de l'acétylcholine.

Le récepteur muscarinique M1 fluorescent est obtenu par fusion de son ADNc avec celui de la EGFP ou de la YPFP ainsi que décrit dans Ilien et al. (2003) et exprimé de manière stable dans des cellules HEK 293. Les cellules sont mises en suspension à une concentration de 1 000 000 cellules/ml dans du tampon physiologique Hepes (en mM: 137,5 NaCl ; 1,25 MgCl₂ ; 1,25 CaCl₂ ; 6 KCl; 5,6 glucose; 10 Hepes; 0,4 NaH₂PO₄ ; 1% BSA (w/v) ; pH 7,4) et disposées dans une cuvette de fluorescence de 1 ml, elle-même placée dans le portoir agitant d'un spectrofluorimètre. La suspension de cellules est excitée à 470 nm et l'émission de fluorescence est enregistrée à 510 nm (EGFP) ou à 530 nm (EYFP). L'addition de 70 nM de bodipy(558-568)-pirenzepine provoque une diminution d'émission de fluorescence de la protéine fluorescente portée par le récepteur qui reflète l'étape d'association du ligand au récepteur. Le décours temporel de cette étape est suivi par enregistrement de l'émission à 510 ou 530 nm. Alternativement, le complexe entre le récepteur et la bodipy-pirenzepine est formé dans une cuvette de fluorescence placée dans le spectrofluorimètre avant d'ajouter un excès (5 µM) d'atropine pour initier l'étape de dissociation du complexe récepteur ligand. Le décours temporel de cette étape de dissociation est enregistré à 510 ou 530 nm. Pour identifier un effecteur allostérique du récepteur muscarinique M1 de l'acétylcholine, on pré incube les cellules avec une, ou plusieurs, molécule(s), connue(s) ou inconnue(s), et l'on enregistre le décours temporel de l'étape d'association et/ou de l'étape de dissociation. Les cinétiques enregistrées sont ensuite ajustées par une courbe multiexponentielle de type y = λ1 exp(-k1ₐₚₚ × T) + λ₂ exp(-k2ₐₚₚ × T)+ "droite", où λ1 et λ2 sont les amplitudes des relaxations rapide et lente, k1ₐₚₚ et k2ₐₚₚ sont les constantes de vitesse apparente d'association ou de dissociation rapide et lente, T est le temps et "droite" est une correction mathématique de la dérive du signal. Un effecteur allostérique soit accélère, soit en ralentit le décours temporel soit de l'étape d'association, soit de l'étape de dissociation, soit des deux. Cela se traduit par une variation relative des amplitudes d'association (ou de dissociation) rapide et lente, comparativement à la condition expérimentale contrôle (sans effecteur).

### EXEMPLE 12 : Identification d'effecteurs allostériques du récepteur CXCR4 des chimiokines.

Le récepteur CXCR4 fluorescent est obtenu par fusion de son ADNc avec celui de la EGFP ainsi que décrit dans Valenzuela et al. (2001) et dans Palanché et al. (2001) et exprimé de manière stable dans des cellules HEK 293. Les cellules sont mises en suspension à une concentration de 1 000 000 cellules/ml dans du tampon physiologique Hepes (en mM : 137,5 NaCl ; 1,25 MgCl₂ ; 1,25 CaCl₂ ; 6 KCl ; 5,6 glucose ; 10 Hepes ; 0,4 NaH₂PO₄; 1% BSA (w/v) ; pH 7,4) et disposées dans une cuvette de fluorescence de 1 ml elle même placée dans le portoir agitant d'une spectrofluorimètre. La suspension de cellules est excitée à 470 nm et l'émission de fluorescence est enregistrée à 510 nm. L'addition de 50 nM de Texas Red-SDF1α provoque une diminution d'émission de fluorescence de la protéine fluorescente portée par le récepteur qui reflète l'étape d'association du ligand au récepteur. Le décours temporel de cette étape est suivi par enregistrement de l'émission à 510 nm. Alternativement, le complexe entre le récepteur et la Texas Red-SDF1α est formé dans une cuvette de fluorescence placée dans le spectrofluorimètre avant d'ajouter un excès (500 nM) de SDF1α pour initier l'étape de dissociation du complexe récepteur ligand. Le décours temporel de cette étape de dissociation est enregistré à 510 nm. Pour identifier un effecteur allostérique du récepteur CXCR4, on pré-incube les cellules avec une, ou plusieurs, molécule(s), connue(s) ou inconnue(s), et l'on enregistre le décours temporel de l'étape d'association et/ou de l'étape de dissociation. Les cinétiques enregistrées sont ensuite ajustées par une courbe multiexponentielle de type y = λ₁ exp(-k1ₐₚₚ × T) + λ₂ exp(-k2ₐpp × T)+ "droite", où λ₁ et λ₂ sont les amplitudes des relaxations rapide et lente, k1ₐₚₚ et k2ₐₚₚ sont les constantes de vitesse apparente d'association ou de dissociation rapide et lente, T est le temps et "droite" est une correction mathématique de la dérive du signal. Un effecteur allostérique soit accélère, soit en ralentit, le décours temporel soit de l'étape d'association, soit de l'étape de dissociation, soit des deux. Cela se traduit par une variation relative des amplitudes d'association (ou de dissociation) rapide et lente, comparativement à la condition expérimentale contrôle (sans effecteur).

### RÉFÉRENCES

- Alber et Kawasaki (1982) *J. Mol. Appl. Gen.,* **1**, 419-434,
- Baulmann et al. (2000) *Neuroscience,* **95**, 813-820,
- Beaucage and Caruthers (1981) *Tett. Lett.,* **22,** 1859-1869,
- Befort et al. (1996) *Neurochem. Res.,* **11,** 1301-1307,
- Birdsall et al. (1999) *Mol. Pharmacol.,* **55,** 778-786,
- Brisco, J. et al. (1996) *Phylos. Trans. R. Soc. Lond. B. Biol. Sci.,* **351,** 167-171,
- Bruns & Ferguns (1990) *Mol Pharmacol,* **38,** 939-949,
- Burstein et al (1997) *Mol. Pharmacol.* **51,** 312-319,
- Chalfie (1995) *Photochem. Photobiol.,* **62,** 651-656,
- Clegg (1995) *Current Opinion in Biotechnol.,* **6**, 103-110,
- Cormack et al. (1995) *Gene*, **173**, 33-38,
- Cormack et al. (1996) *Gene,* **173**, 1246-1251,
- Crameri et al. (1996) *Nature Biotechnol.,* **14**, 315-319,
- Ehrig et al. (1995) *FEBS Lett.* **367,** 163-166
- Fawzi et al. (2001) *Mol. Pharmacol.* **59**, 30-37,
- Förster (1951) in Fluoreszenz organischer Verbindung. Vandenhoek and Rupprecht, Göttingen,
- Galzi & Alix (1997), demande internationale WO 98/55873,
- Galzi et al. (1996) *EMBO J.,* **15**, 5824-5832,
- Galzi et al. (1996) *PNAS,* **93**, 1853-1858,
- Gicquiaux et al. (2002) *J. Biol. Chem.* **277**, 6645-6655,
- Haas et al. (1996) *Curr. Biol.,* **6**, 315-323,
- Hall (2000) *Mol. Pharmacol.,* **58**, 1412-1423,
- Hausdorff et al. (1990) *J. Biol. Chem* **265,** 1388-1393,
- Heim & Tsien (1996) *Current Biology,* vol. n° 6, p178-182,
- Heim, Cubitt et Tsien (1995) *Nature,* **373**, 663-664,
- Hille, B. (1992) in Ion channels of excitable membranes, Sinauer Associates, Sunderlands, Massachussets,
- Hitzeman et al. (1980) *J. Biol. Chem.,* **255,** 12073-12080,
- Hoare & Strange (1995) *Biochem. Soc. Trans.,* **23,** 92S,
- Hoare et al. (2000) *British J. Pharmacol.* **130,** 1045-1059,
- Honneger et al. (1988) *EMBO J.* **7, 3053-3060,**
- Ihle, J.N. (1995) *Nature* **377**, 591-594,
- Ilien et al. (2003) *J*. *Neurochem* **85,** 768-778,
- Jakubik et al. (1997) *Mol. Pharmacol.,* **52,** 172-179,
- Ko et al. (2002) *Mol. Cell. Biol.,* **22,** 357-369,
- Kostenis et Mohr (1996), *Trends in Pharmacol. Sci.,* **17**, 280-283,
- Krause et al. (1997) *Mol Pharmacol.,* **53**, 283-294
- Lakey et al. (1991) *J*. *Mol. Biol.,* **218**, 639-653
- Lazareno & Birdsall (1995) *Mol. Pharmacol.,* **48,** 362-378
- Lefkowitz (1998) *J. Biol. Chem.,* **273,** 18677-18680,
- Mangelsdorf et al. (1995), *Cell,* **83,** 835-839,
- Massot et al. (1996), *Mol. Pharmacol.,* **50,** 752-762,
- McKnight et al. (1985) *EMBO J.,* **4**, 2093-2099,
- Miyawaki et al. (1997) *Nature,* vol. 388, p882-887,
- Monod et al. (1965) *J. Mol. Biol.*, **12**, 88-118,
- Moutou et al. (1994) *Tet. Lett.,* **35**, 6883-6886,
- Mulle et al. (1992) *Neuron.,* **8,** 937-945,
- Nicolini et al. (1995) *Biosens. Bioelection.,* **10**, 723-733,
- Nunnari et al. (1987) *J. Biol. Chem.,* **262,** 12387-12392,
- Palanché et al. (2001) *J. Biol. Chem.,* **276,** 34853-34861,
- Pan et al. (2001) *Aneastesiology,* **95,** 416-420,
- Prasher et al. (1992) *Gene,* 111, 229-233,
- Reichel et al. (1996) *Proc. Natl. Acad. Sci.,* **93,** 5888-5893,
- Rubin et Changeux (1966) *J. Mol. Biol.,* **21,** 265-274,
- Russell (1985) *Gene,* **40**,125-130,
- Subramani et al. (1981) *Mol Cell. Biol.,***1**, 854-864,
- Tucek et Proska (1995) *TIPS,* **16**, 205-212,
- Valenzuela et al. (2001) *J. Biol. Chem.,* **276**, 26550-26558,
- Vasuvedan et al. (1992) *FEBS Lett.,* **311,** 7-11,
- Vlack et al. (1988) *J*. *Gen. Virol.,* **69,** 765-776,
- Vollmer et al (1999) *J. Biol. Chem.,* **274,** 37915-37922,
- Vuong et Chabre (1990) *Nature,* **346,** 71-74,
- Ward et al. (1980) *Photochem. Photobiol.,* **31,** 611-615,
- Waugh et al. (1999) *J. Pharm. Exp. Ther,* **291,** 1164-1171,
- Weill et al. (1999) *J*. *Neuro. Chem.,* **73,** 791-801,
- Wu and Brand (1994) *Anal. Biochem.,* **218,** 1-13,
- Wurtz, J.L. et al. (1996) *Nature Struct. Biol.,* **3**, 206,
- Yan et al. (1996) *Nucleic Ac. Res.,* **24,** 4592-4593,
- Yarden, Y. and Ullrich, A. (1988) *Biochemistry,* **27,** 3113-3119,
- Young et al. (1982) dans Genetic Engineering of microorganisms for chemicals (Hollaender et *al.* eds), Plenum Press, NY 1982,
- Yuan et al. (2000) *J. Biol. Chem.,* **275,** 2157-2164,
- Zolotukhin et al. (1996) *J. Virol.,* **70,** 4646-4654.

## Revendications

1. Procédé de mise en évidence d'un effecteur allostérique d'un récepteur, par détermination de la variation :
- de la cinétique de dissociation et/ou d'association du complexe formé entre le susdit récepteur et l'un de ses ligands en présence dudit effecteur allostérique, par rapport à la cinétique de dissociation et/ou d'association du complexe formé entre ledit récepteur et ledit ligand, en l'absence dudit effecteur,
- et/ou de l'amplitude de la liaison formée entre le susdit récepteur et l'un de ses ligands en présence dudit effecteur allostérique, par rapport à l'amplitude de la liaison formée entre ledit récepteur et ledit ligand, en l'absence dudit effecteur,
sous réserve que lorsque la variation de la susdite amplitude est négative, on détecte également l'existence de la variation de la susdite cinétique,
ledit récepteur et ledit ligand étant impliqués dans au moins une réponse biologique dans des conditions physiologiques appropriées, et l'effecteur allostérique étant capable de moduler au moins l'une des réponses,
ledit récepteur étant marqué par une protéine fluorescente choisie parmi les protéines fluorescentes issues ou dérivées de protéines autofluorescentes de cnidaires, dont le coefficient d'extinction moléculaire est supérieur à environ 14000M⁻¹.cm⁻¹ et le rendement quantique de fluorescence est supérieur à environ 0,38,
ledit ligand étant marqué par un marqueur constitué :
- soit par une molécule susceptible d'absorber la lumière émise par la protéine fluorescente,
- soit par une substance fluorescente,
lesdites déterminations de variation de cinétique de dissociation et/ou d'association et de variation d'amplitude étant effectuées par transfert d'énergie de fluorescence :
• entre la protéine fluorescente susmentionnée et la susdite substance fluorescente, la substance fluorescente étant telle que soit elle est excitable à la longueur d'onde d'émission de la protéine fluorescente susmentionnée, soit elle émet à la longueur d'excitation de la protéine fluorescente susmentionnée, ou
• entre la protéine fluorescente susmentionnée et la susdite molécule susceptible d'absorber la lumière émise par la protéine fluorescente.

2. Procédé selon la revendication 1, ledit récepteur étant marqué par une protéine fluorescente choisie parmi :
- la protéine fluorescente verte (GFP ou EGFP), la protéine fluorescente cyan (CFP ou ECFP), la protéine fluorescente jaune (YFP ou EYFP) ou GFPUV, ou,
- des variants dérivés de la GFP, de la CFP, de la YFP ou de la GFPUV, par addition, délétion ou substitution d'un ou plusieurs acides aminés, sous réserve que ces variants conservent la propriété de fluorescence,
- ou des fragments de la GFP, de la CFP, de la YFP ou de la GFPUV, ou de fragments des susdits variants, sous réserve que ces fragments conservent la propriété de fluorescence,
ledit ligand étant marqué par un marqueur constitué :
- soit par une molécule susceptible d'absorber la lumière émise par la protéine fluorescente,
- soit par une substance fluorescente,

3. Procédé selon l'une des revendications 1 à 2, **caractérisé en ce que** le ligand est un antagoniste.

4. Procédé selon l'une des revendications 1 à 2, **caractérisé en ce que** le ligand est un agoniste.

5. Procédé selon l'une des revendications 1 à 2, **caractérisé en ce que** l'on détermine :
- la variation de l'amplitude de la liaison formée entre le susdit récepteur et l'un de ses ligands en présence dudit effecteur allostérique, par rapport à l'amplitude de la liaison formé entre ledit récepteur et ledit ligand, en l'absence dudit effecteur,
et que ladite variation est négative, ce qui nécessite la détermination de :
- la variation de la cinétique de dissociation du complexe formé entre le susdit récepteur et l'un de ses ligands en présence dudit effecteur allostérique, par rapport à la cinétique de dissociation du complexe formé entre ledit récepteur et ledit ligand, en l'absence dudit effecteur.

6. Procédé selon la revendication 5, **caractérisé en ce que** ladite variation de cinétique de dissociation est positive ou négative, ce qui implique que le composé testé est un effecteur allostérique.

## Claims

1. Process for detecting an allosteric effector of a receptor, by determination of the variation:
- in the dissociation and/or association kinetics of the complex formed between the abovementioned receptor and one of its ligands in the presence of said allosteric effector, relative to the dissociation and/or association kinetics of the complex formed between said receptor and said ligand, in the absence of said effector,
- and/or in the amplitude of the bond formed between the abovementioned receptor and one of its ligands in the presence of said allosteric effector, relative to the amplitude of the bond formed between said receptor and said ligand, in the absence of said effector,
provided that when the variation in the abovementioned amplitude is negative, the existence of the variation in the abovementioned kinetics is also detected,
said receptor and said ligand being involved in at least one biological response under appropriate physiological conditions, and the allosteric effector being capable of modulating at least one of the responses,
said receptor being marked by a fluorescent protein chosen from the fluorescent proteins obtained or derived from autofluorescent proteins of cnidaria, the molecular extinction coefficient of which is greater than approximately 14000M⁻¹.cm⁻¹ and the fluorescence quantum efficiency is greater than approximately 0.38,
said ligand being marked by a marker constituted:
- either by a molecule capable of absorbing the light emitted by the fluorescent protein,
- or by a fluorescent substance,
said determinations of variation in dissociation and/or association kinetics and of variation in amplitude being carried out by fluorescence energy transfer:
• between the abovementioned fluorescent protein and the abovementioned fluorescent substance, the fluorescent substance being such that either it is excitable at the emission wavelength of the abovementioned fluorescent protein, or it emits at the excitation wavelength of the abovementioned fluorescent protein, or
• between the abovementioned fluorescent protein and the abovementioned molecule capable of absorbing the light emitted by the fluorescent protein.

2. Process according to claim 1, wherein said receptor is marked by a fluorescent protein chosen from:
- green fluorescent protein (GFP or EGFP), cyan fluorescent protein (CFP or ECFP), yellow fluorescent protein (YFP or EYFP) or GFPUV, or,
- variants derived from GFP, CFP, YFP or GFPUV, by addition, deletion or substitution of one or more amino acids, provided that these variants preserve the property of fluorescence,
- or fragments of GFP, CFP, YFP or GFPUV, or fragments of the abovementioned variants, provided that these fragments preserve the property of fluorescence,
said ligand being marked by a marker constituted:
- either by a molecule capable of absorbing the light emitted by the fluorescent protein,
- or by a fluorescent substance.

3. Process according to one of claims 1 to 2, **characterized in that** the ligand is an antagonist.

4. Process according to one of claims 1 to 2, **characterized in that** the ligand is an agonist.

5. Process according to one of claims 1 to 2, **characterized in that**:
- the variation in the amplitude of the bond formed between the abovementioned receptor and one of its ligands in the presence of said allosteric effector, relative to the amplitude of the bond formed between said receptor and said ligand, in the absence of said effector is determined,
and that said variation is negative, which requires the determination of:
- the variation in the dissociation kinetics of the complex formed between the abovementioned receptor and one of its ligands in the presence of said allosteric effector, relative to the dissociation kinetics of the complex formed between said receptor and said ligand, in the absence of said effector.

6. Process according to claim 5, **characterized in that** said variation in dissociation kinetics is positive or negative, which implies that the compound tested is an allosteric effector.

## Patentansprüche

1. Verfahren zum Nachweis eines allosterischen Effektors eines Rezeptors durch Bestimmen der Änderung:
- der Dissoziations- und/oder Assoziationskinetik des zwischen dem Rezeptor und einem seiner Liganden gebildeten Komplexes in Gegenwart dieses allosterischen Effektors im Vergleich zur Dissoziations- und/oder Assoziationskinetik des zwischen dem Rezeptor und dem Liganden gebildeten Komplexes in Abwesenheit des Effektors,
- und/oder der Amplitude der zwischen dem Rezeptor und einem seiner Liganden gebildeten Bindung in Gegenwart dieses allosterischen Effektors im Vergleich zur Amplitude der zwischen dem Rezeptor und dem Liganden gebildeten Bindung in Abwesenheit des Effektors,
mit der Maßgabe, dass man, wenn die Änderung dieser Amplitude negativ ist, das Vorhandensein der Änderung der genannten Kinetik auch nachweist,
wobei der Rezeptor und der Ligand unter geeigneten physiologischen Bedingungen in mindestens eine biologische Reaktion involviert sind und der allosterische Effektor in der Lage ist, mindestens eine der Reaktionen zu modulieren,
wobei der Rezeptor durch ein fluoreszierendes Protein markiert ist, das aus den fluoreszierenden Proteinen ausgewählt ist, die von autofluoreszierenden Proteinen von Cnidaria stammen oder abgeleitet sind, deren molekularer Extinktionskoeffizient größer als etwa 14 000 M⁻¹.cm⁻¹ und deren Fluoreszenzquantenausbeute größer als etwa 0,38 ist,
wobei der Ligand durch einen Marker markiert ist, bestehend aus:
- einem Molekül, das in der Lage ist, das von dem fluoreszierenden Protein emittierte Licht zu absorbieren, oder
- einer fluoreszierenden Substanz,
wobei die Bestimmungen der Änderung der Dissoziations- und/oder Assoziationskinetik und der Änderung der Amplitude durch Übertragung von Fluoreszenzenergie erfolgen:
- zwischen dem fluoreszierenden Protein und der fluoreszierenden Substanz, wobei die fluoreszierende Substanz entweder bei der Emissionswellenlänge des fluoreszierenden Proteins anregbar ist oder bei der Anregungswellenlänge des fluoreszierenden Proteins emittiert, oder
- zwischen dem fluoreszierenden Protein und dem Molekül, das in der Lage ist, das von dem fluoreszierenden Protein emittierte Licht zu absorbieren.

2. Verfahren nach Anspruch 1, wobei der Rezeptor durch ein fluoreszierendes Protein markiert ist, welches ausgewählt ist aus:
- grün fluoreszierendem Protein (GFP oder EGFP), cyan fluoreszierendem Protein (CFP oder ECFP), gelb fluoreszierendem Protein (YFP oder EYFP) oder GFPUV oder
- Varianten, die von GFP, CFP, YFP oder GFPUV durch Addition, Deletion oder Substitution einer oder mehrerer Aminosäuren abgeleitet sind, mit der Maßgabe, dass die Varianten die Fluoreszenzeigenschaft beibehalten,
- oder Fragmenten von GFP, CFP, YFP oder GFPUV oder Fragmenten der oben genannten Varianten, mit der Maßgabe, dass die Fragmente die Fluoreszenzeigenschaft beibehalten,
wobei der Ligand durch einen Marker markiert ist, bestehend aus:
- einem Molekül, das in der Lage ist, das von dem fluoreszierenden Protein emittierte Licht zu absorbieren, oder
- einer fluoreszierenden Substanz.

3. Verfahren nach einem der Ansprüche 1 bis 2, **dadurch gekennzeichnet, dass** der Ligand ein Antagonist ist.

4. Verfaluen nach einem der Ansprüche 1 bis 2, **dadurch gekennzeichnet, dass** der Ligand ein Agonist ist.

5. Verfahren nach einem der Ansprüche 1 bis 2, **dadurch gekennzeichnet, dass**
- die Änderung der Amplitude der zwischen dem Rezeptor und einem seiner Liganden gebildeten Bindung in Gegenwart des allosterischen Effektors im Vergleich zur Amplitude der zwischen dem Rezeptor und dem Liganden gebildeten Bindung in Abwesenheit des Effektors bestimmt wird
und dass diese Änderung negativ ist, was die Bestimmung
- der Änderung der Dissoziationskinetik des zwischen dem Rezeptor und einem seiner Liganden gebildeten Komplexes in Gegenwart des allosterischen Effektors im Vergleich zur Dissoziationskinetik des zwischen dem Rezeptor und dem Liganden gebildeten Komplexes in Abwesenheit des Effektors erfordert.

6. Verfahren nach Anspruch 5, **dadurch gekennzeichnet, dass** die Änderung der Dissoziationskinetik positiv oder negativ ist, was impliziert, dass die getestete Verbindung ein allosterischer Effektor ist.
